# EUROPEAN PATENT APPLICATION

(11) **EP 4 306 640 A1**
(43) Date of publication of application: **17.01.2024**
(21) Application number: 22180211.9
(22) Date of filing: 21.06.2022
(51) Int. Cl.: C12N 15/115, A61K 31/00, C07K 16/00

(54) **METHOD FOR TREATING A TUMOR IN A SUBJECT**

(71) Applicant: TME Pharma AG, 10589 Berlin (DE)
(72) Inventor: The designation of the inventor has not yet been filed
(74) Representative: Bohmann, Armin K.

(57) **Abstract**

The present invention is related to a C-X-C motif chemokine 12 (CXCL12) antagonist for use in a method for treating a tumor in a subject, wherein the method comprises administering to the subject
- the C-X-C motif chemokine 12 (CXCL12) antagonist,
- a radiotherapy, and
- an anti-angiogenic compound,
wherein the tumor is a brain tumor.

## Description

The present invention is related to a method for treating a tumor in a subject.

Brain cancer is a disease of the brain in which cancer cells arise in the brain tissue. Cancer cells grow to form a tumor, i.e. a mass of cancer tissue which also contains supportive non-cancerous cells (the tumor stroma) that interfere with brain functions such as muscle control, sensation, memory, and other normal body functions. Tumors that contain cancer cells that grow in an uncontrolled way and can invade nearby tissues and spread to other parts of the body through the blood and lymph system are called malignant tumors, and those without tendencies to invade nearby tissues or spread to distant body parts are called benign tumors. Tumors that develop from brain tissue are called primary brain tumors while tumors that spread from other body sites to the brain are termed metastatic or secondary brain tumors. Statistics suggest that brain cancer occurs infrequently (1.4% of all new cancer patients per year), so it is likely to develop in about 23,770 new people per year with about 16,050 deaths as estimated by the National Cancer Institute (NCI) and the American Cancer Society. Only about 5% of brain tumors may be due to hereditary genetic conditions such as neurofibromatosis, tuberous sclerosis, and a few others.

Depending on how tumor cells in the tumor appear microscopically, the National Cancer Institute (NCI) lists the following grades of brain tumors
**Grade I:** The tissue is benign. The cells look nearly like normal brain cells, and they grow slowly.
**Grade II:** The tissue is malignant. The cells look less like normal cells than do the cells in a grade I tumor.
**Grade III:** The malignant tissue has cells that look very different from normal cells. The abnormal cells are actively growing and have a distinctly abnormal appearance (anaplastic).
**Grade IV:** The malignant tissue has cells that look most abnormal and tend to grow quickly.

Most of the symptoms caused by brain cancer are not specific for brain cancer and include the following ones: Difficulty walking and/or dizziness/vertigo, seizures muscle weakness (for example, arm and leg weakness), headaches (persistent and/or severe). Other common symptoms that may occur include nausea, vomiting, blurry vision, a change in a person's alertness, sleepiness, mental capacity reduction and/or confusion, memory problems, changes in speech, such as difficulty speaking, impaired voice, or inability to speak, personality changes, hallucinations, weakness on one side of the body, coordination problems, fatigue, and pins and needles sensations and/or reduced sensation of touch.

Treatment of brain tumor typically involves surgery, radiation therapy and chemotherapy. Other treatment options include hyperthermia, immunotherapy or steroid therapy. Dependent on the individual brain tumor a combination of two or more of the above options may be realized in the treatment of a subject suffering from brain tumor.

The problem underlying the present invention is the provision of a method for treating a tumor in a subject, preferably said tumor is a brain tumor.

Another problem underlying the present invention is the provision of a method for treating a malignant brain tumor in a subject.

Another problem underlying the present invention is the provision of a method for treating a brain tumor in a subject, wherein the brain tumor is treated or can be treated by radiotherapy.

A further problem underlying the present invention is the provision of a method for treating a brain tumor in a subject, wherein the brain tumor is one which cannot be treated by surgery, typically one which cannot be treated by surgery because of the size of the tumor or its location close to essential neighboring tissues.

These and other problems are solved by the subject matter of the attached independent claim; preferred embodiments may be taken from the attached dependent claims.

Irrespective thereof, the problems underlying the present invention are solved by the subject matter of the following embodiments, whereby Embodiment 1 is also referred to as the first aspect of the present invention.

Embodiment 1. A method for treating a tumor in a subject, wherein the method comprises administering to the subject
- a C-X-C motif chemokine 12 (CXCL12) antagonist,
- a radiotherapy, and
- an anti-angiogenic compound,
wherein the tumor is a brain tumor.

Embodiment 2. The method of embodiment 1, wherein the tumor is selected from the group comprising glioblastoma, glioma, acoustic neuroma, astrocytoma, brain metastases, choroid plexus carcinoma, craniopharyngioma, embryonal tumor, medulloblastoma, meningioma, pediatric brain tumor, pineoblastoma and pituitary tumor.

Embodiment 3. The method of any one of embodiments 1 and 2, wherein the tumor is selected from the group comprising glioblastoma and glioma.

Embodiment 4. The method of embodiment any one of embodiments 1 to 3, wherein the tumor is a grade IV astrocytoma or glioblastoma.

Embodiment 5. The method of embodiment 4, wherein the tumor is supratentorial glioblastoma.

Embodiment 6. The method of any one of embodiments 4 and 5, wherein glioblastoma is glioblastoma with unmethylated O⁶-methylguanine DNA methyltransferase (MGMT) promoter status.

Embodiment 7. The method of any one of embodiments 1 to 6, preferably any one of embodiments 4 to 6, wherein the tumor is non-responsive to temozolomide.

Embodiment 8. The method of any one of embodiments 1 to 7, preferably any one of embodiments 4 to 7, wherein the tumor is expressing CXCL12 and VEGF.

Embodiment 9. The method of embodiment 8, wherein the tumor expresses CXCL12 and VEGF in different compartments of the tumor.

Embodiment 10. The method of any one of embodiments 8 and 9, wherein CXCL12 is expressed in a leading edge of the tumor, in a hyperplastic blood vessel of the tumor and/or in a microvascular proliferation zone of the tumor.

Embodiment 11. The method of any one of embodiments 8 to 10, preferably of embodiment 10, wherein VEGF is expressed in a cellular tumor compartment of the tumor, by a region with pseudopalisading cells of the tumor and/or in a perinecrotic zone of the tumor and wherein preferably the tumor is a glioblastoma.

Embodiment 12. The method of any one of embodiments 1 to 3, wherein the tumor is selected from the group comprising astrocytoma, ependymoma and oligodendroglioma.

Embodiment 13. The method of any one of embodiments 1 to 12, preferably any one of embodiments 4 to 11, wherein the tumor is an incompletely resected tumor.

Embodiment The method of embodiment 13, wherein the tumor is an incompletely resected tumor which is detectable per post-operative T1-weighted, contrast-enhanced MRI scan.

Embodiment 15. The method of any one of embodiments 1 to 14, wherein the CXCL12 antagonist inhibits signaling of CXCL12 via one or both of CXCL12 receptor C-X-C chemokine receptor type 4 (CXCR4) and CXCL12 receptor C-X-C chemokine receptor type 7 (CXCR7).

Embodiment 16. The method of embodiment 15, wherein the CXCL12 antagonist inhibits the binding of CXCL12 to CXCL12 receptor C-X-C chemokine receptor type 4 (CXCR4) and/or to CXCL12 receptor C-X-C chemokine receptor type 7 (CXCR7).

Embodiment 17. The method of embodiment 16, wherein the CXCL12 antagonist is binding to CXCL12.

Embodiment 18. The method of embodiment 17, wherein the CXCL12 antagonist is a nucleic acid molecule binding to CXCL12, wherein the nucleic acid molecule is preferably selected from the group comprising an SDF-1 binding nucleic acid molecule of type B, an SDF-1 binding nucleic acid molecule of type C, an SDF-1 binding nucleic acid molecule of type A and an SDF-1 binding nucleic acid molecule of type D.

Embodiment 19. The method of embodiment 18, wherein the SDF-1 binding nucleic acid molecule of type B comprises a central stretch of nucleotides, whereby the central stretch of nucleotides comprises the following nucleotide sequence:
5' GUGUGAUCUAGAUGUADWGGCUGWUCCUAGUYAGG 3' (SEQ ID NO: 52).

Embodiment 20. The method of embodiment 19, wherein the central stretch of nucleotides comprises the following nucleotide sequence:
5' GUGUGAUCUAGAUGUADUGGCUGAUCCUAGUCAGG 3' (SEQ ID NO: 53).

Embodiment 21. The method of any one of embodiments 19 to 20, wherein the SDF-1 binding nucleic acid molecule of type B comprises in 5'->3' direction a first terminal stretch of nucleotides, the central stretch of nucleotides, and a second terminal stretch of nucleotides.

Embodiment 22. The method of any one of embodiments 19 to 20, wherein the SDF-1 binding nucleic acid molecule of type B comprises in 5'->3' direction a second terminal stretch of nucleotides, the central stretch of nucleotides, and a first terminal stretch of nucleotides.

Embodiment 23. The method of any one of embodiments 21 to 22, wherein the first terminal stretch of nucleotides comprises a nucleotide sequence of 5' X1X2SVNS 3' and the second terminal stretch of nucleotides comprises a nucleotide sequence of 5' BVBSX3X4 3', whereby
X1 is either absent or is A, X2 is G, X3 is C and X4 is either absent or is U;
   or
X1 is absent, X2 is either absent or is G, X3 is either absent or is C and X4 is absent.

Embodiment 24. The method of any one of embodiments 21 to 23, preferably embodiment 50, wherein the first terminal stretch of nucleotides comprises a nucleotide sequence of 5' X1X2CRWG 3' and the second terminal stretch of nucleotides comprises a nucleotide sequence of 5' KRYSX3X4 3',
whereby X1 is either absent or A, X2 is G, X3 is C and X4 is either absent or U.

Embodiment 25. The method of any one of embodiments 21 to 24, preferably embodiment 23 or 24, wherein the first terminal stretch of nucleotides comprises a nucleotide sequence of 5' X1X2CGUG 3' and the second terminal stretch of nucleotides comprises a nucleotide sequence of 5' UACGX3X4 3',
whereby X1 is either absent or A, X2 is G, X3 is C, and X4 is either absent or U,
preferably the first terminal stretch of nucleotides comprises a nucleotide sequence of 5' AGCGUG 3' and the second terminal stretch of nucleotides comprises a nucleotide sequence of 5' UACGCU 3'.

Embodiment 26. The method of any one of embodiments 21 to 23, preferably embodiment 23, wherein the first terminal stretch of nucleotides comprises a nucleotide sequence of 5' X1X2SSBS 3' and the second terminal stretch of nucleotides comprises a nucleotide sequence of 5' BVSSX3X4 3',
whereby X1 is absent, X2 is either absent or G, X3 is either absent or C, and X4 is absent, preferably the first terminal stretch of nucleotides comprises a nucleotide sequence of 5' GCGUG 3' and the second terminal stretch of nucleotides comprises a nucleotide sequence of 5' UACGC 3'.

Embodiment 27. The method of any one of embodiments 18 to 26, wherein the SDF-1 binding nucleic acid molecule of type B comprises a nucleotide sequence according to any one of SEQ ID NO: 5 to SEQ ID NO: 20 and SEQ ID NO: 22 to SEQ ID NO: 28 or a nucleotide sequence at least 80 % identical thereto,
preferably any one of SEQ ID NO: 5 to SEQ ID NO: 7, SEQ ID NO: 16, SEQ ID NO: 22 and SEQ ID NO: 28 or a nucleotide sequence at least 80 % identical thereto,
more preferably any one of SEQ ID NO: 22 and SEQ ID NO: 28 or a nucleotide sequence at least 80 % identical thereto.

Embodiment 28. The method of embodiment 18, wherein the SDF-1 binding nucleic acid molecule of type C comprises a central stretch of nucleotides, whereby the central stretch of nucleotides comprises a nucleotide sequence of GGUYAGGGCUHRX_{A}AGUCGG (SEQ ID NO: 108),
whereby X_{A} is either absent or is A.

Embodiment 29. The method of embodiment 28, wherein the central stretch of nucleotides comprises a nucleotide sequence of 5' GGUYAGGGCUHRAAGUCGG 3' (SEQ ID NO: 109), 5' GGUYAGGGCUHRAGUCGG 3' (SEQ ID NO: 110) or 5' GGUUAGGGCUHGAAGUCGG 3' (SEQ ID NO: 111), preferably 5' GGUUAGGGCUHGAAGUCGG 3' (SEQ ID NO: 111).

Embodiment 30. The method of any one of embodiments 28 to 29, wherein the SDF-1 binding nucleic acid molecule of type C comprises in 5'->3' direction a first terminal stretch of nucleotides, the central stretch of nucleotides, and a second terminal stretch of nucleotides.

Embodiment 31. The method of any one of embodiments 28 to 29, wherein the SDF-1 binding nucleic acid molecule of type C comprises in 5'->3' direction a second terminal stretch of nucleotides, the central stretch of nucleotides, and a first terminal stretch of nucleotides.

Embodiment 32. The method of any one of embodiments 30 to 31, wherein the first terminal stretch of nucleotides comprises a nucleotide sequence of 5' RKSBUSNVGR 3' (SEQ ID NO: 138) and the second stretch of nucleotides comprises a nucleotide sequence of 5' YYNRCASSMY 3' (SEQ ID NO: 139),
preferably the first terminal stretch of nucleotides comprises a nucleotide sequence of 5' RKSBUGSVGR 3' (SEQ ID NO: 140) and the second terminal stretch of nucleotides comprises a nucleotide sequence of 5' YCNRCASSMY 3' (SEQ ID NO: 141).

Embodiment 33. The method of any one of embodiments 30 to 31, wherein the first terminal stretch of nucleotides comprises a nucleotide sequence of 5' XSSSSV 3' and the second terminal stretch of nucleotides comprises a nucleotide sequence of 5' BSSSXS 3', whereby Xs is either absent or is S,
preferably the first terminal stretch of nucleotides comprises a nucleotide sequence of 5' SGGSR 3' and the second terminal stretch of nucleotides comprises a nucleotide sequence of 5' YSCCS 3'.

Embodiment 34. The method of any one of embodiments 30 to 31, wherein
a) the first terminal stretch of nucleotides comprises a nucleotide sequence of 5' GCCGG 3' and the second terminal stretch of nucleotides comprises a nucleotide sequence of 5' CCGGC 3'; or
b) the first terminal stretch of nucleotides comprises a nucleotide sequence of 5' CGUGCGCUUGAGAUAGG 3' (SEQ ID NO: 220) and the second terminal stretch of nucleotides comprises a nucleotide sequence of 5' CUGAUUCUCACG 3' (SEQ ID NO: 221); or
c) the first terminal stretch of nucleotides comprises a nucleotide sequence of 5' UGAGAUAGG 3' and the second terminal stretch of nucleotides comprises a nucleotide sequence of 5' CUGAUUCUCA 3' (SEQ ID NO: 222); or
d) the first terminal stretch of nucleotides comprises a nucleotide sequence of 5' GAGAUAGG 3' and the second terminal stretch of nucleotides comprises a nucleotide sequence of 5' CUGAUUCUC 3'.

Embodiment 35. The method of any one of embodiments 28 to 34, wherein the type C SDF-1 binding nucleic acid molecule comprises a nucleotide sequence according to any one of SEQ ID NO: 95 to SEQ ID NO: 107, SEQ ID NO: 112 to SEQ ID NO: 137, SEQ ID NO: 223 and SEQ ID NO: 224,
preferably any one of SEQ ID NO: 120, SEQ ID NO: 128, SEQ ID NO: 129, SEQ ID NO:134, SEQ ID NO: 135, SEQ ID NO: 223 and SEQ ID NO: 224.

Embodiment 36. The method of embodiment 18, wherein the SDF-1 binding nucleic acid molecule of type A comprises a central stretch of nucleotides, whereby the central stretch of nucleotides comprises a nucleotide sequence of 5' AAAGYRACAHGUMAAX_{A}UGAAAGGUARC 3' (SEQ ID NO: 74),
whereby X_{A} is either absent or is A.

Embodiment 37. The method of embodiment 36, wherein the central stretch of nucleotides comprises a nucleotide sequence of
5'AAAGYRACAHGUMAAUGAAAGGUARC 3' (SEQ ID NO: 75), or
5' AAAGYRACAHGUMAAAUGAAAGGUARC 3' (SEQ ID NO: 76), or
5' AAAGYAACAHGUCAAUGAAAGGUARC 3'(SEQ ID NO: 77), preferably the central stretch of nucleotides comprises a nucleotide sequence of 5' AAAGYAACAHGUCAAUGAAAGGUARC 3' (SEQ ID NO: 77).

Embodiment 38. The method of any one of embodiments 36 to 37, wherein the SDF-1 binding nucleic acid molecule of type A comprises in 5'->3' direction a first terminal stretch of nucleotides, the central stretch of nucleotides, and a second terminal stretch of nucleotides.

Embodiment 39. The method of any one of embodiments 36 to 37, wherein the SDF-1 binding nucleic acid molecule of type A comprises in 5'->3' direction a second terminal stretch of nucleotides, the central stretch of nucleotides, and a first terminal stretch of nucleotides.

Embodiment 40. The method of any one of embodiments 38 to 39, wherein the first terminal stretch of nucleotides comprises a nucleotide sequence of 5' X₁X₂NNBV 3' and the second terminal stretch of nucleotides comprises a nucleotide sequence of 5' BNBNX₃X₄ 3'
whereby X₁ is either absent or R, X₂ is S, X₃ is S and X₄ is either absent or Y;
   or
X₁ is absent, X₂ is either absent or S, X₃ is either absent or S and X₄ is absent.

Embodiment 41. The method of any one of embodiments 38 to 40, preferably 40, wherein the first terminal stretch of nucleotides comprises a nucleotide sequence of 5' RSHRYR 3' and the second terminal stretch of nucleotides comprises a nucleotide sequence of 5' YRYDSY 3',
preferably the first terminal stretch of nucleotides comprises a nucleotide sequence of 5' GCUGUG 3' and the second terminal stretch of nucleotides comprises a nucleotide sequence of 5' CGCAGC 3'.

Embodiment 42. The method of any one of embodiments 38 to 40, preferably 40, wherein the first terminal stretch of nucleotides comprises a nucleotide sequence of 5' X₂BBBS 3' and the second terminal stretch of nucleotides comprises a nucleotide sequence of 5' SBBVX₃ 3',
whereby X₂ is either absent or is S and X₃ is either absent or is S;
preferably the first terminal stretch of nucleotides comprises a nucleotide sequence of 5' CUGUG 3' and the second terminal stretch of nucleotides comprises a nucleotide sequence of 5' CGCAG 3';
or the first terminal stretch of nucleotides comprises a nucleotide sequence of 5' GCGUG 3'and the second terminal stretch of nucleotides comprises a nucleotide sequence of 5' CGCGC 3'.

Embodiment 43. The method of any one of embodiments 36 to 42, wherein the SDF-1 binding nucleic acid molecule of type A comprises a nucleotide sequence according to any one of SEQ ID NO: 60 to SEQ ID NO: 73, SEQ ID NO: 78 to SEQ ID NO: 82, SEQ ID NO: 84 to SEQ ID NO: 87, SEQ ID NO: 89 to SEQ ID NO: 94, and SEQ ID NO: 145 or a nucleotide sequence at least 80 % identical thereto,
preferably any one of SEQ ID NO: 60, SEQ ID NO: 63, SEQ ID NO: 66, SEQ ID NO: 78, SEQ ID NO: 84, and SEQ ID NO: 146 or a nucleotide sequence at least 80 % identical thereto,
more preferably any one of SEQ ID NO: 84 and SEQ ID NO: 146 or a nucleotide sequence at least 80 % identical thereto.

Embodiment 44. The method of embodiment 18, wherein the SDF-1 binding nucleic acid molecule of type D comprises a nucleotide sequence according to any one of SEQ ID NO: 142 to SEQ ID NO: 144 or a nucleotide sequence at least 80 % identical thereto.

Embodiment 45. The method of any one of embodiments 18 to 44, wherein the nucleic acid molecule comprises a modification, whereby the modification is preferably a high molecular weight moiety and/or whereby the modification preferably allows to modify the characteristics of the nucleic acid molecule in terms of residence time in the animal or human body, preferably the human body.

Embodiment 46. The method of embodiment 45, wherein the modification is selected from the group comprising a HES moiety, a PEG moiety, biodegradable modifications and combinations thereof.

Embodiment 47. The method of embodiment 46, wherein the modification is a PEG moiety consisting of a straight PEG or branched PEG, whereby preferably the molecular weight of the straight or branched PEG is from about 20,000 to 120,000 Da, more preferably from about 30,000 to 80,000 Da and most preferably about 40,000 Da.

Embodiment 48. The method of embodiment 46, wherein the modification is a HES moiety, whereby preferably the molecular weight of the HES moiety is from about 10,000 to 200,000 Da, more preferably from about 30,000 to 170.000 Da and most preferably about 150,000 Da.

Embodiment 49. The method of any one of embodiments of 45 to 48, wherein the modification is attached to the nucleic acid molecule via a linker, wherein preferably the linker is a biostable or biodegradable linker.

Embodiment 50. The method of any one of embodiments of 45 to 49, wherein the modification is attached to the nucleic acid molecule at the 5'-terminal nucleotide of the nucleic acid molecule and/or the 3'-terminal nucleotide of the nucleic acid molecule and/or to a nucleotide of the nucleic acid molecule between the 5'-terminal nucleotide of the nucleic acid molecule and the 3'-terminal nucleotide of the nucleic acid molecule

Embodiment 51. The method of any one of embodiments 18 to 50, wherein the nucleotides of the nucleic acid molecule or the nucleotides forming the nucleic acid molecule are L-nucleotides.

Embodiment 52. The method of any one of embodiments 18 to 51, whereby the nucleic acid molecule is an L-nucleic acid molecule.

Embodiment 53. The method of embodiment 18, wherein the nucleic acid molecule is an L-ribonucleic acid molecule comprising a nucleotide sequence of
GCGUGGUGUGAUCUAGAUGUAUUGGCUGAUCCUAGUCAGGUACGC (SEQ ID NO: 22).

Embodiment 54. The method of embodiment 53, wherein the L-ribonucleic acid molecule comprises a 40 kDa polyethylene glycol moiety, wherein the 40 kDa polyethylene glycol moiety is attached to the 5'end of nucleotide sequence.

Embodiment 55. The method of embodiment 54, wherein N-[ω-methoxypoly(oxy-1,2-ethanediyl], N'-[ω-methoxypoly(oxy-1,2-ethanediyl]-acetyl-9-amino-8-oxo-7-aza-nonyloxy phosphate is interspersed between the 5' terminal nucleotide and the 40 kDa polyethylene glycol moiety.

Embodiment 56. The method of embodiment 55, wherein the L-ribonucleic acid molecule is present as a sodium salt, preferably the L-nucleic acid molecule is NOX-A12.

Embodiment 57. The method of any one of embodiments 1 to 16, wherein the CXCL12 antagonist is an aptamer binding to CXCL12.

Embodiment 58. The method of any one of embodiments 1 to 16, wherein the CXCL12 antagonist is an antibody binding to CXCL12.

Embodiment 59. The method of embodiment 58, wherein the antibody binding to CXCL12 is mAB-30D8, 1131-H12 or 1143-H1.

Embodiment 60. The method of any one of embodiments 1 to 16, wherein the CXCL12 antagonist is a CXCL12 binding protein.

Embodiment 61. The method of embodiment 60, wherein the protein is a TRAP protein or an anticalin.

Embodiment 62. The method of any one of embodiments 1 to 16, wherein the CXCL12 antagonist targets CXCR4.

Embodiment 63. The method of embodiment 62, wherein the CXCL12 antagonist is binding to CXCR4.

Embodiment 64. The method of any one of embodiments 62 to 63, wherein the CXCR4 binding CXCL12 antagonist is selected from the group comprising a small molecule, a peptide, a synthetic peptide, a recombinant protein, a fusion protein and an antibody.

Embodiment 65. The method of embodiment 64, wherein the CXCR4 binding CXCL12 antagonist is a small molecule.

Embodiment 66. The method of embodiment 65, wherein the CXCR4 binding CXCL12 antagonist is a small molecule selected from the group comprising plerixafor, mavorixafor, Q-122, HPH-112, BKT-300, X4P-002, X4P-003, X4-136, GP-01CR01, GP-01CR11, GP-01CR21, Pentixather, BKT-170, BMS-585248, CS-3955, CTCE-0012, CTCE-9908, GBV-4086, HPH-112, HPH-211, NB-325, SP-10 and USL-311.

Embodiment 67. The method of embodiment 66, wherein the small molecule is selected from the group comprising plerixafor, mavorixafor, Q-122, HPH-112, BKT-300, X4P-002, X4P-003, X4-136, GP-01CR01, GP-01CR11, GP-01CR21, and pentixather.

Embodiment 68. The method of embodiment 64, wherein the CXCR4 binding CXCL12 antagonist is a peptide.

Embodiment 69. The method of embodiment 68, wherein the CXCR4 binding CXCL12 antagonist is motixafortide.

Embodiment 70. The method of embodiment 64, wherein the CXCR4 binding CXCL12 antagonist is a synthetic peptide.

Embodiment 71. The method of embodiment 70, wherein the CXCR4 binding CXCL12 antagonist is a synthetic peptide selected from the group comprising balixafortide, LY-2510924, ALB-408 and POL-5551.

Embodiment 72. The method of embodiment 71, wherein the CXCR4 binding CXCL12 antagonist is selected from the group comprising balixafortide, and LY-2510924.

Embodiment 73. The method of embodiment 64, wherein the CXCR4 binding CXCL12 antagonist is a recombinant protein.

Embodiment 74. The method of embodiment 73, wherein the recombinant protein is selected from the group comprising PTX-9098 and NNL-121.

Embodiment 75. The method of embodiment 64, wherein the CXCR4 binding CXCL12 antagonist is a fusion protein.

Embodiment 76. The method of embodiment 75, wherein the fusion protein is selected from the group comprising AD-214 and AM-3114.

Embodiment 77. The method of embodiment 64, wherein the CXCR4 binding CXCL12 antagonist is an antibody or an antigen-binding fragment of the antibody.

Embodiment 78. The method of embodiment 77, wherein the antibody is a monoclonal antibody.

Embodiment 79. The method of any one of embodiments 77 and 78, wherein the CXCR4 binding CXCL12 antagonist is an antibody.

Embodiment 80. The method of embodiment 79, wherein the antibody is a monoclonal antibody selected from the group comprising ulocuplumab, hz-515H7, KY-1051, PF-06747143, ALX-0651, AT-009, LY-2624587 and STIA-220X.

Embodiment 81. The method of embodiment 80, wherein the antibody is a monoclonal antibody selected from the group comprising ulocuplumab, hz-515H7 and KY-1051.

Embodiment 82. The method of any one of embodiments 1 to 16, wherein the CXCL12 antagonist is binding to CXCR7.

Embodiment 83. The method of embodiment 82, wherein the CXCR7 binding CXCL12 antagonist is selected from the group comprising a small molecule, a synthetic peptide and an antibody.

Embodiment 84. The method of embodiment 83, wherein the CXCR7 binding CXCL12 antagonist is a small molecule.

Embodiment 85. The method of embodiment 84, wherein the CXCR7 binding CXCL12 antagonist is a small molecule is a small molecule selected from the group comprising ACT-10041239, CCX-650, CCX-662 and CCX-771.

Embodiment 86. The method of embodiment 85, wherein the CXCR7 binding CXCL12 antagonist is ACT-10041239.

Embodiment 87. The method of embodiment 83, wherein the CXCR7 binding CXCL12 antagonist is a synthetic peptide.

Embodiment 88. The method of embodiment 87, wherein the synthetic peptide is selected from the group comprising LIH-383 and POL-6926.

Embodiment 89. The method of embodiment 88, wherein the synthetic peptide is LIH-383.

Embodiment 90. The method of embodiment 83, wherein the CXCR7 binding CXCL12 antagonist is an antibody or an antigen-binding fragment of the antibody.

Embodiment 91. The method of embodiment 90, wherein the antibody is a monoclonal antibody.

Embodiment 92. The method of any one of embodiments 90 and 91, wherein the antibody is a monoclonal antibody selected from the group comprising JT-07, X-7Ab and STIA-230X.

Embodiment 93. The method of embodiment 92, wherein the antibody is a monoclonal antibody selected from the group comprising JT-07 and X-7Ab.

Embodiment 94. The method of any one of embodiments 17 to 93, preferably any one of embodiments 17 to 56, wherein an amount of the CXCL12 antagonist is administered to the subject, wherein the amount is effective in inhibiting neovascularization of the tumor.

Embodiment 95. The method of embodiment 94, wherein neovascularization is effected by CXCL12-mediated recruiting of endothelial cells or bone marrow-derived pro-angiogenic cells.

Embodiment 96. The method of any one of embodiments 94 to 95, wherein the CXCL12-mediated recruiting is dependent on CXCR4 and/or CXCR7.

Embodiment 97. The method of any one of embodiments 1 to 96, preferably any one of embodiments 17 to 57, wherein the radiotherapy makes use of ionizing electromagnetic radiation or a particle beam.

Embodiment 98. The method of embodiment 97, wherein the electromagnetic radiation is selected from the group comprising X-rays and gamma-rays.

Embodiment 99. The method of embodiment 98, wherein the X-rays have a wavelength from about 10 picometers to about 10 nanometers.

Embodiment 100. The method of any one of embodiments 98 to 99, wherein the energy of the X-rays is from about 145 eV to about 124 keV.

Embodiment 101. The method of embodiment 98, wherein the gamma-rays have a wavelength of 10 picometer of less.

Embodiment 102. The method of embodiment 101, wherein the energy of the gamma-rays is 100 keV or more.

Embodiment 103. The method of embodiment 97, wherein the radiotherapy makes use of a particle beam.

Embodiment 104. The method of embodiment 103, wherein the particle beam is a particle beam selected from the group comprising a proton beam, a photon beam and an electron beam.

Embodiment 105. The method of any one of embodiments 97 to 104, wherein the radiotherapy makes use of intensity-modulated radiation therapy (IMRT), tomotherapy which is also referred to as image-guided radiation therapy (IGRT), and/or stereotactic radiosurgery.

Embodiment 106. The method of any one of embodiments 97 to 105, wherein radiotherapy is administered by means of external radiotherapy, preferably external beam radiotherapy.

Embodiment 107. The method of any one of embodiments 97 to 106, wherein radiotherapy is administered by means of brachytherapy.

Embodiment 108. The method of embodiment 107, wherein brachytherapy is provided by means of a low-dose rate (LDH) implant, a high-dose rate (HDR) implant, a permanent implant, a formulation containing a therapeutically active radionuclide, preferably a liquid formulation, or a combination thereof.

Embodiment 109. The method of any one of embodiments 97 to 108, wherein the radiotherapy targets cells of the tumor or is directed towards cells of the tumor.

Embodiment 110. The method of any one of embodiments 97 to 109, wherein the radiotherapy targets or is directed towards microvasculature of the tumor.

Embodiment 111. The method of embodiment 110, wherein the radiotherapy depletes microvasculature of the tumor.

Embodiment 112. The method of any one of embodiments 97 to 111, wherein radiotherapy generates intratumoral hypoxia.

Embodiment 113. The method of any one of embodiments 97 to 112, preferably embodiment 112, wherein the tumor expresses CXCL12 upon radiotherapy.

Embodiment 114. The method of any one of embodiments 97 to 113, preferably any one of embodiments 112 to 113, wherein the tumor is revascularized upon radiotherapy.

Embodiment 115. The method of any one of embodiments 97 to 114, preferably any one of embodiments 112 to 114, wherein radiotherapy results in immunosuppression.

Embodiment 116. The method of any one of embodiments 97 to 115, wherein a total absorbed radiation dose of the radiotherapy is from about 10 Gy to about 100 Gy, preferably from about 40 Gy to about 60 Gy, more preferably about 40 Gy or about 60 Gy.

Embodiment 117. The method of embodiment 116, wherein the radiotherapy is a normofractionated radiotherapy.

Embodiment 118. The method of embodiment 116, wherein the radiotherapy is administered as hypofractionated radiotherapy.

Embodiment 119. The method of embodiment 118, wherein the individual absorbed radiation of the fractionated radiotherapy is from about 0.5 Gy to about 5 Gy, preferably from about 2 Gy to about 2.7 Gy, more preferably about 2 Gy or about 2.7 Gy.

Embodiment 120. The method of any one of embodiments 97 to 119, wherein the radiotherapy comprises intraoperative radiotherapy (IORT).

Embodiment 121. The method of any one of embodiments 1 to 120, wherein the antiangiogenic compound inhibits angiogenesis.

Embodiment 122. The method of embodiment 121, wherein angiogenesis is the formation of a new blood vessel from a pre-existing blood vessel.

Embodiment 123. The method of any one of embodiments 121 and 122, wherein the antiangiogenic compound inhibits angiogenesis towards and/or in the tumor.

Embodiment 124. The method of any one of embodiments 121 to 123, wherein the antiangiogenic compound inhibits growth of the tumor.

Embodiment 125. The method of any one of embodiments 121 to 124, wherein the antiangiogenic compound is inhibiting the interaction between a vascular endothelial growth factor (VEGF) and a VEGF receptor.

Embodiment 126. The method of embodiment 125, wherein the VEGF is human VEGF including any isoform thereof and any proteolytically cleaved form thereof.

Embodiment 127. The method of embodiment 126, wherein the isoform is selected from the group comprising VEGF121, VEGF165, VEGF189, VEGF206, VEGF145, VEGF183 and VEGF165b, preferably the isoform is selected from the group comprising VEGF121, VEGF165, VEGF189, VEGF206 and VEGF 145.

Embodiment 128. The method of embodiment 126, wherein the proteolytically cleaved form of VEGF is VEGF110.

Embodiment 129. The method of any one of embodiments 125 to 128, wherein the antiangiogenic compound is inhibiting the interaction between a VEGF receptor and at least one of human vascular endothelial growth factor (VEGF), an isoform of VEGF and a proteolytically cleaved form of VEGF or of an isoform of VEGF.

Embodiment 130. The method of any one of embodiments 125 to 129, wherein the VEGF receptor is selected from the group comprising VEGFR1 and VEGFR2.

Embodiment 131. The method of embodiment 130, wherein the VEGF receptor is VEGFR1.

Embodiment 132. The method of any one of embodiments 125 to 131, wherein the antiangiogenic compound is binding to VEGF.

Embodiment 133. The method of embodiment 132, wherein the anti-angiogenic compound binding to VEGF is selected from the group comprising an anti-VEGF antibody, a fragment of an anti-VEGF antibody, a VEGF-binding protein and a VEGF-binding nucleic acid molecule.

Embodiment 134. The method of embodiment 133, wherein the anti-angiogenic compound is an anti-VEGF antibody.

Embodiment 135. The method of embodiment 134, wherein the anti-VEGF antibody is a human anti-VEGF antibody, a humanized anti-VEGF antibody or a human anti-VEGF antibody.

Embodiment 136. The method of any one of embodiments 134 and 135, wherein the anti-VEGF antibody comprises the following CDRs of the light chain variable region: CDR1: QDISNY, CDR2: FTS, and CDR3: QQYSTVPWT.

Embodiment 137. The method of any one of embodiments 134 to 136, wherein the anti-VEGF antibody comprises the following CDRs of the heavy chain variable region: CDR1: GYTFTNYG, CDR2: INTYTGEP, and CDR3: AKYPHYYGSSHWYFDV.

Embodiment 138. The method of any one of embodiments 136 and 137, wherein the antibody comprises the following CDRs of the light chain variable region CDR1: QDISNY, CDR2: FTS, and CDR3: QQYSTVPWT, and the following CDRs of the heavy chain variable region CDR1: GYTFTNYG, CDR2: INTYTGEP, and CDR3: AKYPHYYGSSHWYFDV.

Embodiment 139. The method of any one of embodiments 134 to 138, wherein the antibody comprises a light chain comprising the following amino acid sequence:

DIQMTQSPSSLSASVGDRVTITCSASQDISNYLNWYQQKPGKAPKVLIYFTSSLHSGVPSRFSG
SGSGTDFTLTISSLQPEDFATYYCQQYSTVPWTFGQGTKVEIKRTVAAPSVFIFPPSDEQLKSG
TASVVCLLNNFYPREAKVQWKVDNALQSGNSQESVTEQDSKDSTYSLSSTLTLSKADYEKH
KVYACEVTHQGLSSPVTKSFNRGEC.

Embodiment 140. The method of any one of embodiments 134 to 139, wherein the antibody comprises a heavy chain comprising the following amino acid sequence:

EVQLVESGGGLVQPGGSLRLSCAASGYTFTNYGMNWVRQAPGKGLEWVGWINTYTGEPTY
AADFKRRFTFSLDTSKSTAYLQMNSLRAEDTAVYYCAKYPHYYGSSHWYFDVWGQGTLVT
VSSASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGGPSVF
LFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSREEMTKNQVS
LTCLVKGFYPSDIAVEWESNGQPENNYKTTPPVLDSDGSFFLYSKLTVDKSRWQQGNVFSCS
VMHEALHNHYTQKSLSLSPGK.

Embodiment 141. The method of any one of embodiments 134 to 140, wherein the antibody comprises a light chain comprising the following amino acid sequence: 
DIQMTQSPSSLSASVGDRVTITCSASQDISNYLNWYQQKPGKAPKVLIYFTSSLHSGVPSRFSG
SGSGTDFTLTISSLQPEDFATYYCQQYSTVPWTFGQGTKVEIKRTVAAPSVFIFPPSDEQLKSG
TASVVCLLNNFYPREAKVQWKVDNALQSGNSQESVTEQDSKDSTYSLSSTLTLSKADYEKH
KVYACEVTHQGLSSPVTKSFNRGEC
 and a heavy chain comprising the following amino acid sequence

EVQLVESGGGLVQPGGSLRLSCAASGYTFTNYGMNWVRQAPGKGLEWVGWINTYTGEPTY
AADFKRRFTFSLDTSKSTAYLQMNSLRAEDTAVYYCAKYPHYYGSSHWYFDVWGQGTLVT
VSSASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGGPSVF
LFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSREEMTKNQVS
LTCLVKGFYPSDIAVEWESNGQPENNYKTTPPVLDSDGSFFLYSKLTVDKSRWQQGNVFSCS
VMHEALHNHYTQKSLSLSPGK.

Embodiment 142. The method of any one of embodiments 134 to 141, wherein the anti-VEGF antibody is a IgGlk isotype antibody.

Embodiment 143. The method of any one of embodiments 134 to 142, wherein the anti-VEGF antibody is Bevacizumab.

Embodiment 144. The method of embodiment 143, wherein the anti-VEGF antibody is an Fab fragment of Bevacizumab.

Embodiment 145. The method of embodiment 144, wherein the Fab fragment of Bevacizumab is Ranibizumab.

Embodiment 146. The method of embodiment 133, wherein the anti-angiogenic compound is a VEGF binding protein.

Embodiment 147. The method of embodiment 146, wherein the VEGF binding protein is a functional derivative of a VEGF receptor.

Embodiment 148. The method of embodiment 147, wherein the VEGF receptor is selected from the group comprising VEGFR1 and VEGFR2.

Embodiment 149. The method of any one of embodiments 147 and 148, wherein the functional derivative of a VEGF receptor comprises at least one VEGF-binding domains of VEGFR1 and/or VEGFR2.

Embodiment 150. The method of any one of embodiments 147 to 149, wherein the VEGF binding protein is ziv aflibercerpt.

Embodiment 151. The method of embodiment 146, wherein the VEGF binding protein is a VEGF binding anticalin.

Embodiment 152. The method of embodiment 133, wherein the anti-angiogenic compound binding to VEGF is a VEGF-binding nucleic acid molecule.

Embodiment 153. The method of embodiment 152, wherein the VEGF-binding nucleic acid molecule is a VEGF-binding D-nucleic acid molecule.

Embodiment 154. The method of embodiment 153, wherein the VEGF-binding D-nucleic acid molecule is a VEGF-binding aptamer.

Embodiment 155. The method of embodiment 154, wherein the VEGF-binding D-nucleic acid molecule is pegaptanib (Macugen), preferably binding to VEGF isoform VEGF165.

Embodiment 156. The method of embodiment 152, wherein the VEGF-binding nucleic acid molecule is a VEGF-binding L-nucleic acid molecule.

Embodiment 157. The method of embodiment 156, wherein the VEGF-binding nucleic acid molecule is a VEGF-binding spiegelmer.

Embodiment 158. The method of any one of embodiments 125 to 131, wherein the antiangiogenic compound is binding to a VEGFR.

Embodiment 159. The method of embodiment 158, wherein the VEGFR is VEGFR1, VEGFR2 or both VEGFR1 and VEGFR2, preferably the VEGFR is VEGFR2.

Embodiment 160. The method of any one of embodiment 158 to 159, wherein the antiangiogenic compound binding to VEGF is selected from the group comprising an anti-VEGFR antibody, a fragment of an anti-VEGFR antibody, a VEGFR-binding protein and a VEGFR-binding nucleic acid molecule.

Embodiment 161. The method of embodiment 160, wherein the anti-angiogenic compound is an anti-VEGFR antibody.

Embodiment 162. The method of embodiment 161, wherein the anti-VEGFR antibody is a human anti-VEGFR antibody, a humanized anti-VEGFR antibody or a human anti-VEGFR antibody.

Embodiment 163. The method of any one of embodiments 161 to 162, wherein the anti-VEGFR antibody comprises the following CDRs of the light chain variable region: CDR1: RASQGIDNWLG, CDR2: DASNLDT, CDR3: QQAKAFPPT.

Embodiment 164. The method of any one of embodiments 161 to 163, wherein the anti-VEGFR antibody comprises the following CDRs of the heavy chain variable region: CDR1: GFTFS SYSMN, CDR2: S ISSSSSYIYY ADSVKG and CDR3: VTDAFDI.

Embodiment 165. The method of any one of embodiments 161 to 164, wherein the anti-VEGFR antibody comprises the following CDRs of the light chain variable region: CDR1: RASQGID NWLG, CDR2: DASNLD, CDR3: QQAKAFPPT; and the following CDRs of the heavy chain variable region CDR1: GFTFS SYSMN, CDR2: S ISSSSSYIYY ADSVKG and CDR3: VTDAFDI.

Embodiment 166. The method of any one of embodiments 161 to 165, wherein the anti-VEGFR antibody comprises a light chain comprising the following amino acid sequence:

DIQMTQSPSS VSASIGDRVT ITCRASQGID NWLGWYQQKP GKAPKLLIYD ASNLDTGVPS
RFSGSGSGTY FTLTISSLQA EDFAVYFCQQ AKAFPPTFGG GTKVDIKRTV AAPSVFIFPP
SDEQLKSGTA SVVCLLNNFY PREAKVQWKV DNALQSGNSQ ESVTEQDSKD STYSLSSTLT
LSKADYEKHK VYACEVTHQG LSSPVTKSFN RGEC.

Embodiment 167. The method of any one of embodiments 161 to 166, wherein the anti-VEGFR antibody comprises a heavy chain comprising the following amino acid sequence:

EVQLVQSGGG LVKPGGSLRL SCAASGFTFS SYSMNWVRQA PGKGLEWVSS ISSSSSYIYY
ADSVKGRFTI SRDNAKNSLY LQMNSLRAED TAVYYCARVT DAFDIWGQGT MVTVSSASTK
GPSVLPLAPS SKSTSGGTAA LGCLVKDYFP EPVTVSWNSG ALTSGVHTFP AVLQSSGLYS
LSSVVTVPSS SLGTQTYICN VNHKPSNTKV DKRVEPKSCD KTHTCPPCPA PELLGGPSVF
LFPPKPKDTL MISRTPEVTC VVVDVSHEDP EVKFNWYVDG VEVHNAKTKP REEQYNSTYR
VVSVLTVLHQ DWLNGKEYKC KVSNKALPAP IEKTISKAKG QPREPQVYTL PPSREEMTKN
QVSLTCLVKG FYPSDIAVEW ESNGQPENNY KTTPPVLDSD GSFFLYSKLT VDKSRWQQGN
VFSCSVMHEA LHNHYTQKSL SLSPGK.

Embodiment 168. The method of any one of embodiments 161 to 167, wherein the anti-VEGFR antibody comprises a light chain comprising the following amino acid sequence: 
DIQMTQSPSS VSASIGDRVT ITCRASQGID NWLGWYQQKP GKAPKLLIYD ASNLDTGVPS
RFSGSGSGTY FTLTISSLQA EDFAVYFCQQ AKAFPPTFGG GTKVDIKRTV AAPSVFIFPP
SDEQLKSGTA SVVCLLNNFY PREAKVQWKV DNALQSGNSQ ESVTEQDSKD STYSLSSTLT
LSKADYEKHK VYACEVTHQG LSSPVTKSFN RGEC;
 and a heavy chain comprising the following amino acid sequence:

EVQLVQSGGG LVKPGGSLRL SCAASGFTFS SYSMNWVRQA PGKGLEWVSS ISSSSSYIYY
ADSVKGRFTI SRDNAKNSLY LQMNSLRAED TAVYYCARVT DAFDIWGQGT MVTVSSASTK
GPSVLPLAPS SKSTSGGTAA LGCLVKDYFP EPVTVSWNSG ALTSGVHTFP AVLQSSGLYS
LSSVVTVPSS SLGTQTYICN VNHKPSNTKV DKRVEPKSCD KTHTCPPCPA PELLGGPSVF

LFPPKPKDTL MISRTPEVTC VVVDVSHEDP EVKFNWYVDG VEVHNAKTKP REEQYNSTYR
VVSVLTVLHQ DWLNGKEYKC KVSNKALPAP IEKTISKAKG QPREPQVYTL PPSREEMTKN
QVSLTCLVKG FYPSDIAVEW ESNGQPENNY KTTPPVLDSD GSFFLYSKLT VDKSRWQQGN
VFSCSVMHEA LHNHYTQKSL SLSPGK.

Embodiment 169. The method of any one of embodiments 161 to 168, wherein the anti-VEGFR antibody is an IgG isotype antibody.

Embodiment 170. The method of any one of embodiments 161 to 169, wherein the anti-VEGFR antibody is Ramucirumab (Cyramza).

Embodiment 171. The method of embodiment 160, wherein the anti-angiogenic compound is a VEGFR binding protein.

Embodiment 172. The method of embodiment 171, wherein the VEGFR binding protein is a VEGFR binding anticalin.

Embodiment 173. The method of embodiment 160, wherein the anti-angiogenic compound binding to VEGFR is a VEGFR-binding nucleic acid molecule.

Embodiment 174. The method of embodiment 173, wherein the VEGFR-binding nucleic acid molecule is a VEGFR-binding D-nucleic acid molecule.

Embodiment 175. The method of embodiment 174, wherein the VEGFR-binding D-nucleic acid molecule is a VEGFR-binding aptamer.

Embodiment 176. The method of embodiment 152, wherein the VEGFR-binding nucleic acid molecule is a VEGFR-binding L-nucleic acid molecule.

Embodiment 177. The method of embodiment 176, wherein the VEGF-binding nucleic acid molecule is a VEGFR-binding spiegelmer.

Embodiment 178. The method of any one of embodiments 121 to 124, wherein the antiangiogenic compound inhibits signaling of a VEGFR.

Embodiment 179. The method of embodiment 178, wherein the VEGFR is VEGFR1, VEGFR2, VEGFR3 and any combination thereof.

Embodiment 180. The method of any one of embodiments 178 to 179, wherein the antiangiogenic compound inhibits the intracellular signaling of the VEGFR.

Embodiment 181. The method of any one of embodiments 178 to 180, wherein the antiangiogenic compound inhibits the signal cascade triggered upon binding of VEGF to the VEGFR.

Embodiment 182. The method of any one of embodiments 180 and 181, wherein the antiangiogenic compound is a tyrosine kinase inhibitor.

Embodiment 183. The method of embodiment 182, wherein the tyrosine kinase inhibitor is a multi-tyrosine kinase inhibitor.

Embodiment 184. The method of any one of embodiments 182 to 183, wherein the tyrosine kinase inhibitor is selected from the group comprising pazopanib (Votrient), sunitinib (Sutent), sorafenib (Nexavar), axitinib (Inlyta), ponatinib (Iclusig), cabozantinib (Cometriq/Cabometyx), regorafenib (Stivarga), vandetanib (Caprelsa) and lenvatinib (Lenvima).

Embodiment 185. The method of any one of embodiments 1 to 184, wherein the method further comprises administering a chemotherapy to the subject.

Embodiment 186. The method of embodiment 185, wherein the chemotherapy is a chemotherapy for the treatment of the cancer.

Embodiment 187. The method of any one of embodiments 185 to 186, wherein the tumor is glioblastoma and the chemotherapy is a chemotherapy for the treatment of glioblastoma.

Embodiment 188. The method of any one of embodiments 185 to 187, wherein the chemotherapy is selected from the group comprising temozolomide, lomustine, irinotecan, dianhydrogalactitol, a combination of irinotecan and bevacizumab, and a combination of procarbazine, lomustine and vincristine (PCV).

Embodiment 189. The method of embodiment 188, wherein the method comprises administering temozolomide to the subject.

Embodiment 190. The method of any one of embodiments 1 to 189, wherein the CXCL12 antagonist is intravenously or subcutaneously administered to the subject, preferably if the CXCL12 antagonist is a spiegelmer or an aptamer, more preferably a spiegelmer.

Embodiment 191. The method of any one of embodiments 1 to 190, preferably embodiment 190, wherein the CXCL12 antagonist is continuously administered to the subject over a period of time.

Embodiment 192. The method of embodiment 191, wherein the period of time is one week, two weeks, three weeks, four weeks, five weeks, six weeks or more.

Embodiment 193. The method of any one of embodiments 1 to 192, preferably any one of embodiments 190 to 192, wherein the CXCL12 antagonist is a CXCL12-binding nucleic acid molecule comprising a 40 kDa PEG moiety.

Embodiment 194. The method of embodiment 193, wherein the CXCL12 antagonist is NOX-A12.

Embodiment 195. The method of any one of embodiments 193 to 194, wherein the weekly dose of the CXCL12 antagonist administered to the subject is between about 100 mg and about 1,000 mg, preferably between about 200 mg and about 600 mg, more preferably between about 400 and about 600 mg.

Embodiment 196. The method of any one of embodiments 1 to 195, preferably any one of embodiments 190 to 195, wherein the anti-angiogenic compound is intravenously, subcutaneously or orally administered to the subject, preferably the anti-angiogenic compound is intravenously administered, more preferably intravenously administered if the anti-angiogenic compound is bevacizumab.

Embodiment 197. The method of any one of embodiments 1 to 196, preferably any one of embodiment 190 to 196, wherein the anti-angiogenic compound is administered weekly, biweekly or threeweekly over a period of time.

Embodiment 198. The method of embodiment 197, wherein the period of time is one week, two weeks, three weeks, four weeks, five weeks or six weeks.

Embodiment 199. The method of any one of embodiments 197 to 198, wherein
(a) the anti-angiogenic compound is administered weekly and the period of time is one week, two weeks, three weeks, four weeks, five weeks or six weeks,
(b) the anti-angiogenic compound is administered biweekly and the period of time is two weeks, four weeks, or six weeks, or
(c) the anti-angiogenic compound is administered threeweekly and the period of time is three weeks or six weeks.

Embodiment 200. The method of any one of embodiments 1 to 199, preferably any one of embodiments 190 to 199, wherein the anti-angiogenic compound is an anti-VEGF antibody, preferably an IgG antibody, and more preferably Bevacizumab.

Embodiment 201. The method of any one of embodiments 1 to 200, preferably any one of embodiments 190 to 200, wherein
(a) if the anti-angiogenic compound is administered weekly, the dose is 5 mg/kg body weight of the subject,
(b) if the anti-angiogenic compound is administered biweekly, the dose is 10 mg/kg body weight of the subject, or
(c) if the anti-angiogenic compound is administered threeweekly, the dose is 15 mg/kg body weight of the subject.

Embodiment 202. The method of any one of embodiments 1 to 201, preferably any one of embodiments 190 to 201, wherein the chemotherapy is administered to the subject.

Embodiment 203. The method of any one of embodiments 1 to 202, preferably any one of embodiments 190 to 202, wherein the chemotherapy is administered daily to the subject.

Embodiment 204. The method of any one of embodiments 1 to 203, preferably any one of embodiments 190 to 203, wherein the chemotherapy is administered to the subject over a period of time, preferably the period of time is one week, two weeks, three weeks, four weeks, five weeks or six weeks, more preferably the chemotherapy is administered to the subject daily over the period of time. Embodiment 205. The method of any one of embodiments 1 to 204, preferably any one of embodiments 190 to 203, more preferably any one of embodiments 202 to 204, wherein the chemotherapy comprises administering to the subject temozolomide.

Embodiment 206. The method of any one of embodiments 1 to 205, preferably any one of embodiments 190 to 205, wherein the period of time over which the chemotherapy is administered to the subject is the period of time over which the radiotherapy is administered to the subject.

Embodiment 207. The method of any one of embodiments 190 to 206, preferably any one of embodiments 202 to 206, wherein the daily dose of temozolomide administered to the subject is about 75 mg/m² body surface.

Embodiment 208. The method of any one of embodiments 1 to 207, preferably any one of embodiments 190 to 207, wherein the radiotherapy is administered to the subject on several days of a week over a period of time.

Embodiment 209. The method of embodiment 208, wherein the radiotherapy is administered to the subject on five consecutive days of one week over a period of time.

Embodiment 210. The method of any one of embodiments 208 to 209, wherein the period of time is one week, two weeks, three weeks, four weeks, five weeks or six weeks.

Embodiment 211. The method of any one of embodiments 208 to 210, wherein the radiotherapy is fractionated radiotherapy

Embodiment 212. The method of any one of embodiments 208 to 211, wherein the absorbed radiation dose is about 2 Gy or about 2.7 Gy per session, preferably there are five consecutive daily sessions within one week.

Embodiment 213. The method of any one of embodiments 191 to 212, wherein the period of time constitutes a first phase of treatment.

Embodiment 214. The method of embodiment 213, wherein in the first phase of treatment
- the CXCL12 antagonist is NOX-A12 which is administered to the subject intravenously, continuously over a period of time of six weeks, wherein the weekly dose administered to the subject is about 200 mg/kg body weight of the subject, about 400 mg/kg body weight of the subject, about 600 mg/kg body weight of the subject or about 800 mg/kg body weight of the subject,
- the antiangiogenic compound is Bevacizumab which is administered to the subject intravenously, biweekly over a period of time of six weeks, wherein the biweekly dose administered to the subject is 10 mg/kg body weight of the subject, and
- the radiotherapy is normofractionated radiotherapy which is administered to the subject on five consecutive days of a week over a period of time of six weeks, wherein the daily absorbed radiation dose is about 2 Gy or
   the radiotherapy is hypofractionated radiotherapy which is administered to the subject on five consecutive days of a week over a period of time of three weeks, wherein the daily absorbed radiation dose is about 2.7 Gy.

Embodiment 215. The method of embodiment 214, wherein in the first phase of treatment a chemotherapy is administered to the subject, wherein the chemotherapy is temozolomide which is orally or intravenously administered to the subject continuously over a period of time of six weeks, wherein the daily dose of temozolomide administered to the subject is 75 mg/m² surface area of the subject.

Embodiment 216. The method of any one of embodiments 213 to 215, wherein a second phase of treatment follows the first phase of treatment.

Embodiment 217. The method of embodiment 216, wherein in the second phase of treatment the CXCL12 antagonist is intravenously administered to the subject.

Embodiment 218. The method of embodiment 217, wherein the CXCL12 antagonist is continuously administered to the subject over a second period of time.

Embodiment 219. The method of embodiment 218, wherein the second period of time is any period of time of 1 weeks to 20 weeks or a period of time longer than 20 weeks.

Embodiment 220. The method of any one of embodiments 216 to 219, wherein the CXCL12 antagonist is a CXCL12-binding nucleic acid molecule comprising a 40 kDa PEG moiety. Embodiment 221. The method of embodiment 220, wherein the CXCL12 antagonist is NOX-A12.

Embodiment 222. The method of any one of embodiments 220 to 221, wherein the weekly dose of the CXCL12 antagonist administered to the subject is about 200 mg, about 400 mg, about 600 mg or about 800 mg.

Embodiment 223. The method of any one of embodiments 216 to 222, wherein the antiangiogenic compound is intravenously administered to the subject.

Embodiment 224. The method of any one of embodiments 216 to 223, wherein the antiangiogenic compound is administered weekly, biweekly or threeweekly over a second period of time.

Embodiment 225. The method of embodiment 224, wherein the second period of time is any period of time of 1 weeks to 20 weeks or a period of time longer than 20 weeks.

Embodiment 226. The method of any one of embodiments 224 to 225, wherein
(a) the anti-angiogenic compound is administered weekly and the second period of time is a multiple of one week,
(b) the anti-angiogenic compound is administered biweekly and the second period of time is a multiple of two weeks, or
(c) the anti-angiogenic compound is administered threeweekly and the second period of time is a multiple of three weeks.

Embodiment 227. The method of any one of embodiments 216 to 226, wherein the antiangiogenic compound is an anti-VEGF antibody, preferably an IgG antibody, and more preferably Bevacizumab.

Embodiment 228. The method of any one of embodiments 216 to 227, wherein
(a) if the anti-angiogenic compound is administered weekly, the dose is about 5 mg/kg body weight of the subject,
(b) if the anti-angiogenic compound is administered biweekly, the dose is about 10 mg/kg body weight of the subject, or
(c) if the anti-angiogenic compound is administered threeweekly, the dose is about 15 mg/kg body weight of the subject.

Embodiment 229. The method of any one of embodiments 216 to 228, wherein in the second phase of treatment the chemotherapy is administered in cycles of 28 days to the subject.

Embodiment 230. The method of any one of embodiments 216 to 229, preferably embodiment 229, wherein in the second phase the chemotherapy is administered for one, two, three, four, five, six or more cycles, preferably six cycles.

Embodiment 231. The method of embodiment 230, wherein a cycle consists of 28 days.

Embodiment 232. The method of embodiment 231, wherein temozolomide is administered to the subject on the first five days of a cycle followed by 23 days of rest.

Embodiment 233. The method of any one of embodiments 229 to 232, the chemotherapy comprises temozolomide.

Embodiment 234. The method of embodiment 233, wherein the daily dose of temozolomide is from about 150 to about 200 mg/m² body surface of the subject.

Embodiment 235. The method of any one of embodiments 216 to 234, wherein in the second phase of treatment
- the CXCL12 antagonist is NOX-A12 which is administered to the subject intravenously, continuously over a period of time of about 1 to about 20 weeks, wherein the weekly dose administered to the subject is about 200 mg/kg body weight of the subject, about 400 mg/kg body weight of the subject, about 600 mg/kg body weight of the subject or about 800 mg/kg body weight of the subject, and
- the antiangiogenic compound is Bevacizumab which is administered to the subject intravenously, biweekly over a period of time of about 1 weeks to about 20 weeks, wherein the biweekly dose administered to the subject is 10 mg/kg body weight of the subject.

Embodiment 236. The method of any one of embodiments 216 to 234, wherein in the second phase of treatment
- the CXCL12 antagonist is NOX-A12 which is administered to the subject intravenously, continuously over a period of time of about 1 to about 20 weeks, wherein the weekly dose administered to the subject is about 200 mg/kg body weight of the subject, about 400 mg/kg body weight of the subject, about 600 mg/kg body weight of the subject or about 800 mg/kg body weight of the subject,
- the antiangiogenic compound is Bevacizumab which is administered to the subject intravenously, biweekly over a period of time of about 1 weeks to about 20 weeks, wherein the biweekly dose administered to the subject is about 10 mg/kg body weight of the subject; and
- the chemotherapy is temozolomide which is orally or intravenously administered to the subject for six cycles, wherein each cycles consist of 28 days, wherein temozolomide is administered to the subject on each of days 1, 2, 3, 4, and 5 of each cycle, and wherein the daily dose of temozolomide administered to the subject is about 150 to about 200 mg/m² body surface of the subject.

Embodiment 237. The method of any one of embodiments 226 to 236, preferably any of embodiments 235 and 236, wherein in the second phase of treatment no radiotherapy is administered to the subject.

More specifically, the problems underlying the present invention are solved in a first aspect by a method for treating a tumor in a subject, wherein the method comprises administering to the subject
- a C-X-C motif chemokine 12 (CXCL12) antagonist,
- a radiotherapy, and
- an anti-angiogenic compound,
wherein the tumor is a brain tumor.

More specifically, the problem underlying the present invention is solved in a second aspect by a C-X-C motif chemokine 12 (CXCL12) antagonist for use in a method for treating a brain tumor in a subject, wherein the method comprises administering to the subject
- the C-X-C motif chemokine 12 (CXCL12) antagonist,
- a radiotherapy, and
- an anti-angiogenic compound,
wherein the tumor is a brain tumor.

More specifically, the problem underlying the present invention is solved in a third aspect by a radiotherapy for use in a method for treating a brain tumor in a subject, wherein the method comprises administering to the subject
- a C-X-C motif chemokine 12 (CXCL12) antagonist,
- the radiotherapy, and
- an anti-angiogenic compound,
wherein the tumor is a brain tumor.

More specifically, the problem underlying the present invention is solved in a fourth aspect by an antiangiogenic compound for use in a method for treating a brain tumor in a subject, wherein the method comprises administering to the subject
- a C-X-C motif chemokine 12 (CXCL12) antagonist,
- a radiotherapy, and
- the anti-angiogenic compound,
wherein the tumor is a brain tumor.

More specifically, the problem underlying the present invention is solved in a fifth aspect by a chemotherapy for use in a method for treating a brain tumor in a subject, wherein the method comprises administering to the subject
- a C-X-C motif chemokine 12 (CXCL12) antagonist,
- a radiotherapy,
- an anti-angiogenic compound, and
- the chemotherapy,
wherein the tumor is a brain tumor.

It will be appreciated by a person skilled in the art that any embodiment of the first aspect is also an embodiment of each and any one of the second, third, fourth and fifth aspect, and vice versa. It will also be appreciated by a person skilled in the art that an embodiment of the present invention is an embodiment of the first, second, third, fourth and fifth aspect of the present invention unless explicitly indicated differently.

The method of the present invention for treating a brain tumor comprising administering to the subject
- a C-X-C motif chemokine 12 (CXCL12) antagonist,
- a radiotherapy,
- an anti-angiogenic compound, and
- a chemotherapy
can, in principle, be applied to any brain tumor.

Brain tumors can be classified based on the cells forming such tumors or based on the microscopic appearance of the tumor cells. The most dominant brain tumors are gliomas, meningiomas, pituitary adenomas, vestibular schwannomas and medulloblastomas. Gliomas comprise several subtypes such as astrocytomas, oligodendromas, ependymomas and choroid plexus papillomas. Classification of the microscopic appearance of the cells forming a brain tumor is based on Grades I to IV as defined in the introductory part which is incorporated herein by reference.

In an embodiment of the present invention, the brain tumor is a Grade IV brain tumor. A particularly preferred brain tumor is a glioblastoma which is a Grade IV astrocytoma. Within glioblastoma, a further distinction can be made between supratentorial glioblastoma and infratentorial glioblastoma. Both forms can be treated by means of the method of the present invention, whereby supratentorial glioblastoma occurs more frequently.

Brain tumors which can be treated by the method of the present invention may be further or alternatively characterized in terms of treatment options available or treatment already applied to the subject suffering from the tumor.

In an embodiment of the present invention, the tumor which may be treated by the method of the present invention is one which is susceptible to radiotherapy, one which can be treated by radiotherapy or one which has been treated by radiotherapy prior to administering the method of the present invention to the subject suffering from the brain tumor. This kind of radiotherapy is known to a person skilled in the art. It will be appreciated by a person skilled in the art that this kind of radiotherapy can be the same kind of radiotherapy to be administered to the subject in accordance with the present invention the respective disclosure of which is incorporated herewith by reference.

In an embodiment of the present invention, the tumor which may be treated by the method of the present invention is one which is remaining after surgery, typically an incompletely resected tumor. As indicated above, surgery is still one of the most frequently used treatments for the treatment of brain tumors, whereby it will be acknowledged that some tumors cannot be treated by surgery as collateral tissue damage due to surgery might result in even more severe overall damage than the brain tumor itself or even in death. However, if a brain tumor is treated by surgery, some residual tumor tissue may escape from surgical removal, typically due to its small size or its position within the subject's brain. The position within the subject's brain can, for example, be a position not accessible by surgery for mechanical reasons, e.g. the non-tumor brain tissue does not allow access to the tumor tissue, or for medical reasons, e.g. the tumor tissue being too close to essential structures which must not be damaged for reason of the subject's well-being. This kind of residual tumor tissue may, in an embodiment of the present invention, be subject to the method of present invention.

Means for - typically post-operatively - detecting a tumor which is an incompletely resected tumor is a magnetic resonance imaging (MRI) scan. Preferably, the MRI scan is a contrast-enhanced MRI scan, and more preferably a T1-weighted, contrast-enhanced MRI scan. The basis of T1-weighted imaging is the longitudinal relaxation, i.e. return of longitudinal magnetization to its equilibrium value after excitation. A T1 weighted magnetic resonance image is created typically by using short echo times (TE) and repetition (TR) times. The final image is a reflection of more than one of these pulse sequence parameters, weighted according to the type of sequence and its timing. T1 signals determine predominantly the contrast and brightness in this type of images but proton density will always contribute to the image intensity. The T1 dependence is mainly determined by the repetition time or any pre-pulses (such as in an inversion recovery pulse sequence). In accordance therewith, in an embodiment, the tumor is a tumor providing a signal in a T1-weighted MRI scan; in a preferred embodiment, the tumor is an incompletely resected tumor providing a signal in a T1-weighted MRI scan. In both embodiments, the T1-weighted MRI scan is a T1-weighted, contrast-enhanced MRI scan.

Due to the larger longitudinal and transverse magnetization, fat has a higher signal and will appear bright on a T1 contrast MR image. Conversely, water has less longitudinal magnetization prior to a RF pulse, therefore less transverse magnetization after a RF pulse yielding low signal appearing dark on a T1 contrast image. Often, a paramagnetic contrast agent, a gadolinium compound, is administered, and both pre-contrast T1 weighted images and post-contrast T1 weighted images are obtained.

In an embodiment of the present invention, the tumor to be treated can be characterized by certain biochemical characteristics. Such biochemical characteristics include, but are not limited to, resistance towards one or medicament and secretion of factors like chemokines and/or angiogenic factors. It will be appreciated by a person skilled in the art that the tumor may also exhibit a combination of such biochemical characteristics.

In an embodiment of the present invention, resistance towards one or more medicaments is resistance to temozolomide (TMZ). Temozolomide is an alkylating agent and sold under the brand name Temodar among others. It is used as a standard therapy in the treatment of brain tumors such as glioblastoma (first-line treatment) and anaplastic astrocytoma (second line treatment). It is taken by mouth or via intravenous infusion. The most common side effects with temozolomide are nausea, vomiting, constipation, loss of appetite, alopecia (hair loss), headache, fatigue, convulsions (seizures), rash, neutropenia or lymphopenia (low white-blood-cell counts), and thrombocytopenia (low blood platelet counts).

Resistance to temozolomide therapy is a major cause of glioma and glioblastoma treatment failure and, therefore, providing means for the treatment of glioma and glioblastoma being resistant to temozolomide therapy is critical. The demethylating enzyme O6-methylguanine-DNA methyltransferase (MGMT) has been implicated in intrinsic TMZ-resistance (TMZ-R) and recurrence by removing alkyl groups from the O6 position of guanine directly. However, epigenetic silencing of MGMT is common in gliomas, and gliomas with low MGMT levels (deficiency and low-expression) are still sufficient to confer TMZ-R, suggesting the existence of an MGMT-independent mechanism of acquired TMZ-R. With low MGMT level, TMZ-R gliomas exhibited a gene mutation. For instance, recurrent gliomas exhibited transcriptional silencing of the MGMT gene, followed by dysfunction of the mismatch repair (MMR) system and hyperfunction of the DNA repair system. With deficiency of MMR, DNA damage repair (DDR) systems were activated, leading to TMZ-R. Therefore, the functional availability of the DDR system presumably regulates the response of recurrent gliomas to TMZ. Hence, it is hypothesized that DDR signaling is enhanced in TMZ-R glioma cells, which contributes to their phenotypic resistance. It was shown that Rho-associated kinases 2 (ROCK2) was hyper-activated in TMZ-R models and inhibition of ROCK2 reversed TMZ-R with an increase in TMZ sensitivity, suggesting an association between ROCK2 and TMZ-R, but the mechanism remains elusive (Zhang X et al., Cell Death & Disease 13, Article number 138 (2022)).

In accordance therewith, in an embodiment of the present invention, the tumor is a tumor which is non-responsive or resistant to temozolomide. Also, in an embodiment of the present invention, the tumor is glioblastoma with unmethylated O⁶-methylguanine DNA methyltransferase (MGMT) promoter status.

In an embodiment of the present invention, the tumor is a tumor secreting a factor, wherein the factor is a chemokine and/or an angiogenic factor. It is particularly preferred if the tumor is expressing both a chemokine and an angiogenic factor. In a preferred embodiment, the chemokine is CXC-motif chemokine 12 (CXCL12) and the angiogenic factor is vascular endothelial growth factor (VEGF). CXC-motif chemokine 12 (CXCL12), also known as stromal cell-derived factor 1 (SDF-1) is a key chemokine that is responsible for the attraction or repulsion of many types of leukocytes by signaling through the receptor CXC-motif chemokine receptor 4 (CXCR4) and potentially through its alternative receptor CXCR7.

CXCL12 is an angiogenic CXC-motif chemokine that does not contain the ELR motif typical of the IL-8-like chemokines (Salcedo, Wasserman et al. 1999; Salcedo and Oppenheim 2003) but binds and activates the G-protein coupled receptor CXCR4. As a result of alternative splicing, there are further forms of CXCL12, such as CXCL12α (68 amino acids, SEQ ID NO: 1) and CXCL12 β (SEQ ID NO: 2), which, compared to CXCL12 α carries five additional amino acids at the C-terminus (Shirozu, Nakano et al. 1995).

The amino acid sequence conservation between CXCL12 from different species is remarkable: Human CXCL12α (SEQ.ID. 1) and murine CXCL12α (SEQ ID NO: 3) are virtually identical. There is only a single conservative change of V to I at position 18 (Shirozu, Nakano et al. 1995).

Since the CXCL12 receptor CXCR4 is widely expressed on leukocytes, mature dendritic cells, endothelial cells, brain cells, and megakaryocytes, the activities of CXCL12 are pleiotropic. This chemokine, more than any other identified thus far, exhibits the widest range of biological functions. The most significant functional effects of CXCL12 are:
- Homing and attachment of epithelial cells to neovascular sites in the choroid portion of the retina;
- CXCL12 is required to maintain stem cells and progenitor cells, e.g. hematopoietic progenitor (usually CD34+) cells in the bone marrow of the adult;
- SDF 1 supports proliferation of pre-B cells and augments the growth of bone marrow B cell progenitors and it induces specific migration of pre- and pro-B cells, while not acting as a significant chemoattractant for mature B cells;
- CXCL12 is one of the most efficacious T cell chemoattractants; and
- CXCL12 and its receptor CXCR4 are essential for embryonic development.

Altered expression levels of CXCL12 or its receptor CXCR4 or altered responses towards those molecules are said to be associated with many human diseases, such as retinopathy (Brooks, Caballero et al. 2004; Butler, Guthrie et al. 2005; Meleth, Agron et al. 2005); cancer of breast (Muller, Homey et al. 2001; Cabioglu, Sahin et al. 2005), ovaries (Scotton, Wilson et al. 2002), pancreas (Koshiba, Hosotani et al. 2000), thyroid (Hwang, Chung et al. 2003) and nasopharynx (Wang, Wu et al. 2005); glioma (Zhou, Larsen et al. 2002); neuroblastoma (Geminder, Sagi-Assif et al. 2001); B cell chronic lymphocytic leukemia (Burger, Tsukada et al. 2000); WHIM syndrome (WHIM is an abbreviation for Warts, Hypogammaglobulinemia, Infections, Myelokathexis syndrome) (Gulino, Moratto et al. 2004; Balabanian, Lagane et al. 2005b; Kawai, Choi et al. 2005); immunologic deficiency syndromes (Arya, Ginsberg et al. 1999; Marechal, Arenzana-Seisdedos et al. 1999; Soriano, Martinez et al. 2002); pathologic neovascularization (Salvucci, Yao et al. 2002; Yamaguchi, Kusano et al. 2003; Grunewald, Avraham et al. 2006); inflammation (Murdoch 2000; Fedyk, Jones et al. 2001; Wang, Guan et al. 2001); multiple sclerosis (Krumbholz, Theil et al. 2006); rheumatoid arthritis / osteoarthritis (Buckley, Amft et al. 2000; Kanbe, Takagishi et al. 2002; Grassi, Cristino et al. 2004).

Tumors, including solid and hematological neoplasias and malignancies, are not just masses of cancer cells: infiltration of tumors with immune-cells is a characteristic of cancer. Many human cancers have a complex chemokine network that influences the extent and phenotype of this infiltrate, as well as tumor growth, survival, migration, and angiogenesis. Most solid tumors contain many non-malignant stromal cells. Indeed, stromal cells sometimes outnumber cancer cells. The predominant stromal cells that are found in cancers are macrophages, lymphocytes, endothelial cells and fibroblasts.

Cells from different cancer types have different profiles of chemokine-receptor expression, but the CXCL12 receptor CXCR4 is most commonly found in tumor cells of mouse and man: tumor cells from at least 23 different types of human cancers of epithelial, mesenchymal, and haematopoietic origin express CXCR4 (Balkwill 2004) with CXCL12 being the only known ligand for CXCR4. Apart from the bone marrow and secondary lymphoid tissue, where it is constitutively expressed, CXCL12 is found in primary tumor sites in lymphoma (Corcione, Ottonello et al. 2000) and brain tumors of both neuronal and astrocytic lineage. Furthermore, it is present at high levels in ovarian (Scotton, Wilson et al. 2002) and pancreatic cancer (Koshiba, Hosotani et al. 2000) as well as at sites of metastasis in breast (Muller, Homey et al. 2001) and thyroid cancer (Hwang, Chung et al. 2003), neuroblastoma and haematological malignancies (Geminder, Sagi-Assif et al. 2001).

Besides CXCR4 another CXCL12 receptor was identified: RDC1/CXCR7 (Balabanian, Lagane et al. 2005a, Burns, Summers et al. 2006). In vitro and in vivo studies with prostate cancer cell lines suggest that alterations in CXCR7/RDC1 expression are associated with enhanced adhesive and invasive activities in addition to a survival advantage. In vitro and in vivo studies have shown that both receptors for CXCL12, namely CXCR4 and the CXCR7 promote tumor growth, metastatic potential and resistance to (chemotherapy induced) apoptosis in a number of tumors, e.g breast cancer, glioblastomas, ovarian cancer, neuroblastoma, lung cancer colorectal and prostate cancer (Burns et al, 2006; Li et al, 2008; Scotton et al, 2002; Yang et al, 2008; Zagzag et al, 2008).

CXCR4 and CXCR7 expression thus seems to be a general characteristic of several tumours.

Vascular endothelial growth factor (VEGF) is a basic, heparin-binding, homodimeric glycoprotein of 45,000 Daltons. These properties correspond to those of VEGF165, the predominant VEGF isoform. Human VEGF gene has been located to chromosome 6p21.3 and is organized in eight exons separated by seven introns. Alternative splicing was shown to result in four major VEGF isoforms (VEGF121, VEGF165, VEGF189, and VEGF206) consisting of 121, 165, 189, and 206 amino acids following signal sequence cleavage, respectively (Ferrara & Henzel 1989, Biochem Biophys Res Comm 161:851; Houck et al. 1991, Mol Endocrinol 5:1806; Leung et al. 1989, Science 246:1306). Less frequent splice variants also have been reported, including VEGF145 (Poltorak et al. 1997, J Biol Chem 272:7151], VEGF183 [Jingjing et al. 1999, Invest Ophthalmol Vis Sci 40:752), and VEGF165b (Bates et al. 2002, Cancer Res 62:4123). An additional level of regulation of VEGF biological activity is provided by the proteolytic cleavage mechanism, including all VEGF isoforms, resulting in the VEGF 110 form (Houck et al. 1992, J Biol Chem 267:26031).

VEGF is a major regulator of angiogenesis during normal and pathological processes, including that associated with tumor growth (reviewed in Ferrara & Gerber 2001, Acta Haematol 106:148, or Ferrara et al. 2003, Nat Med 9:669). VEGF has a key regulatory function during developmental angiogenesis (Ferrara et a. 1996, Nature 380:439; Carmeliet et al. 1996, Nature 380:14756). A well-documented *in vitro* activity of VEGF is the ability to promote growth of vascular endothelial cells (EC) derived from arteries, veins and lymphatics (reviewed in Ferrara & Davis-Smyth 1997, Endocr Rev 18:4), as well as certain non-endothelial cells (reviewed in Matsumoto & Claesson-Welsh 2001, Sci STKE 2001(112): p. RE21). VEGF was shown to be a survival factor for ECs, both in vitro and in vivo Yuan et al. 1996, Proc Natl Acad Sci USA 93:14765; Gerber et al. 1998, J Biol Chem 273:13313; Gerber et al. 1998, J Biol Chem 273:30336; Benjamin et al. 1999, J Clin Invest 103:159. VEGF stimulated production of surfactant proteins by cultured type 2 pneumocytes (Compernolle et al. 2002, Nat Med 8:702). VEGF induces vasodilatation in vitro (Ku et al. 1993, Am J Physiol 265:H586), and is thought to play a role in inflammation due to its ability to induce vascular leakage (Dvorak et al. 1995, Am J Pathol 146:1029; Senger et al. 1983, Science 219:983). VEGF displays chemotactic effects on endothelial cells and increases expression of proteolytic enzymes in endothelial cells involved in stromal degradation. VEGF has also effects on bone marrow-derived cells, promoting monocyte activation and chemotaxis (Clauss et al. 1990, J Exp Med 172:1535). VEGF enhanced colony formation by mature subsets of granulocytemacrophage and erythroid progenitor cells that had been stimulated with a colony stimulating factor (Bates et al. 2002, Cancer Res 62:4123). VEGF also displays immune effects via inhibition of maturation of antigen-presenting dendritic cells (Gabrilovitch et al. 1996, Nat Med 2:1096).

VEGF binds two related recptor tyrosine kinases (RTK), named Flt-1 (VEGFR-1) (de Vries et al. 1992, Science 255:989; Shibuya et al. 1990, Oncogene 5:519), KDR/Flk-1 (VEGFR-2) (Terman et al. 1992, Biochem Biophys Res Commun 187:1579). The fins-like-tyrosine kinase Flt-4 (VEGFR-3) is a member of the same family of RTKs but is not a receptor for VEGF, binding instead to VEGFC and VEGFD8. In addition to these RTKs, VEGF interacts with a family of coreceptors, the neuropilins. Binding of VEGF to VEGFR-1 and VEGFR-2 induces the homodimerization of two receptor subunits, which in turn triggers autophosphorylation of their tyrosine kinase domains located within the cytoplasm. Autophosphorylation of the tyrosine kinase domains subsequently engages a series of specific signal transduction events, ultimately regulating the various biological activities of VEGF on endothelial cells. Most of VEGF's mitogenic and survival activity appears to be mediated by VEGFR-2, including the expression of the anti-apoptotic proteins Bcl-2 and A1. Survival signaling by VEGFR-2 is mediated by the PI3 kinase/Akt pathway (Gerber et al. 1998, J Biol Chem 273:30336). VEGFR-2 was also shown to induce other signal transduction pathways including phospholipase C gamma and mitogen-activated kinases MAPK p44/42 (Gille et al. 2001, J Biol Chem 276:3222).

In an embodiment of the method of the present invention, CXCL12 is expressed in the leading edge of the tumor (the outermost tumor compartment where the tumor spreads towards surrounding tissue), in the compartment with hyperplastic blood vessels (which are blood vessels with thickened walls due to proliferation of endothelial cells.) and/or in the compartment of microvascular proliferation of the tumor (see, e.g., Comba A et al., Front. Oncol., 05 August 2021; https://doi.org/10.3389/fonc.2021.703764).

In an embodiment of the method of the present invention, VEGF is expressed in the cellular tumor compartment of the tumor where the proliferation of tumor cells is most prominent, in the compartment where pseudopalisading cells (characterized by garland-like organization of tumor cells at the edge of areas of tumor necrosis) are present and/or a perinecrotic zone of dead malignant cells of the tumor.

In a particularly preferred embodiment, CXCL12 is expressed in a leading edge of the tumor, in a hyperplastic blood vessel of the tumor and/or in a microvascular proliferation zone of the tumor and VEGF is expressed in a cellular tumor compartment of the tumor by a pseudopalisading cell of the tumor and/or a perinecrotic zone of the tumor.

According to the method of the present invention, a CXCL12 antagonist is administered to the subject suffering from the tumor. The CXCL12 antagonist used in accordance with the present invention may make use of different modes of action. Such modes of action includes that the CXCL12 antagonist inhibits signaling of CXCL12 via one or both of CXCL12 receptor C-X-C chemokine receptor type 4 (CXCR4) and CXCL12 receptor C-X-C chemokine receptor type 7 (CXCR7).

It will be appreciated by a person skilled in the art that such inhibition of the signaling of CXCL12 via one or both of CXCL12 receptor CXCR4 and CXCR7 can be achieved by (a) providing a molecule binding to CXCL12 so as to inhibit the binding of CXCL12 to CXCR4 and/or CXCR7, providing a molecule binding to CXCR4 so as to inhibit the binding of CXCL12 to CXCR4, and (c) providing a molecule binding to CXCR7 so as to inhibit the binding of CXCL12 to CXCR7. Such molecule which is binding to CXCL12 is preferably one selected from the group comprising an aptamer, preferably a CXCL12 binding aptamer, a spiegelmer, preferably a CXCL12-binding spiegelmer", an antibody, preferably a CXCL12 binding antibody, a protein binding to CXCL12, a CXCL12 binding anticalin, a CXCL12 binding peptide, a fusion protein comprising a CXCL12 binding moiety and a CXCL12 binding small molecule.

Such molecule which is binding to CXCR4 to inhibit the signaling of CXCL12 is preferably one selected from the group comprising an aptamer, preferably a CXCR4 binding aptamer, a spiegelmer, preferably a CXCR4-binding Spiegelmer, an antibody, preferably a CXCR4 binding antibody, a protein binding to CXCR4, a CXCR4 binding anticalin, a CXCR4 binding peptide, a fusion protein comprising a CXCR4 binding moiety and a CXCR4 binding small molecule.

Such molecule which is binding to CXCR7 is preferably one selected from the group comprising an aptamer, preferably a CXCR7 binding aptamer, a spiegelmer, preferably a CXCR7-binding spiegelmer", an antibody, preferably a CXCR7 binding antibody, a protein binding to CXCR4, a CXCR7 binding anticalin, a CXCR7 binding peptide, a fusion protein comprising a CXCR7 binding moiety and a CXCR7 binding small molecule.

It will also be appreciated by a person skilled in the art that that such inhibition of the signaling of CXCL12 via one or both of CXCL12 receptor CXCR4 and CXCR7 can be achieved by a compound binding CXCR4 and/or CXCR7, whereby such binding of the compound interferes with signaling, preferably with the signal transducing characteristics of CXCR 4 and/or CXCR7 rather than interfering with the binding of CXCL12 to CXCR4 and/or CXCR7.

A molecule which is binding to CXCR4 and because of its binding to CXCR4 is interfering with the signal transduction characteristics of CXCR4, preferably signal transduction triggered by the binding of CXCL12 to CXCR4, is preferably one selected from the group comprising an aptamer, preferably a CXCR4 binding aptamer, a spiegelmer, preferably a CXCR4 binding spiegelmer, a protein binding to CXCR4, an antibody or antigen-binding moiety of the antibody, preferably a CXCR4 binding antibody or a CXCR4 binding fragment of the CXCR4 binding antibody, an anticalin, preferably a CXCR4 binding anticalin, a CXCR4 binding peptide, a fusion protein comprising a CXCR4 binding moiety and a CXCR4 binding small molecule.

The generation and identification, respectively, of such aptamer, spiegelmer, antibody, binding protein, anticalin, peptide, fusion protein and small molecule is known to a person skilled in the art and also disclosed herein.

In a preferred embodiment, CXCL12 is human CXCL12, CXCR4 is human CXCR4, and CXCR7 is human CXCR7.

In connection with this part of the method of the present invention, i.e. administering to the subject suffering from the tumor a CXCL12 antagonist, it is to be acknowledged that animal studies showed that radiation-induced devascularization is efficiently restored by vasculogenesis after recruitment of bone marrow-derived cells which leads to rapid recurrence of the tumor (Kioi et al. 2010, J Clin Invest 120:694). It is assumed that CXCL12 plays an important role in neovascularization by recruiting endothelial and other bone marrow-derived pro-angiogenic cells through a CXCR4- and CXCR7-dependent mechanism. Notably, irradiation further increases CXCL12 expression (Kioi et al. 2010, J Clin Invest 120:694; Kozin et al. 2010, Cancer Res 70:5679; Liu et al. 2014, Neuro Oncol 16:21).

In an embodiment of the present invention the CXCL12 antagonist inhibits signaling of CXCL12 via one or both of CXCL12 receptor C-X-C chemokine receptor type 4 (CXCR4) and CXCL12 receptor C-X-C chemokine receptor type 7 (CXCR7). In a preferred embodiment thereof, the CXCL12 antagonist inhibits the binding of CXCL12 to CXCL12 receptor C-X-C chemokine receptor type 4 (CXCR4) and/or to CXCL12 receptor C-X-C chemokine receptor type 7 (CXCR7). The interaction between CXCL12 and its receptor CXCR4 is also referred to as CXCL12 - CXCR4 axis. Such CXCL12 - CXCR4 axis may be targeted by a CXCL12 antagonist. Similarly, interaction between CXCL12 and its receptor CXCR7 is also referred to as CXCL12 - CXCR7 axis. Such CXCL12 - CXCR7 axis may be targeted by a CXCL12 antagonist. In an even more preferred embodiment, such binding of CXCL12 to said receptors is effected by the CXCL12 antagonist binding to CXCL12.

CXCL12 antagonists binding to CXCL12 include, among others, a nucleic acid molecule binding to CXCL12. Such CXCL12 binding nucleic acid molecule can be a CXCL12 binding aptamer or a CXCL12 binding spiegelmer.

Aptamers are nucleic acid molecules made of D-nucleotides as building blocks which specifically bind to a target molecule through a mechanism different from Watson Crick base pairing. Spiegelmers are nucleic acid molecules made of L-nucleotides as building blocks which specifically bind to a target molecule through a mechanism different from Watson Crick base pairing. Both aptamers and spiegelmers can be made of ribonucleotides, of deoxyribonucleotides are a combination of both ribonucleotides and deoxyribonucleotides. Aptamers and spiegelmers as such are known to a person skilled in the art and are, among others, described in 'The Aptamer Handbook' (eds. Klussmann, 2006) and spiegelmers comprising both ribonucleotides and deoxyribonucleotides are disclosed in international patent application WO 2012/095303.

In an embodiment of the method of the invention, the CXCL12 antagonist is an L-nucleic acid molecule binding to CXCL12, wherein the L-nucleic acid molecule is preferably selected from the group comprising an CXCL12 binding nucleic acid molecule of type B, an CXCL12 binding nucleic acid molecule of type C, an CXCL12 binding nucleic acid molecule of type A and an CXCL12 binding nucleic acid molecule of type D as defined in more detail herein.

The CXCL12 binding L-nucleic acid molecules can be characterized in terms of stretches of nucleotides which are also referred to herein as Boxes (see, Example 1).

The different types of CXCL12 binding nucleic acid molecules comprise three different stretches of nucleotides: the first terminal stretch of nucleotides, the central stretch of nucleotides and second terminal stretch of nucleotides. In general, CXCL12 binding nucleic acid molecules of the present invention comprise at their 5'-end and the 3'-end the terminal stretches of nucleotides: the first terminal stretch of nucleotides and the second terminal stretch of nucleotides (also referred to as 5'-terminal stretch of nucleotides and 3'-terminal stretch of nucleotides). The first terminal stretch of nucleotides and the second terminal stretch of nucleotides can, in principle due to their base complementarity, hybridize to each other, whereby upon hybridization a double-stranded structure is formed. However, such hybridization is not necessarily realized in the molecule under physiological and/or nonphysiological conditions. The three stretches of nucleotides of CXCL12 binding nucleic acid molecules
- the first terminal stretch of nucleotides, the central stretch of nucleotides and second terminal stretch of nucleotides - are arranged to each other in 5' -> 3'-direction: the first terminal stretch of nucleotides
- the central stretch of nucleotides - the second terminal stretch of nucleotides. However, alternatively, the second terminal stretch of nucleotides, the central stretch of nucleotides and the terminal first stretch of nucleotides are arranged to each other in 5' -> 3'-direction.

The differences in the sequences of the defined boxes or stretches between the different CXCL12 binding nucleic acid molecules influence the binding affinity to CXCL12. Based on binding analysis of the different CXCL12 binding nucleic acid molecules used in accordance with the present invention the central stretch and the nucleotides forming the same are individually and more preferably in their entirety essential for binding to human CXCL12.

The terms 'stretch' and 'stretch of nucleotide' are used herein in a synonymous manner if not indicated to the contrary.

In a preferred embodiment the nucleic acid used in accordance the present invention is a single nucleic acid molecule. In a further embodiment, the single nucleic acid molecule is present as a multitude of the single nucleic acid molecule or as a multitude of the single nucleic acid molecule species.

It will be acknowledged by the ones skilled in the art that the nucleic acid molecule used in accordance with the invention preferably consists of nucleotides which are covalently linked to each other, preferably through phosphodiester links or linkages.

It is within the present invention that the nucleic acids used in accordance with present invention comprise two or more stretches or part(s) thereof can, in principle, hybridise with each other. Upon such hybridisation a double-stranded structure is formed. It will be acknowledged by the ones skilled in the art that such hybridisation may or may not occur, particularly under in vitro and/or in vivo conditions. Also, in case of such hybridisation, it is not necessarily the case that the hybridisation occurs over the entire length of the two stretches where, at least based on the rules for base pairing, such hybridisation and thus formation of a double-stranded structure may, in principle, occur. As preferably used herein, a double-stranded structure is a part of a nucleic acid molecule or a structure formed by two or more separate strands or two spatially separated stretches of a single strand of a nucleic acid molecule, whereby at least one, preferably two or more base pairs exist which are base pairing preferably in accordance with the Watson-Crick base pairing rules. It will also be acknowledged by the one skilled in the art that other base pairing such as Hoogsten base pairing may exist in or form such double-stranded structure. It is also to be acknowledged that the feature that two stretches hybridize preferably indicates that such hybridization is assumed to happen due to base complementarity of the two stretches.

In a preferred embodiment, the term arrangement as used herein, means the order or sequence of structural or functional features or elements described herein in connection with the nucleic acids disclosed herein.

It will be acknowledged by the person skilled in the art that the nucleic acids used in accordance with the present invention are capable of binding to CXCL12. Without wishing to be bound by any theory, the CXCL12 binding results from a combination of three-dimensional structural traits or elements of the nucleic acid molecule which are caused by orientation and folding patterns of the primary sequence of nucleotides forming such traits or elements, whereby preferably such traits or elements are the first terminal stretch of nucleotides, the central stretch of nucleotides and the second terminal stretch of nucleotides of CXCL12 binding nucleic acid molecules. It is evident that the individual trait or element may be formed by various different individual sequences the degree of variation of which may vary depending on the three-dimensional structure such element or trait has to form. The overall binding characteristic of the claimed nucleic acid results from the interplay of the various elements and traits, respectively, which ultimately results in the interaction of the claimed nucleic acid with its target, i. e. CXCL12. Again without being wished to be bound by any theory, the central stretch of nucleotides that is characteristic for CXCL12 binding nucleic acids seems to be important for mediating the binding of the claimed nucleic acid molecules with CXCL12. Accordingly, the nucleic acids according to the present invention are suitable for the interaction with CXCL12. Also, it will be acknowledged by the person skilled in the art that the nucleic acids according used in accordance with the present invention are antagonists to CXCL12. Because of this, the nucleic acids according to the present invention are suitable for the treatment and prevention of brain cancer in accordance with the method of the present invention.

The nucleic acid molecules used in accordance with the present invention shall also comprise nucleic acids which are essentially homologous to the particular sequences disclosed herein. The term substantially homologous shall be understood such as the homology is at least 75%, preferably 85%, more preferably 90% and most preferably more that 95 %, 96 %, 97 %, 98 % or 99%.

The actual percentage of homologous nucleotides present in the nucleic acid used in accordance with the present invention will depend on the total number of nucleotides present in the nucleic acid. The percent modification can be based upon the total number of nucleotides present in the nucleic acid.

The homology between two nucleic acid molecules can be determined as known to the person skilled in the art. More specifically, a sequence comparison algorithm may be used for calculating the percent sequence homology for the test sequence(s) relative to the reference sequence, based on the designated program parameters. The test sequence is preferably the sequence or nucleic acid molecule which is said to be homologous or to be tested whether it is homologous, and if so, to what extent, to a different nucleic acid molecule, whereby such different nucleic acid molecule is also referred to as the reference sequence. In an embodiment, the reference sequence is a nucleic acid molecule as described herein, preferably a nucleic acid molecule having a sequence according to any one of SEQ ID NO: 5 to SEQ ID NO: 225, more preferably a nucleic acid molecule having a sequence according to any one of SEQ ID NO: 22, SEQ ID NO: 28, SEQ ID NO: 120, SEQ ID NO: 128, SEQ ID NO: 129, SEQ ID NO: 134, SEQ ID NO: 135, SEQ ID NO: 223, SEQ ID NO: 224, SEQ ID NO: 84, SEQ ID NO: 146, SEQ ID NO: 142, SEQ ID NO: 143, and SEQ ID NO: 144. Optimal alignment of sequences for comparison can be conducted, e.g., by the local homology algorithm of Smith & Waterman (Smith & Waterman, 1981) by the homology alignment algorithm of Needleman & Wunsch (Needleman & Wunsch, 1970) by the search for similarity method of Pearson & Lipman (Pearson & Lipman, 1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection.

One example of an algorithm that is suitable for determining percent sequence identity is the algorithm used in the basic local alignment search tool (hereinafter "BLAST "), see, e.g. Altschul et al (Altschul et al. 1990 and Altschul et al, 1997). Software for performing BLAST analyses is publicly available through the National Center for Biotechnology Information (hereinafter "NCBI"). The default parameters used in determining sequence identity using the software available from NCBI, e.g., BLASTN (for nucleotide sequences) and BLASTP (for amino acid sequences) are described in McGinnis et al (McGinnis et al., 2004).

The nucleic acid molecules used in accordance with the present invention shall also comprise nucleic acid molecules which have a certain degree of identity relative to the nucleic acid moelcules disclosed herein and defined by their nucleotide sequence. More preferably, the instant invention also comprises those nucleic acid molecules which have an identity of at least 75%, preferably 85%, more preferably 90% and most preferably more than 95 %, 96 %, 97 %, 98 % or 99% relative to the nucleic acid molecules disclosed herein and defined by their nucleotide sequence or a part thereof.

The terms nucleic acid(s) and nucleic acid molecule(s) are used interchangeably herein. Also, the nucleic acid molecules used in accordance with the present invention shall also comprise those nucleic acid molecules comprising the nucleic acids sequences disclosed herein or part thereof, such as, e.g., a metabolite or derivative of the nucleic acid molecules used in accordance with the present invention, preferably to the extent that the nucleic acid molecules or said parts are involved in the or capable of binding to CXCL12. Such a nucleic acid molecule may be derived from the ones disclosed herein, e.g., by truncation. Truncation may be related to either or both of the ends of the nucleic acid molecules as disclosed herein. Also, truncation may be related to the inner sequence of nucleotides, i.e. it may be related to the nucleotide(s) between the 5' and the 3' terminal nucleotide, respectively. Moreover, truncation shall comprise the deletion of as little as a single nucleotide from the sequence of the nucleic acids disclosed herein. Truncation may also be related to more than one stretch of the nucleic acid molecules used in accordance with the present invention, whereby the stretch can be as little as one nucleotide long. The binding of a nucleic acid molecules used in accordance with the present invention can be determined by the ones skilled in the art using routine experiments or by using or adopting a method as described herein, preferably as described herein in the example part.

In addition, it is within that present invention that one or several parts of the nucleic acid molecule used in accordance with the present invention are present as D-nucleic acids or at least one or several parts of the nucleic acid molecules are L-nucleic acids. The term "part" of the nucleic acid molecules shall mean as little as one nucleotide. Such nucleic acid molecules are generally referred to herein as D- and L-nucleic acids, respectively. Therefore, in a particularly preferred embodiment, the nucleic acid molecules used in accordance with the present invention consist of L-nucleotides and comprise at least one D-nucleotide. Such D-nucleotide is preferably attached to a part different from the stretches defining the nucleic acid molecules used in accordance with the present invention, preferably those parts thereof, where an interaction with other parts of the nucleic acid molecule is involved. Preferably, such D-nucleotide is attached at a terminus of any of the stretches and of any nucleic acid molecule used in accordance with the present invention, respectively. In a further preferred embodiment, such D-nucleotides may act as a spacer or a linker, preferably attaching modifications such as PEG and HES to the nucleic acids according to the present invention.

It is also within the present invention that the nucleic acid molecules used in accordance with the present invention are part of a longer nucleic acid molecule, whereby this longer nucleic acid molecule comprises several parts, whereby at least one such part is a nucleic acid molecule, or a part thereof, used in accordance with the present invention. The other part(s) of these longer nucleic acid molecules can be either one or several D-nucleic acid(s) or L-nucleic acid(s). Any combination may be used in connection with the present invention. These other part(s) of the longer nucleic acid molecule can exhibit a function which is different from binding, preferably from binding to CXCL12. One possible function is to allow interaction with other molecules, whereby such other molecules preferably are different from CXCL12, such as, e.g., for immobilization, cross-linking, detection or amplification. In a further embodiment of the present invention the nucleic acid molecules used in accordance with the invention comprise, as individual or combined moieties, several of the nucleic acid molecules used in accordance with the present invention. Such nucleic acid molecule comprising several of the nucleic acid molecules used in accordance with the present invention is also encompassed by the term longer nucleic acid.

L-nucleic acid molecules as used herein are nucleic acid consisting of consisting of L-nucleotides, preferably consisting completely of L-nucleotides.

D-nucleic acid molecules as used herein are nucleic acid molecules consisting of D-nucleotides, preferably consisting completely of D-nucleotides.

The terms nucleic acid and nucleic acid molecule are used herein in an interchangeable manner if not explicitly indicated to the contrary.

Also, if not indicated to the contrary, any nucleotide sequence is set forth herein in 5' -> 3' direction.

As preferably used herein, any position of a nucleotide is determined or referred to relative to the 5' end of a sequence, a stretch or a substretch. Accordingly, a second nucleotide is the second nucleotide counted from the 5' end of the sequence, stretch and substretch, respectively. Also, in accordance therewith, a penultimate nucleotide is the second nucleotide counted from the 3' end of a sequence, stretch and substretch, respectively.

It is also within the present invention that the nucleic acid molecules used in accordance with the present invention, regardless whether they are present as D-nucleic acids, L-nucleic acids or D,L-nucleic acids or whether they are DNA or RNA, may be present as single stranded or double stranded nucleic acids. Typically, the nucleic acid molecules used in accordance with the present invention are single stranded nucleic acids which exhibit defined secondary structures due to the primary sequence and may thus also form tertiary structures. The nucleic acid molecules used in accordance with the present invention, however, may also be double stranded in the meaning that two strands which are complementary or partially complementary to each other, are hybridised to each other or at least may be hybridised to each other in light of their base complementarity.

The nucleic acid molecules used in accordance with the present invention may be modified. Such modifications may be related to a single nucleotide of the nucleic acid and are well known in the art. Examples for such modification are described by, among others, Venkatesan et al. (Venkatesan, Kim et al., 2003) and Kusser (Kusser, 2000). Such modification can be a H atom, an F atom or O-CH₃ group or NH₂-group at the 2' position of the individual nucleotide of which the nucleic acid consists. Also, the nucleic acid molecule used in accordance with the present invention can comprises at least one LNA nucleotide. In an embodiment the nucleic acid according to the present invention consists of LNA nucleotides.

In an embodiment, the nucleic acid molecules used in accordance with the present invention may be a multipartite nucleic acid molecule. A multipartite nucleic acid molecule as used herein, is a nucleic acid molecule which consists of at least two separate nucleic acid strands. These at least two nucleic acid strands form a functional unit whereby the functional unit is a ligand to a target molecule. The at least two nucleic acid strands may be derived from any of the nucleic acid molecules used in accordance with the present invention by either cleaving the nucleic acid molecule to generate two strands or by synthesising one nucleic acid corresponding to a first part of the overall nucleic acid molecule and another nucleic acid molecule corresponding to the second part of the overall nucleic acid molecule. It is to be acknowledged that both the cleavage and the synthesis may be applied to generate a multipartite nucleic acid molecule where there are more than two strands as exemplified above. In other words, the at least two separate nucleic acid strands are typically different from two strands being complementary and hybridising to each other although a certain extent of complementarity between said at least two separate nucleic acid strands may exist and whereby such complementarity may result in the hybridisation of said separate strands.

Finally, it is also within the present invention that a fully closed, i.e. circular structure for the nucleic acid molecules used in accordance with the present invention is realized, i.e. that the nucleic acid molecules are closed in an embodiment, preferably through a covalent linkage, whereby more preferably such covalent linkage is made between the 5' end and the 3' end of the nucleic acid sequences as disclosed herein or any derivative thereof.

An option for determining the binding constants of a nucleic acid molecule used in accordance with the present invention is the use of the methods as described in Examples 3 and 4 which confirms the above finding that the nucleic acid molecules used in accordance with the present invention according to the present invention exhibit a favourable K_{D} value range. An appropriate measure in order to express the intensity of the binding between the individual nucleic acid molecule and the target which is in the present case CXCL12, is the so-called K_{D} value which as such as well the method for its determination are known to the one skilled in the art.

Preferably, the K_{D} value shown by the nucleic acid molecules used in accordance with the present invention is below 1 µM. A K_{D} value of about 1 µM is said to be characteristic for a non-specific binding of a nucleic acid to a target. As will be acknowledged by the ones skilled in the art, the K_{D} value of a group of compounds such as the nucleic acid molecules used in accordance with the present invention is within a certain range. The above-mentioned K_{D} of about 1 µM is a preferred upper limit for the K_{D} value. The lower limit for the K_{D} of target binding nucleic acids can be as little as about 10 picomolar or can be higher. It is within the present invention that the K_{D} values of individual nucleic acid molecule binding to CXCL12 is preferably within this range. Preferred ranges can be defined by choosing any first number within this range and any second number within this range. Preferred upper K_{D} values are 250 nM and 100 nM, preferred lower K_{D} values are 50 nM, 10 nM, 1 nM, 100 pM and 10 pM. The more preferred upper K_{D} value is 2.5 nM, the more preferred lower K_{D} value is 100 pM.

In addition to the binding properties of the nucleic acid molecules used in accordance with the present invention, the nucleic acid molecules used in accordance with the present invention inhibit the function of the respective target molecule which is in the present case CXCL12. The inhibition of the function of CXCL12 - for instance the stimulation of the respective receptors as described above - is achieved by binding of a nucleic acid molecule used in accordance with the present invention to CXCL12 and forming a complex of the nucleic acid molecule and CXCL12. Such complex of the nucleic acid molecule and CXCL12 cannot stimulate the receptors that normally are stimulated by CXCL12, such as CXCR4 and CXCR7. Accordingly, the inhibition of receptor function by nucleic acid molecules is independent from the respective receptor that can be stimulated by CXCL12 but results from preventing the stimulation of the receptor by CXCL12 by the nucleic acid molecule.

A possibility to determine the inhibitory constant of the nucleic acid molecule used in accordance with the present invention is the use of the methods as described in Examples 5 and 6 (for CXCR4 and CXCR7, respectively) which confirms the above finding that the nucleic acid molecules used in accordance with the present invention exhibit a favourable inhibitory constant which allows the use of said nucleic acid molecules in the claimed method of treatment and any treatment scheme comprising such method of treatment. An appropriate measure in order to express the intensity of the inhibitory effect of the individual nucleic acid molecule on interaction of the target which is in the present case CXCL12 and the respective receptor, is the so-called half maximal inhibitory concentration (abbr. IC₅₀) which as such as well the method for its determination are known to the one skilled in the art.

Preferably, the IC₅₀ value shown by the nucleic acid molecules used in accordance with the present invention is below 1 µM. An IC₅₀ value of about 1 µM is said to be characteristic for a non-specific inhibition of target functions by a nucleic acid molecule. As will be acknowledged by a person skilled in the art, the IC₅₀ value of a group of compounds such as the nucleic acid molecules used in accordance with the present invention is within a certain range. The above-mentioned IC₅₀ of about 1 µM is a preferred upper limit for the IC₅₀ value. The lower limit for the IC₅₀ of target binding nucleic acid molecules can be as little as about 10 picomolar or can be higher. It is within the present invention that the IC₅₀ values of individual nucleic acid molecule binding to CXCL12 is preferably within this range. Preferred ranges can be defined by choosing any first number within this range and any second number within this range. Preferred upper IC₅₀ values are 250 nM and 100 nM, preferred lower IC₅₀ values are 50 nM, 10 nM, 1 nM, 100 pM and 10 pM. The more preferred upper IC₅₀ value is 2.5 nM, the more preferred lower IC₅₀ value is 100 pM.

The nucleic acid molecules used in accordance with the present invention may have any length provided that they are still able to bind to the target molecule. It will be acknowledged in the art that there are preferred lengths of the nucleic acid molecules used in accordance with the present invention. Typically, the length is between 15 and 120 nucleotides. It will be acknowledged by the ones skilled in the art that any integer between 15 and 120 is a possible length for the nucleic acid molecules used in accordance with the present invention. More preferred ranges for the length of the nucleic acid molecules used in accordance with the present invention are lengths of about 20 to 100 nucleotides, about 20 to 80 nucleotides, about 20 to 60 nucleotides, about 20 to 50 nucleotides and about 29 to 45 nucleotides.

It is within the present invention that the nucleic acids used in accordance with the present invention comprise a moiety which preferably is a high molecular weight moiety and/or which preferably allows to modify the characteristics of the nucleic acid in terms of, among others, residence time in the animal body, preferably the human body. A particularly preferred embodiment of such modification is PEGylation and HESylation of the nucleic acids used in accordance with the present invention. As used herein PEG stands for poly(ethylene glycole) and HES for hydroxyethly starch. PEGylation as preferably used herein is the modification of a nucleic acid used in accordance with the present invention, whereby such modification consists of a PEG moiety which is attached to a nucleic acid used in accordance with the present invention. HESylation as preferably used herein is the modification of a nucleic acid used in accordance with the present invention, whereby such modification consists of a HES moiety which is attached to a nucleic acid used in accordance with the present invention. These modifications as well as the process of modifying a nucleic acid using such modifications, is described in European patent application EP 1 306 382, the disclosure of which is herewith incorporated in its entirety by reference.

In case of PEG being such high molecular weight moiety, the molecular weight is preferably from about 20,000 to about 120,000 Da, more preferably from about 30,000 to about 80,000 Da and most preferably about 40,000 Da. In case of HES being such high molecular weight moiety, the molecular weight is preferably from about 50 to about 1000 kDa, more preferably from about 100 to about 700 kDa and most preferably from about 200 to about 500 kDa. HES exhibits a molar substitution of 0.1 to 1.5, more preferably of 1 to 1.5 and exhibits a substitution sample expressed as the C2/C6 ratio of approximately 0.1 to 15, preferably of approximately 3 to 10. The process of HES modification is, e.g., described in German patent application DE 1 2004 006 249.8 the disclosure of which is herewith incorporated in its entirety by reference.

The modification can, in principle, be made to the nucleic acid molecules used in accordance with the present invention at any position thereof. Preferably such modification is made either to the 5' -terminal nucleotide, the 3'-terminal nucleotide and/or any nucleotide between the 5' nucleotide and the 3' nucleotide of the nucleic acid molecule.

The modification and preferably the PEG and/or HES moiety can be attached to the nucleic acid molecule used in accordance with the present invention either directly or indirectly, preferably through a linker. It is also within the present invention that the nucleic acid molecule used in accordance with the present invention comprises one or more modifications, preferably one or more PEG and/or HES moiety. In an embodiment the individual linker molecule attaches more than one PEG moiety or HES moiety to a nucleic acid molecule used in accordance with the present invention. The linker used in connection with the present invention can itself be either linear or branched. This kind of linkers are known to the ones skilled in the art and are further described in patent applications WO2005/074993 and WO2003/035665.

In a preferred embodiment the linker is a biodegradable linker. The biodegradable linker allows to modify the characteristics of the nucleic acid used in accordance with the present invention in terms of, among other, residence time in an animal body, preferably in a human body, due to release of the modification from the nucleic acid according to the present invention. Usage of a biodegradable linker may allow a better control of the residence time of the nucleic acid molecule used in accordance with the present invention. A preferred embodiment of such biodegradable linker is a biodegradable linker as described in, but not limited to, international patent applications WO2006/052790, WO2008/034122, WO2004/092191 and WO2005/099768.

It is within the present invention that the modification or modification group is a biodegradable modification, whereby the biodegradable modification can be attached to the nucleic acid molecule used in accordance with the present invention either directly or indirectly, preferably through a linker. The biodegradable modification allows to modify the characteristics of the nucleic acid used in accordance with the present invention in terms of, among other, residence time in an animal body, preferably in a human body, due to release or degradation of the modification from the nucleic acid molecule used in accordance with the present invention. Usage of biodegradable modification may allow a better control of the residence time of the nucleic acid according to the present invention. A preferred embodiment of such biodegradable modification is biodegradable as described in, but not restricted to, international patent applications WO2002/065963, WO2003/070823, WO2004/113394 and WO2000/41647, preferably in WO2000/41647, page 18, line 4 to 24.

Beside the modifications as described above, other modifications can be used to modify the characteristics of the nucleic acids used in accordance with the present invention, whereby such other modifications may be selected from the group of proteins, lipids such as cholesterol and sugar chains such as amylase, dextran etc.

Without wishing to be bound by any theory, it seems that by modifying the nucleic acid molecules used in accordance with the present invention with high molecular weight moiety such as a polymer and more particularly one or several of the polymers disclosed herein, which are preferably physiologically acceptable, the excretion kinetic is changed. More particularly, it seems that due to the increased molecular weight of such modified nucleic acids and due to the nucleic acid molecules used in accordance with the present invention not being subject to metabolism particularly when in the L-form, excretion from an animal body, preferably from a mammalian body and more preferably from a human body is decreased. As excretion typically occurs via the kidneys, glomerular filtration rate of the thus modified nucleic acid molecules is significantly reduced compared to nucleic acid molecules not having this kind of high molecular weight modification which results in an increase in the residence time in the animal body. In connection therewith it is particularly noteworthy that, despite such high molecular weight modification the specificity of the nucleic acid molecules used in accordance with the present invention is not affected in a detrimental manner. Insofar, the nucleic acid molecules used in accordance with the present invention have among others, the surprising characteristic - which normally cannot be expected from pharmaceutically active compounds - such that a pharmaceutical formulation providing for a sustained release is not necessarily required to provide for a sustained release of the nucleic acid molecules used in accordance with the present invention. Rather, the nucleic acid molecules used in accordance with the present invention in their modified form comprising a high molecular weight moiety, can as such already be used as a sustained release-formulation as they act, due to their modification, already as if they were released from a sustained-release formulation. Insofar, the modification(s) of the nucleic acid molecules used in accordance with the present invention as disclosed herein and the thus modified nucleic acid molecules used in accordance with the present invention and any composition comprising the same may provide for a distinct, preferably controlled pharmacokinetics and biodistribution thereof. This also includes residence time in circulation and distribution to tissues. Such modifications are further described in the patent application WO2003/035665.

However, it is also within the present invention that the nucleic acid molecules used in accordance with the present invention do not comprise any modification and particularly no high molecular weight modification such as PEGylation or HESylation. Such embodiment is particularly preferred when the nucleic acid molecule used in accordance with the present invention shows preferential distribution to any target organ or tissue in the body or when a fast clearance of the nucleic acid molecule used in accordance with the present invention from the body after administration is desired. Nucleic acid molecules used in accordance with the present invention with a preferential distribution profile to any target organ or tissue in the body would allow establishment of effective local concentrations in the target tissue while keeping systemic concentration of the nucleic acid molecules low. This would allow the use of low doses which is not only beneficial from an economic point of view, but also reduces unnecessary exposure of other tissues to the nucleic acid agent, thus reducing the potential risk of side effects. Fast clearance of the nucleic acid molecules used in accordance with the present invention from the body after administration might be desired, among others, in case of *in vivo* imaging or specific therapeutic dosing requirements using the nucleic acid molecules used in accordance with the present invention or medicaments comprising the same.

In an embodiment, a pharmaceutical composition comprising a nucleic acid molecule used in accordance with the present invention comprises at least one of said nucleic acid molecules and a pharmaceutically acceptable excipient. Such pharmaceutical composition may additionally comprise one or more further pharmaceutically active compounds. Such pharmaceutically acceptable excipient may be, e.g., water, buffer, PBS, glucose solution, preferably a 5% glucose salt balanced solution, starch, sugar, gelatine or any other acceptable carrier substance. Such excipients are generally known to the one skilled in the art. It will be acknowledged by the person skilled in the art that any embodiments, use and aspects of or related to the medicament of the present invention is also applicable to the pharmaceutical composition of the present invention and vice versa.

The indications, diseases and disorders for the treatment and/or prevention of which the nucleic acids, the pharmaceutical compositions and medicaments are used in accordance with or prepared in accordance with the present invention result from the involvement, either direct or indirect, of CXCL12 in the respective pathogenic mechanism.

In a preferred embodiment of the method of the invention, the CXCL12 antagonist is NOX-A12. NOX-A12 (CAS-RN 1322069-19-1) is an (L)-oligonucleotide of 45 (L)-ribonucleotides in length (Mol. Wt. 14.5 kDa) that terminates at the 5'-end in a hexylamino linker, to which a branched 40 kDa monomethoxy polyethylene glycol (PEG) moiety is covalently attached. The PEG moiety increases the plasma half-life of the molecule. NOX-A12 is suitable for parenteral administration and has a good subcutaneous bioavailability. NOX-A12 is a nucleic acid molecule of SEQ ID NO: 28. It will be appreciated by a person skilled in the art that a preferred nucleic acid molecule binding to CXCL12 is an L-nucleic acid molecule having an identity of at least 80 % to an L-nucleic acid molecule of SEQ ID NO: 28 or SEQ ID NO: 22.

The chemical structure of NOX-A12 can also be depicted as L-guanylyl-(3'→5')- L-cytidylyl-(3'→5')-L-guanylyl-(3'→5')- L-uridylyl-(3'→5')- L-guanylyl-(3'→5')- L-guanylyl-(3'→5')- L-uridylyl-(3'→5')-L-guanylyl-(3'→5')- L-uridylyl-(3'→5')- L-guanylyl-(3'→5')- L-adenylyl-(3'→5')- L-uridylyl-(3'→5')-L-cytidylyl-(3'→5')- L-uridylyl-(3'→5')- L-adenylyl-(3'→5')- L-guanylyl-(3'→5')- L-adenylyl-(3'→5')-L-uridylyl-(3'→5')- L-guanylyl-(3'→5')- L-uridylyl-(3'→5')- L-adenylyl-(3'→5')- L-uridylyl-(3'→5')-L-uridylyl-(3'→5')- L-guanylyl-(3'→5')- L-guanylyl-(3'→5')- L-cytidylyl-(3'→5')- L-uridylyl-(3'→5')-L-guanylyl-(3'→5')- L-adenylyl-(3'→5')- L-uridylyl-(3'→5')- L-cytidylyl-(3'→5')- L-cytidylyl-(3'→5')-L-uridylyl-(3'→5')- L-adenylyl-(3'→5')- L-guanylyl-(3'→5')- L-uridylyl-(3'→5')- L-cytidylyl-(3'→5')-L-adenylyl-(3'→5')- L-guanylyl-(3'→5')- L-guanylyl-(3'→5')- L-uridylyl-(3'→5')- L-adenylyl-(3'→5')-L-cytidylyl-(3'→5')- L-guanylyl-(3'→5')- L-cytidylyl-(3'→5'), 5'-ester with N-[ω-methoxypoly(oxy-1,2-ethanediyl], N'-[ω-methoxypoly(oxy-1,2-ethanediyl]-acetyl-9-amino-8-oxo-7-aza-nonyloxy phosphate, sodium salt, whereby it additionally comprises a 40 kDa PEG moiety attached to the 5'-terminal end of the nucleotide sequence by means of the above indicated linker. In case of NOX-A12, the 40 kDa PEG moiety increases plasma half-life in humans from a few minutes (for the non-PEGylated L-nucleic acid molecule) to almost two days.

In an embodiment, the CXCL12 antagonist is Olaptesed pegol. Preferably, the terms NOX-A12 and Olaptesed pegol are used synonymously.

In an embodiment of the present invention, the CXCL12 antagonist is an CXCL12 binding aptamer provided by Aptitude Medical Systems, originally provided for the treatment of wet AMD.

In another embodiment of the present invention, the CXCL12 antagonist binding to CXCL12 and inhibiting binding of CXCL12 to CXCL12 receptor CXCR4 and/or CXCR7 is an antibody binding to CXCL12, preferably an antibody binding to CXCL12 and inhibiting binding of CXCL12 to CXCL12 receptor CXCR4 and/or CXCR7. In this embodiment, CXCL12 is the target or antigen of the antibody. In a preferred embodiment, the antibody is a monoclonal antibody.

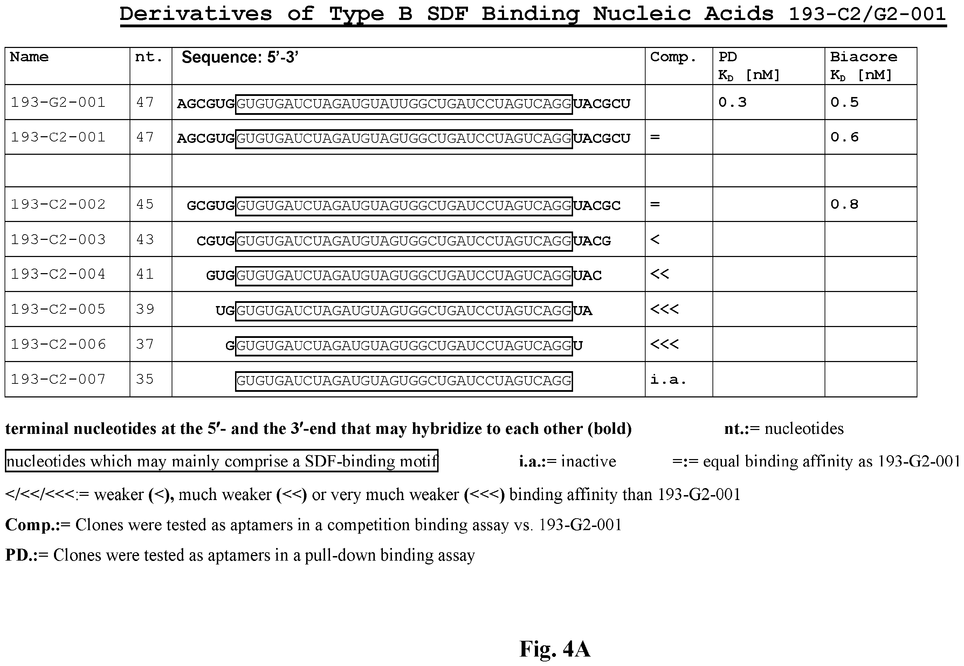

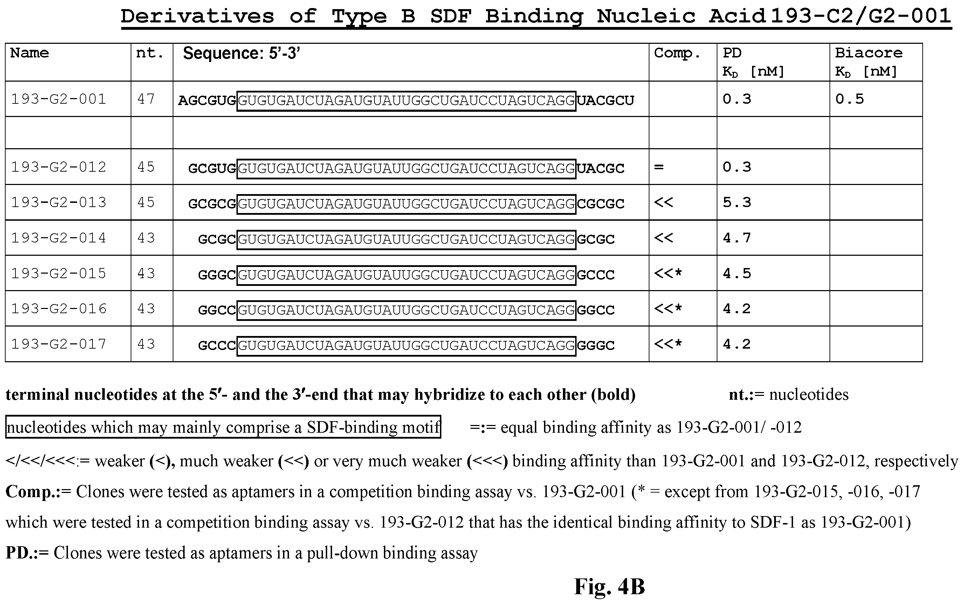

Monoclonal antibodies directed to CXCL12 are known in the art and include, among others, mAB-30D8 (provided by Genentech), 1131-H12 or 1143-H1 (both provided by iOnctura). The antibody sequence of antibody mAB-30D8, including a humanized form thereof, is disclosed in Zhong C et al. (Cancer Therapy Res (2013) 19 (16): 4433-4445; see, suppl. Fig. 4).

In a further embodiment, CXCL12 is bound by a protein, such protein may be a CXCL12 binding TRAP protein (provided by OncoTrap, Inc.) or a CXCL12 binding anticalin. In an embodiment, the TRAP protein is provided through a TRAP protein coding mRNA delivered to the tumor. The expression product of the mRNA is secreted by the transfected cells and diffuses throughout the tumor environment capturing CXCL12 with nanomolar binding affinity, whereupon CXCL12 is trapped and its function in the tumor microenvironment is abolished. In an embodiment and as preferably used herein an anticalin is a target binding polypeptide. This class of target binding polypeptides is, among others, described in German patent application DE 197 42 706.

It will be appreciated by a person skilled in the art that apart from the above classes of compounds, also a small molecule might in principle be used as an antagonist of CXCL12. The latter even more so is due to the either direct or indirect interaction, preferably direct physical interaction of such small molecule the binding of CXCL12 to its receptor CXCR4 and/or CXCR7 is inhibited. As preferably used herein, a small molecule is a molecule the molecule weight of which is 900 Da or less. In an alternative, a small molecule is a molecule which is in compliance with Lipinski's rule of five, i.e. that the compound is no more than one violation of the following criteria: no more than 5 hydrogen bond donors (the total number of nitrogen-hydrogen and oxygen-hydrogen bonds), no more than 10 hydrogen bond acceptors (all nitrogen or oxygen atoms), a molecular mass less than 500 Daltons; and an octanol-water partition coefficient (log P) that does not exceed 5.

In another embodiment of the present invention, the CXCL12 antagonist is binding to CXCR4 and inhibits the binding of CXCL12 to CXCR4. In other words, CXCR4 is the target and antigen, respectively, which is bound by this embodiment of a CXCL12 antagonist. As in case of the CXCL12 antagonist binding to CXCL12 and inhibiting the binding of CXCL12 to CXCR4 and/or CXCR7, the CXCL12 antagonist binding to CXCR4 may be a compound selected from the group comprising the following classes of compounds: aptamers, spiegelmers, antibodies, target binding proteins, target binding peptides, synthetic peptides, peptides, anticalins, a small molecules and fusion proteins.

Various small molecules are disclosed in the prior art which may be used as a CXCL12 antagonist due to their binding to CXCR4 in the method of the present invention. Such small molecules include, but are not limited to, plerixafor (Sanofi), mavorixafor (X4 Pharmaceuticals), Q-122 (Que Oncology), HPH-112 (Harmonic Pharma), BKT-300 (Biokine Therapeutics), X4P-002 (X4 Pharmaceuticals), X4P-003 (X4 Pharmaceuticals), X4-136(X4 Pharmaceuticals), GP-01CR01 (GPCR Therapeutics), GP-01CR11 (GPCR Therapeutics), GP-01CR21(GPCR Therapeutics), Pentixather (Pentixapharm), BKT-170 (Biokine Therapeutics), BMS-585248 (Bristol-Myers Squibb), CS-3955 (Kureha), CTCE-0012 (British Canadian Biosciences), CTCE-9908 (British Canadian Biosciences), GBV-4086 (Globavir Biosciences), HPH-112 (Harmonic Pharma), HPH-211 (Harmonic Pharma), NB-325 (Novaflux Biosciences), SP-10 (Samaritan Pharmaceuticals) and USL-311 (Upsher-Smith Laboratories).

Peptides are disclosed in the prior art which may be used as a CXCL12 antagonist due to their binding to CXCR4 in the method of the present invention. Such peptides include, but are not limited to, motixafortide (BioLineRx).

Synthetic peptides are disclosed in the prior art which may be used as a CXCL12 antagonist due to their binding to CXCR4 in the method of the present invention. Such synthetic peptides include, but are not limited to, balixafortide (Spexis), LY-2510924 (Eli Lilly), ALB-408 (Pharis Biotec) and POL-5551 (Spexis).

Recombinant proteins are disclosed in the prior art which may be used as a CXCL12 antagonist due to their binding to CXCR4 in the method of the present invention. Such recombinant proteins include, but are not limited to, PTX-9098 (Pertinax Therapeutics) and NNL-121 (Nanoligent).

Fusion proteins are disclosed in the prior art which may be used as a CXCL12 antagonist due to their binding to CXCR4 in the method of the present invention. Such recombinant fusion proteins include, but are not limited to, AD-214 (Adalta) and AM-3114 (Adalta).

Antibodies and more specifically monoclonal antibodies are disclosed in the prior art which may be used as a CXCL12 antagonist due to their binding to CXCR4 in the method of the present invention. Such antibodies include, but are not limited to, ulocuplumab (Bristol-Myers Squibb), hz-515H7 (Centre d'Immunologie Pierre Fabre), KY-1051 (Kymab), PF-06747143 (Pfizer), ALX-0651 (Ablynx), AT-009 (Affitech), LY-2624587 (Eli Lilly) and STIA-220X Sorrento Therapeutics).

In another embodiment of the present invention, the CXCL12 antagonist is binding to CXCR7 and inhibits the binding of CXCL12 to CXCR7. In other words, CXCR7 is the target and antigen, respectively, which is bound by this embodiment of a CXCL12 antagonist. As in case of the CXCL12 antagonist binding to CXCL12 and inhibiting the binding of CXCL12 to CXCR4 and/or CXCR7, the CXCL12 antagonist binding to CXCR4 may be a compound selected from the group comprising the following classes of compounds: aptamers, spiegelmers, antibodies, target binding proteins, target binding peptides, synthetic peptides, peptides, anticalins, a small molecules and fusion proteins.

Various small molecules are disclosed in the prior art which may be used as a CXCL12 antagonist due to their binding to CXCR7 in the method of the present invention. Such small molecules include, but are not limited to, ACT-10041239 (Idorsia Pharmaceutical), CCX-650 (ChemoCentryx), CCX-662 (ChemoCentryx) and CCX-771 (ChemoCentryx).

Various synthetic peptides are disclosed in the prior art which may be used as a CXCL12 antagonist due to their binding to CXCR7 in the method of the present invention. Such small synthetic peptides include, but are not limited to, LIH-383 (Luxembourg Institute of Health) and POL-6926 (Spexis).

Various antibodies or antibody fragments are disclosed in the prior art which may be used as a CXCL12 antagonist due to their binding to CXCR7 in the method of the present invention. Such antibodies and antibody fragments include, but are not limited to, JT-07 (Jyant Technologies), X-7Ab (The Palo Alto Research Center) and STIA-230X (Serrento Therapeutics).

It will be appreciated by a person skilled in the art that the amount and dosage, respectively, of the CXCL12 antagonist to be administered to the subject will vary depending upon such factors as the patient's age, weight, height, sex, general medical condition and previous medical history. However, such dosage of the CXCL12 antagonist to be administered to the subject shall preferably be effective in inhibiting neovascularization of the tumor. Preferably, such neovascularization is effected by CXCL12-mediated recruiting of endothelial cells or bone marrow-derived pro-angiogenic cells. More preferably, CXCL12-mediated recruiting is dependent on CXCR4 and/or CXCR7.

It will be appreciated by a person skilled in the art that the exact dosage for administration to the subject in the practicing of the method of the invention can be determined by routine measures.

The way (route of administration and/or formulation) CXCL12 antagonist is administered is defined by the chemical nature of said anti-CXCL12 antagonist. For example, if the CXCL12 antagonist is a spiegelmer, aptamer or an antibody, it will be typically administered by intravenous administration. In accordance with the present invention, the CXCL12 antagonist will be continuously administered to the subject. Even if the CXCL12 antagonist is discontinuously administered to the subject, it is preferred that a therapeutically active level of the CXCL12 antagonist is realized within the patient. It is preferred that such level is a therapeutically active level in terms of anti-vasculogenic and/or immunostimulatory effect in the tumor and/or the tumor environment such as tumor vasculature, and/or the subject's vascular compartment.

In the embodiment where the CXCL12 antagonist is a CXCL12 binding spiegelmer or a CXCL12 binding aptamer, the weekly dose of such CXCL12 antagonist administered to the subject is from about 100 to 1,000 mg, preferably from about 200 to about 600 mg, more preferably from about 400 to about 600 mg. This dose applies in particular if the CXCL12 antagonist is NOX-A12, preferably with NOX-A12 being administered continuously by means of intravenous injection.

According to the method of the present invention, a radiotherapy is administered to the subject suffering from the tumor.

For brain tumors such as glioblastoma, radiotherapy is the most commonly prescribed treatment. It can be used as a primary treatment if a surgeon believes that removing a tumor would be too risky, or after an operation to destroy any remaining cancerous cells that were not visible to or accessible by the surgeon. During radiation therapy for glioblastoma, ionizing magnetic radiation or a particle beam is aimed at a tumor to destroy the cancerous cells and their supportive environment. As cancerous cells and their supportive environment are destroyed and eliminated by the body's immune system, the tumor shrinks; this helps alleviate pressure on the brain.

Radiotherapy is not only directly targeting tumor cells but also depletes the tumor microvasculature. The resulting intra-tumoral hypoxia initiates a chain of events that ultimately leads to re-vascularization, immunosuppression and, ultimately, tumor-regrowth. The key component of this cascade is overexpression of the CXC-motive chemokine ligand 12 (CXCL12), formerly known as stromal-cell derived factor 1 (SDF-1).

In an embodiment of the method of the present invention, radiotherapy as administered to the subject suffering from the tumor is a standard of care radiotherapy. In general, two forms of standard of care radiotherapy schedules exist for brain cancer each of which may be subject to a preferred embodiment of the method of the present invention. One form is normofractionated radiotherapy (nfRT); another form is hypofractionated radiotherapy (hfRT).

In a first embodiment of normofractionated radiotherapy (nfRT), a total of 60 Gy is administered to the subject in 30 sessions a 2 Gy over six weeks, wherein in each week a radiotherapy is administered to the subject at five consecutive days followed by a two day period without administration of any radiotherapy to the subject. Preferably, normofractionated radiotherapy is administered to the subject by means of an external beam radiotherapy (EBRT). More preferably, such normofractionated radiotherapy is administered to the subject in accordance with the guidelines provided by the European Organization for Research and Treatment of Cancer (EORTC), the European Society of Therapeutic Radiology and Oncology (ESTRO) and the Advisory Committee in Radiation Oncology Practice (ACROP), respectively. In accordance with said guidelines the gross tumor volume (GTV) is defined as the resection cavity on the post-operative magnetic resonance imaging (MRI) and planning computed tomography (CT) scan. To create the clinical target volume (CTV), the GTV should be expanded by up to 20 mm in all directions (ideally also covering abnormal fluid-attenuated inversion recovery (FLAIR) signals). It may be reduced at anatomical barriers (e.g. ventricles, falx, tentorium: 5 mm; bone: 0 mm) or critical organs at risk (OAR, e.g. brain stem, chiasm, optic nerves: 0 mm). The planning target volume (PTV) is then defined as the CTV plus an isotropic 3-5 mm margin.

In a second embodiment of normofractionated radiotherapy (nfRT), also a total of 60 Gy is administered to the subject, however, 46 Gy are administered to a first planning target volume 1 (PTV1) in fractions of 2 Gy per session in the initial 23 sessions, followed by 14 Gy to a second planning target volume 2 (PTV2) in fractions of 2 Gy per session in the next 7 sessions (which is also referred to as "boost" or "conedown"). More specifically and in accordance with the guidelines provided by the Radiation Therapy Oncology Group (RTOG), the gross tumor volume 1 (GTV1) resembles the edema (if visible) in T2-FLAIR sequences and must also cover the complete resection cavity and any contrast-enhancing lesion in the T1-post contrast sequences. The clinical target volume 1 (CTV1) is then defined as the GTV1 plus a 2 cm margin. The CTV1 may be reduced around natural margins for tumors (e.g., skull, falx, etc.). The planning target volume 1 (PTV1) then resembles the CTV1 plus an additional margin of 2-5 mm. All (T1-)contrast-enhancing lesions/abnormalities (which must include the cavity margins) will be defined as boost volume (GTV2). The boost clinical target volume (CTV2) will be the GTV2 plus a margin of 2 cm. The CTV2 may be reduced around natural margins for tumors (e.g., skull, falx, etc.). The boost planning target volume (PTV2) resembles the CTV2 plus an additional margin of 2-5 mm. In connection with the above it will be appreciated by a person skilled in the art that factually any glioblastoma has an edema. The edema is one of the main reasons for increased intracranial pressure that leads to typical glioblastoma symptoms. Because tumor cells are more likely to infiltrate this damages region, the edema region is also irradiated. Further, it will be acknowledged that T2 FLAIR is particularly suitable to detect edema because it allows to differentiate between free and tissue-bound liquid. T2 refers to the transversal relaxation. The FLAIR (Fluid Attenuated Inversion Recovery) sequence is a particular MRI sequence which is very similar to the T2 sequence but it is normalized to the signal of the liquor in the brain which is set to zero. The liquor will thus appear black.

In hypofractionated radiotherapy (hfRT), the subject suffering from the tumor will receive a total dose of about 40 Gy, typically 40.05 Gy, administered in 15 daily fractions over a period of 3 weeks. Typically, this means that in each and any of said three weeks, 2.67 Gy are each administered to the subject at five consecutive days followed by two days where no radiotherapy is administered to the subject. More specifically and in accordance with the guidelines provided by the Canadian Cancer Society Research Institute, the gross tumor volume (GTV) is defined as the contrast-enhancing volume on the postsurgical planning magnetic resonance imaging (MRI) scan and included the surgical bed. The clinical target volume (CTV) is a 1.5-cm margin respecting anatomical boundaries beyond the gross-tumor-volume contour, and a planning target volume (PTV) margin of 0.5 cm is applied.

Radiotherapy may make use of ionizing electromagnetic radiation or a ionizing particle beam. Such ionizing electromagnetic radiation can be X-rays or gamma-rays. Typically, X-rays have a wavelength from about 10 picometers to about 10 nanometers, and the energy of X-rays is from about 145 eV to about 124 keV. As to gamma-rays, their wavelength is typically 10 picometer or less and, the energy of gamma-rays is typically 100 keV or more.

Such ionizing particle beam may be a proton beam, a photon beam or an electron beam, whereby, preferably, such particle beam is used for External Beam Radiation Therapy (EBRT). In an embodiment of the present invention, EBRT, including any facets thereof, is used in the radiotherapy administered to the subject suffering from the tumor in accordance with the present invention.

In External Beam Radiotherapy (EBRT), the tumor is exposed to an external source of ionizing radiation megavoltage X-rays are used to treat deep-seated tumors such as GBM. Such X-rays or photon beams are produced in most radiation therapy machines. Photon beams can reach tumors deep in the body. As they travel through the body, photon beams scatter little bits of radiation along their path. These beams do not stop once they reach the tumor but go into normal tissue past it. Megavoltage X-rays have 1 to 25 MeV. Such megavoltage X-rays are produced in linear accelerators (linacs). Commercially available medical linacs produce X-rays with an energy range from 4 MeV up to around 25 MeV whereas in clinical practice, nominal energies above 15 MeV are unusual, thus an energy range from 4-15 MeV is the preferred range. The X-rays themselves are produced by the rapid deceleration of electrons in a target material, typically a tungsten alloy, which produces an X-ray spectrum via bremsstrahlung radiation. The shape and intensity of the beam produced by a linac may be modified or collimated by a variety of means.

Alternatively, like photon beams, proton beams can also reach tumors deep in the body and are sometimes used in recurrent brain tumor and recurrent glioblastoma in particular. However, proton beams do not scatter radiation on their path through the body and they stop once they reach the tumor. Proton beams are thus thought to reduce the amount of normal tissue that is exposed to radiation. In general, the high cost and size of the machines are limiting their use.

It will be appreciated by a person skilled in the art that radiotherapy may also make use of "heavier" particles such as protons or He ions, so as to generate radiotherapy effects. The use of such heavier particles constitutes another embodiment of the method of the present invention.

EBRT may be administered to the subject in various ways. Some of such ways are outlined in the following and constitute further embodiments of the method of the present invention, namely 3-D conformal radiation therapy, Intensity-Modulated Radiation Therapy (IMRT), Image-Guided Radiation Therapy (IGRT), and stereotactic radiosurgery.

3-D conformal radiation therapy is a common type of EBRT. It uses images from CT, MRI, and PET scans to precisely plan the treatment area. Images are analyzed and a radiation field is designed to conform to the shape of the tumor.

Intensity-modulated radiation therapy (IMRT) is a more advanced form of 3-D conformational radiotherapy. It uses sophisticated software and 3-D images from CT scans to focus radiation directly on a tumor. These pencil-thin beams vary in intensity (so as to provide higher doses to certain parts of the tumor) and conform to the specific shape and size of the tumor. This highly focused approach reduces radiation exposure to healthy tissue in the brain. IMRT can also decrease the chance of side effects

Image-guided radiation therapy (IGRT) or tomography is even more advanced than IMRT by using imaging scans not only for treatment planning before radiation therapy sessions but also during radiation therapy sessions so as to guide the placement of the radiation beams.

Stereotactic radiosurgery is the use of focused, high-energy beams to treat small tumors with well-defined edges in the brain and central nervous system. This precise treatment positions a radioactive source closer to the tumor than would be possible with conventional radiation therapy. Because of this, it may be an option if surgery is too risky due to age or other health problems or if the tumor cannot safely be reached with surgery. While traditional glioblastoma radiation therapy involves numerous sessions scheduled over the course of several months, stereotactic radiosurgery is delivered in fewer sessions.

In another embodiment of the method of the present invention, radiotherapy is brachytherapy. Brachytherapy is a type of local, internal radiation therapy in which seeds, ribbons, or capsules that contain a radiation source or liquid formulations containing a radiation source are placed in or near the tumor, preferably in the resection cavity after surgery. It is often used to treat cancers of the head and neck, breast, cervix, prostate, and eye, but is also applicable in the treatment of brain tumors. Basically, there three types of brachytherapy: (a) low-dose rate (LDR) implants where the radiation source stays in place for 1 to 7 days, (b) high-dose rate (HDR) implants where the radiation source is left in place for just 10 to 20 minutes per session; and (c) permanent implants, where radiation source remains in place for indefinite time, but the radiation gets weaker each day. In an embodiment, such radiation source is a therapeutically active radionuclide. In a preferred embodiment, the therapeutically active radionuclide is selected from the group comprising ¹⁸⁶Re, ⁴⁷Sc, ⁶⁷Cu, ⁸⁹Sr, ⁹⁰Y, ¹⁵³Sm, ¹⁴⁹Tb, ¹⁶¹Tb, ¹⁷⁷Lu, ¹⁸⁸Re, ²¹²Pb, ²¹³Bi, ²²³Ra, ²²⁵Ac, ²²⁶Th, ²²⁷Th, ¹³¹I, ²¹¹At, more preferably the therapeutically active radionuclide is selected from the group comprising ¹⁸⁶Re, ⁴⁷Sc, ⁶⁷Cu, ⁹⁰Y, ¹⁷⁷Lu, ¹⁸⁸Re, ²¹²Pb, ²¹³Bi, ²²⁵Ac, ²²⁷Th, ¹³¹I, ²¹¹At and most preferably the therapeutically active radionuclide is selected from the group comprising ¹⁸⁶Re, ⁹⁰Y, ¹⁷⁷Lu, ²²⁵Ac, ²²⁷Th, ¹³¹I and ²¹¹At.

In an embodiment of the method of the present invention, a modified brachytherapy is used. Such modified brachytherapy doesn't use solid sources of radiation that are implanted but injection of nanoliposomes that contain ¹⁸⁶Re which is a beta ray-emitting therapeutic radionuclide with a 90-hour half-life, 1.8-mm radiation path range, and high β/γ-energy ratio suitable for cancer brachytherapy. The ¹⁸⁶Re nanoliposomes are formulated to enhance the delivery profile of long half-life ¹⁸⁶Re energy to achieve long term tumor retention and achieve very high absorbed radiation doses to tumors. Preclinically, such nanoliposomes infused via convection enhanced delivery achieves very high doses of targeted radiation and a wide therapeutic index. Such modified brachytherapy is, for example, described in Floyd 2021, Int. J. Radiat. Oncol. Biol. Phys. Volume 111, Issue 3, Supplement, 1 November 2021, Page e589.

In another embodiment of the method of the present invention, radiotherapy is intraoperative radiotherapy (IORT). Intraoperative radiation therapy (IORT) constitutes delivery of radiation to the tumour/tumour bed while the area is exposed during surgery. IORT is capable of delivering high doses of radiation, precisely to the tumour bed with minimal exposure to the surrounding healthy tissues. It will be appreciated by a person skilled in the art that IORT is typically combined with external beam radiotherapy and allows avoiding repopulation of tumor cells at the site where a tumor has been resected or otherwise treated.

IORT allows (i) precise localisation of the tumour bed and targeted delivery of high-dose radiation to the tumour bed; (ii) minimal exposure of the dose-limiting normal tissues that are displaced away from the tumour bed and shielded from radiation; (iii) opportunities for dose escalation beyond that which can be achieved with EBRT; and (iv) opportunities for re-irradiation especially in recurrent cancers where further irradiation with EBRT may not be possible. Thus, IORT can deliver higher total effective dose to the tumour bed, facilitate dose escalation without significantly increasing normal tissue complications and improve therapeutic ratio compared with EBRT. Methods of IORT are (i) electron IORT/IOERT (electron beams), (ii) kV IORT (X-rays), and (iii) HDR IORT (High-dose-rate brachytherapy).

It is within the present invention that the method for treating a tumor comprises an anti-angiogenic therapy. Preferably such anti-angiogenic therapy comprises the administration of an anti-angiogenic compound to the subject suffering from the tumor.

It will be appreciated by a person skilled in the art that this part of the method of the present invention, i.e. administering an anti-angiogenic therapy to the subject suffering from the tumor, shall avoid or a least reduce angiogenesis. As preferably used herein, angiogenesis is the formation of new blood vessels within and/or towards the tumor. More preferably, angiogenesis starts from a pre-existing vessel.

Formation of new blood vessels is an indispensable prerequisite for tumor growth as also malign tissues are dependent on nutrients and oxygen. Very small tumors can be supplied by diffusion, but as soon as a tumor gets larger than 1-2 mm in diameter, further growth requires an own vascular system. In order to generate such system, tumors secrete various growth factors that stimulate the formation of blood vessels (angiogenic switch). The most prominent growth factor for angiogenesis in tumors is VEGF (vascular endothelial growth factor). By blocking the signaling of VEGF via its receptors VEGFR1 (Flt-1), VEGFR2 (KDR/Flk-1), endothelial cell proliferation and subsequent formation of new blood vessels in the tumor is inhibited. In most normal tissues there is a very low or undetectable expression of the VEGF receptors, whereas VEGF is upregulated in most human tumor types. VEGF expression is associated with tumor progression or patient survival in a variety of human cancers.

In light thereof, it will be acknowledged by a person skilled in the art that inhibiting the interaction of VEGF and at least one of its receptors VEGFR1 and VEGFR2, preferably inhibiting the binding of VEGF to at least one of its receptors VEGFR1 and VEGFR2 results in an anti-angiogenic effect which is therapeutically effective. It will be appreciated by a person skilled in the art that such inhibition can be achieved by (a) providing a molecule binding to VEGF so as to inhibit the binding of VEGF to VEGFR1 and/or VEGFR2, providing a molecule binding to VEGFR1 so as to inhibit the binding of VEGF to VEGFR1, and (c) providing a molecule binding to VEGFR2 so as to inhibit the binding of VEGF to VEGFR2.

Such molecule which is binding to VEGF is preferably one selected from the group comprising an aptamer, preferably a VEGF binding aptamer, a spiegelmer, preferably a VEGF binding antibody, a protein binding to VEGF, a VEGF binding anticalin, a VEGF binding peptide, a fusion protein comprising a VEGF binding moiety and a VEGF binding small molecule.

Such molecule which is binding to VEGFR1 is preferably one selected from the group comprising an aptamer, preferably a VEGFR1 binding aptamer, a spiegelmer, preferably a VEGFR1 binding antibody, a protein binding to VEGFR1, a VEGFR1 binding anticalin, a VEGFR1 binding peptide, a fusion protein comprising a VEGFR1 binding moiety and a VEGFR1 binding small molecule.

Such molecule which is binding to VEGFR2 is preferably one selected from the group comprising an aptamer, preferably a VEGFR2 binding aptamer, a spiegelmer, preferably a VEGFR2 binding antibody, a protein binding to VEGFR2, a VEGFR2 binding anticalin, a VEGFR2 binding peptide, a fusion protein comprising a VEGFR2 binding moiety and a VEGFR2 binding small molecule.

It will also be appreciated by a person skilled in the art that that such inhibition of the interaction of VEGF via one or both of VEGF receptor VEGFR1 and VEGFR2 can be achieved a compound binding VEGFR1 and/or VEGFR2, whereby such binding of the compound interferes with the signaling of VEGFR1 and/or VEGFR2 rather than interfering with the binding of VEGF to VEGFR1 and/or VEGFR2.

A molecule which is binding to VEGFR1 and because of its binding to VEGFR1 is interfering with the signal transduction characteristics of VEGFR1, preferably signal transduction triggered by the binding of VEGF to VEGFR1, is preferably one selected from the group comprising an aptamer, preferably a VEGFR1 binding aptamer, a spiegelmer, preferably a VEGFR1 binding antibody, a protein binding to VEGFR1, a VEGFR1 binding anticalin, a VEGFR1 binding peptide, a fusion protein comprising a VEGFR1 binding moiety and a VEGFR1 binding small molecule.

The generation and identification, respectively, of such aptamer, spiegelmer, antibody, binding protein, anticalin, peptide, fusion protein and small molecule is known to a person skilled in the art and also disclosed herein.

In an embodiment, VEGF is human VEGF, VEGFR1 is human VEGFR1, and VEGFR2 is human VEGF2.

VEGF exists as a full-length form, but also as isoforms generated by alternative splicing or proteolytic cleavage by proteinases plasmin, MMP3 and uPA. Such isoforms and proteolytically cleaved forms of VEGF are, for example, disclosed in Vempati et al. (Vempati P et al., Cytokine Growth Factor Rev. 2014 Feb; 25(1). 1-19) and Guyot M and Pages G (Guyot M and Pages G, Methods Mol. Biol 2015; 1332:2-23). Isoforms are disclosed in particular in Guyot M and Pages G and amino acid sequences of different forms of VEGF depicted in Fig. 20. An isoform of VEGF is preferably one selected from the group comprising VEGF121, VEGF165, VEGF189, VEGF206, VEGF145, VEGF183 and VEGF165b. The proteolytically cleaved form of VEGF is VEGF 110. It will be appreciated by a person skilled in the art that whenever reference is made herein to VEGF and a compound binding to or interfering with the function of VEGF, such VEGF is, in an embodiment, any one of the above full-length VEGF, isoforms of VEGF or a proteolytically cleaved form thereof. It will be acknowledged by a person skilled in the art that it is within the present invention that a molecule binding to VEGF may be binding to the form of VEGF which was used in the generation and identification, respectively, of such binding molecule, and, additionally to one or more of the above mentioned forms of VEGF, including any isoform or proteolytically cleaved form.

In a preferred embodiment, the anti-angiogenic compound is an anti-VEGF antibody. The anti-VEGF antibody may be a human, humanized or chimeric anti-VEGF antibody. The term anti-VEGF antibody as preferably used herein, also encompassed any fragment of such anti-VEGF antibody, whereby such fragment is still binding or still capable of binding to VEGF.

In a preferred embodiment, the anti-angiogenic antibody comprises the following light chain CDRS: CDR1: QDISNY, CDR2: FTS, and CDR3: QQYSTVPWT; or the following heavy chain CDRS: CDR1: GYTFTNYG, CDR2: INTYTGEP, and CDR3: AKYPHYYGSSHWYFDV, or a combination of the following light chain CDRS: CDR1: QDISNY, CDR2: FTS, and CDR3: QQYSTVPWT; and the following heavy chain CDRS: CDR1: GYTFTNYG, CDR2: INTYTGEP, and CDR3: AKYPHYYGSSHWYFDV.

In another preferred embodiment, the anti-angiogenic antibody comprises a light chain comprising the following amino acid sequence: 
DIQMTQSPSSLSASVGDRVTITCSASQDISNYLNWYQQKPGKAPKVLIYFTSSLHSGVPSRFSG
SGSGTDFTLTISSLQPEDFATYYCQQYSTVPWTFGQGTKVEIKRTVAAPSVFIFPPSDEQLKSG
 
TASVVCLLNNFYPREAKVQWKVDNALQSGNSQESVTEQDSKDSTYSLSSTLTLSKADYEKH
KVYACEVTHQGLSSPVTKSFNRGEC,
 a heavy chain comprising the following amino acid sequence: 
EVQLVESGGGLVQPGGSLRLSCAASGYTFTNYGMNWVRQAPGKGLEWVGWINTYTGEPTY
AADFKRRFTFSLDTSKSTAYLQMNSLRAEDTAVYYCAKYPHYYGSSHWYFDVWGQGTLVT
VSSASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGGPSVF
LFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSREEMTKNQVS
LTCLVKGFYPSDIAVEWESNGQPENNYKTTPPVLDSDGSFFLYSKLTVDKSRWQQGNVFSCS
VMHEALHNHYTQKSLSLSPGK, or
 a combination of a light chain comprising the following amino acid sequence: 
DIQMTQSPSSLSASVGDRVTITCSASQDISNYLNWYQQKPGKAPKVLIYFTSSLHSGVPSRFSG
SGSGTDFTLTISSLQPEDFATYYCQQYSTVPWTFGQGTKVEIKRTVAAPSVFIFPPSDEQLKSG
TASVVCLLNNFYPREAKVQWKVDNALQSGNSQESVTEQDSKDSTYSLSSTLTLSKADYEKH
KVYACEVTHQGLSSPVTKSFNRGEC, and
 a heavy chain comprising the following amino acid sequence:

EVQLVESGGGLVQPGGSLRLSCAASGYTFTNYGMNWVRQAPGKGLEWVGWINTYTGEPTY
AADFKRRFTFSLDTSKSTAYLQMNSLRAEDTAVYYCAKYPHYYGSSHWYFDVWGQGTLVT
VSSASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGGPSVF
LFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSREEMTKNQVS
LTCLVKGFYPSDIAVEWESNGQPENNYKTTPPVLDSDGSFFLYSKLTVDKSRWQQGNVFSCS
VMHEALHNHYTQKSLSLSPGK.

In a preferred embodiment, the anti-VEGF-antibody is Bevacizumab. Bevacizumab is a recombinant humanized monoclonal antibody to VEGF; USAN/BAN/JAN Name: Bevacizumab; Laboratory Code: RO487-6646; CAS Registry Number: 216974-75-3; Bevacizumab is also referred to as rhuMAb VEGF, and anti-VEGF.

Bevacizumab is an inhibitor of angiogenesis. As such, it prevents the formation of new vasculature. Bevacizumab binds selectively with high affinity to all isoforms of human VEGF, the key driver of angiogenesis, and thereby inhibits the binding of VEGF to its receptors (Flt-1, VEGFR1 and KDR, VEGFR2) on the surface of endothelial cells. Neutralizing the biological activity of VEGF through a steric blocking of the binding of VEGF to its receptors regresses the vascularization of tumors, normalizes remaining tumor vasculature, and inhibits the formation of new tumor vasculature, thereby inhibiting tumor growth. Receptors activation normally induces their tyrosine phosphorylation and the subsequent series of signal transduction events elicit mitogenic and pro-survival activity signals for the vascular endothelial cells. Since there is a very low or undetectable expression of VEGF receptors in most normal tissues (with exception of renal glomeruli) but a significant up-regulation in the vasculature of many tumors, the neutralization of VEGF by bevacizumab provides the rationale a relative specific inhibition of the tumor angiogenesis and thereby inhibition of tumor growth and metastasizing.

Bevacizumab is a recombinant humanized monoclonal IgG1κ isotype antibody that contains human framework regions (93%) and murine complementarity-determining regions (7%). The antibody is composed of two identical light chains (214 amino acid residues) and two heavy chains (453 residues) with a total molecular weight of 149 kDa. The heavy chains demonstrate C-terminal heterogeneity (lysine variants) and also contain one N-linked glycosylation site at asparagine 303. The oligosaccharides are of complex biantennary structures with a core fucose and with the two branches terminating mainly with zero (G0), one (G1) or two (G2) galactose residues. The G0 glycoform predominates at approximately 80 % relative abundance. Each light chain is covalently coupled through a disulfide bond at cysteine 214 to a heavy chain at cysteine 226. The two heavy chains are covalently coupled to each other through two inter-chain disulfide bonds, which is consistent with the structure of a human IgG1. Bevacizumab was generated by humanization of the murine parent antibody A4.6.1 which was produced at Genentech using hybridomas generated from mice immunized with the 165-residue-form of recombinant human vascular endothelial growth factor (rhuVEGF165) conjugated with keyhole limpet hemocyanin.

The humanization of the A4.6.1 antibody involved insertion of the six CDRs of A4.6.1, in place of those of a selected human antibody Fab framework (pEMX1), which has a consensus human kappa subgroup I light chain (domains VL-CL) and a truncated human subgroup III immunoglobulin gamma (IgG1) heavy chain (domains VH-CH1). A series of framework residue substitutions were made to produce the final humanized version, Fab-12, which contains eight substitutions of the human framework outside of the CDRs. The VH and VL domains of Fab-12 were combined with human IgG1 constant domains CH1-CH2-CH3 and CL, respectively, to produce bevacizumab. The expression plasmid pSVID5.ID.LLnspeV.xvegf36HC.LC encoding bevacizumab was introduced into Chinese hamster ovary parental cells CHO DP-12 by lipofection and cells were selected in the presence of increasing concentrations of methotrexate (MTX). Isolates were selected for secretion of active bevacizumab. Isolated subclone 107N was used for the production of phase I and phase II clinical materials and as the starting point for the development of the more highly productive G7 cell line, which was used for the production of phase III clinical materials and which will be used for the production of drug substance intended for marketing.

In another embodiment, the anti-VEGF antibody is ranibizumab (Lucentis; also Roche). The CDRs of bevacizumab and ranibizumab are very similar but have few changes (see IMGT Repertoire (IG and TR obtainable from https://www.imgt.org/IMGTrepertoire/GenesClinical/humanized/bevacizumab/bevacizumab_ProteinD isplay.html). Ranibizumab is the Fab fragment of bevacizumab. In ranibizumab, 6 amino acids are modified thereof 4 in the CDRs. Ranibizumab is not glycosylated. More specifically, ranibizumab has the following amino acid changes compared to bevacizumab: in the VH (CDR1-IMGT: T29→D, N36→H, CDR3-IMGT: H109→Y, S112→T) and one amino acid change in V-KAPPA (M4→L). There is also an additional change in the hinge (T10>L) of bevacizumab.

In a still other embodiment, the anti-angiogenic compound binding to VEGF is a VEGF-binding protein. Preferably such VEGF-binding protein is a functional derivative of a VEGF receptor, more preferably of VEGFR1 and/or VEGFR2. In a particularly preferred embodiment, the anti-angiogenic compound is ziv-aflibercept (Zaltrap), a 'VEGF trap' that inhibits VEGF-A, VEGF-B and PlGF-2. Zaltrap which is also referred to as aflibercept or also known as VEGF TRAP in the scientific literature, is a recombinant fusion protein consisting of VEGF-binding portions from the extracellular domains of human VEGF receptors 1 and 2 fused to the Fc portion of the human IgG1. Aflibercept is produced by recombinant DNA technology in a Chinese hamster ovary (CHO) K-1 mammalian expression system. Aflibercept is a dimeric glycoprotein with a protein molecular weight of 97 kilodaltons (kDa) and contains glycosylation, constituting an additional 15 % of the total molecular mass, resulting in a total molecular weight of 115 kDa. Aflibercept acts as a soluble decoy receptor that binds to VEGF-A, with higher affinity than its native receptors, as well as the related ligands PlGF and VEGF-B. By acting as a ligand trap, aflibercept prevents binding of endogenous ligands to their cognate receptors and thereby blocks receptor mediated signaling. Aflibercept blocks the activation of VEGF receptors and the proliferation of endothelial cells, thereby inhibiting the growth of new vessels that supply tumours with oxygen and nutrients. Aflibercept binds to human VEGF-A (equilibrium dissociation constant KD of 0.5 pM for VEGF-A165 and 0.36 pM for VEGF-A121), to human PlGF (KD of 39 pM for PlGF-2), and to human VEGF-B (KD of 1.92 pM) to form a stable, inert complex which has no detectable biological activity (see, European Medicines Agency, entry related to zaltrap: https://www.ema.europa.eu/en/medicines/human/EPAR/zaltrap).

In an embodiment of the method of the present invention, the anti-angiogenic compound is a compound binding to a VEGFR. VEGFR may be VEGFR1, VEGFR2 or a combination thereof. In a more preferred embodiment, the anti-angiogenic compound binding to a VEGFR is an anti-VEGFR antibody.

In an embodiment, the anti-VEGFR antibody comprises the following light chain CDRs CDR1: RASQGID NWLG, CDR2: DASNLDT, and CDR3: QQ AKAFPPT, or the following heavy chain CDRs CDR1: GFTFS SYSMN, CDR2: S ISSSSSYIYY ADSVKG and CDR3: VTDAFDI, or the following light chain CDRs CDR1: RASQGID NWLG, CDR2: DASNLDT, and CDR3: QQ AKAFPPT, the following heavy chain CDRs CDR1: GFTFS SYSMN, CDR2: S ISSSSSYIYY ADSVKG and CDR3: VTDAFDI.

In an embodiment, the anti-VEGFR antibody comprises the following light chain DIQMTQSPSS VSASIGDRVT ITCRASQGID NWLGWYQQKP GKAPKLLIYD ASNLDTGVPS RFSGSGSGTY FTLTISSLQA EDFAVYFCQQ AKAFPPTFGG GTKVDIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC, or the following EVQLVQSGGG LVKPGGSLRL SCAASGFTFS SYSMNWVRQA PGKGLEWVSS ISSSSSYIYY ADSVKGRFTI SRDNAKNSLY LQMNSLRAED TAVYYCARVT DAFDIWGQGT MVTVSSASTK GPSVLPLAPS SKSTSGGTAA LGCLVKDYFP EPVTVSWNSG ALTSGVHTFP AVLQSSGLYS LSSVVTVPSS SLGTQTYICN VNHKPSNTKV DKRVEPKSCD KTHTCPPCPA PELLGGPSVF LFPPKPKDTL MISRTPEVTC VVVDVSHEDP EVKFNWYVDG VEVHNAKTKP REEQYNSTYR VVSVLTVLHQ DWLNGKEYKC KVSNKALPAP IEKTISKAKG QPREPQVYTL PPSREEMTKN QVSLTCLVKG FYPSDIAVEW ESNGQPENNY KTTPPVLDSD GSFFLYSKLT VDKSRWQQGN VFSCSVMHEA LHNHYTQKSL SLSPGK, or a combination of the following light chain CDRs CDR1: RASQGID NWLG, CDR2: DASNLDT, and CDR3: QQ AKAFPPT, or the following heavy chain CDRs CDR1: GFTFS SYSMN, CDR2: S ISSSSSYIYY ADSVKG and CDR3: VTDAFDI, or the following light chain CDRs CDR1: RASQGID NWLG, CDR2: DASNLDT, and CDR3: QQ AKAFPPT, the following heavy chain CDRs CDR1: GFTFS SYSMN, CDR2: S ISSSSSYIYY ADSVKG and CDR3: VTDAFDI.

In an embodiment, the anti-VEGFR antibody comprises the following light chain DIQMTQSPSS VSASIGDRVT ITCRASQGID NWLGWYQQKP GKAPKLLIYD ASNLDTGVPS RFSGSGSGTY FTLTISSLQA EDFAVYFCQQ AKAFPPTFGG GTKVDIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC, and the following EVQLVQSGGG LVKPGGSLRL SCAASGFTFS SYSMNWVRQA PGKGLEWVSS ISSSSSYIYY ADSVKGRFTI SRDNAKNSLY LQMNSLRAED TAVYYCARVT DAFDIWGQGT MVTVSSASTK GPSVLPLAPS SKSTSGGTAA LGCLVKDYFP EPVTVSWNSG ALTSGVHTFP AVLQSSGLYS LSSVVTVPSS SLGTQTYICN VNHKPSNTKV DKRVEPKSCD KTHTCPPCPA PELLGGPSVF LFPPKPKDTL MISRTPEVTC VVVDVSHEDP EVKFNWYVDG VEVHNAKTKP REEQYNSTYR VVSVLTVLHQ DWLNGKEYKC KVSNKALPAP IEKTISKAKG QPREPQVYTL PPSREEMTKN QVSLTCLVKG FYPSDIAVEW ESNGQPENNY KTTPPVLDSD GSFFLYSKLT VDKSRWQQGN VFSCSVMHEA LHNHYTQKSL SLSPGK.

In another embodiment, the anti-angiogenic compound binding to VEGF is an aptamer, i.e. a D-nucleic acid, more specifically pegaptanib (Macugen). Pegaptanib (Macugen) is an anti-VEGF aptamer, developed as an anti-angiogenic medicine for the treatment of neovascular (wet) age-related macular degeneration. Pegaptanib is a pegylated aptamer that specifically binds to the 165 isoform of VEGF.

More preferably, the anti-VEGFR antibody is Ramucirumab (Cyramza), which is a specific VEGFR2 inhibitor. The amino acid sequence of the heavy chain of ramucirumab is 
EVQLVQSGGG LVKPGGSLRL SCAASGFTFS SYSMNWVRQA PGKGLEWVSS ISSSSSYIYY
ADSVKGRFTI SRDNAKNSLY LQMNSLRAED TAVYYCARVT DAFDIWGQGT MVTVSSASTK
GPSVLPLAPS SKSTSGGTAA LGCLVKDYFP EPVTVSWNSG ALTSGVHTFP AVLQSSGLYS
LSSVVTVPSS SLGTQTYICN VNHKPSNTKV DKRVEPKSCD KTHTCPPCPA PELLGGPSVF
LFPPKPKDTL MISRTPEVTC VVVDVSHEDP EVKFNWYVDG VEVHNAKTKP REEQYNSTYR
VVSVLTVLHQ DWLNGKEYKC KVSNKALPAP IEKTISKAKG QPREPQVYTL PPSREEMTKN
QVSLTCLVKG FYPSDIAVEW ESNGQPENNY KTTPPVLDSD GSFFLYSKLT VDKSRWQQGN
VFSCSVMHEA LHNHYTQKSL SLSPGK;
 and the light chain of ramucirumab is

DIQMTQSPSS VSASIGDRVT ITCRASQGID NWLGWYQQKP GKAPKLLIYD ASNLDTGVPS
RFSGSGSGTY FTLTISSLQA EDFAVYFCQQ AKAFPPTFGG GTKVDIKRTV AAPSVFIFPP
SDEQLKSGTA SVVCLLNNFY PREAKVQWKV DNALQSGNSQ ESVTEQDSKD STYSLSSTLT
LSKADYEKHK VYACEVTHQG LSSPVTKSFN RGEC.

These sequences may be taken from the information provided by the following link: https://www.genome.jp/entry/D09371.

In another embodiment of the method of the present invention, the anti-angiogenic compound is a compound which inhibits signaling of a VEGFR, preferably VEGFR1 and VEGFR2. It is, however, also within the present invention that the signaling of one or more of the following receptors is inhibited: platelet-derived growth factor receptor (PDGFR), fibroblast growth factor receptor (FGFR) and angiopoietin-1 receptor (Tie-2).

In a preferred embodiment, the anti-angiogenic compound inhibits the signal cascade triggered upon binding of VEGF to the VEGFR. Assays used for determining whether a compound inhibits said signal cascade, are known in the art. More specifically, cell culture assays have been used for such purpose and the characterization of inhibitors of VEGFR. The assays detect activation of intracellular signaling pathways, which are in the case of VEGFR the Ras/MAPK pathway, regulating cell proliferation and gene expression; the FAK/paxillin pathway, involved in the rearrangement of the cytoskeleton; the PI3K/AKT pathway, regulating cell survival; the PLCγ pathway, controlling vascular permeability.

More preferably, the anti-angiogenic compound inhibits a tyrosine kinase activity of the VEGFR. In such embodiment, the anti-angiogenic compound is a tyrosine kinase inhibitor, more preferably a multi-tyrosine kinase inhibitor.

In a more preferred embodiment, the anti-angiogenic compound being a tyrosine kinase inhibitor is selected from the group comprising the tyrosine kinase inhibitor is selected from the group comprising pazopanib (Votrient inhibiting VEGFR1-3, PDGFRα and β, FGFR1 and 3, KIT, ITK, LCK, c-FMS), sunitinib (Sutent inhibiting VEGFR1-3, PDGFRα and β, KIT, FLT-3, CSF-1R, RET,), sorafenib (Nexavar inhibiting intracellular (c-RAF, BRAF and mutant BRAF) and cell surface kinases (KIT, FLT-3, RET, RET/PCT, VEGFR1-3, PDGFR-β)), axitinib (Inlyta inhibiting VEGFR1-3), ponatinib (Iclusig inhibiting VEGFR, PDGFR, FGFR, EPH receptors, SCR family of kinases, KIT, RET, TIE2 and FLT3), cabozantinib (Cometriq/Cabometyx inhibiting RET, MET, VEGFR1-3, KIT, TKRB, FLT-3, AXL, ROS1, TYRO3, MER, TIE-2), regorafenib (Stivarga inhibiting RET, VEGFR1-3, KIT, PDGFRα and β, FGFR1-2, TIE2, DDR2, TRKA, EPH2A, RAF-1, BRAF, BRAF V600E, SAPK2, PTKS, ABL, CSF1R), vandetanib (Caprelsa inhibiting EGFR and VEGFR families, RET, BRK, TIE-2, members of EPHR and Src kinase families) and lenvatinib (Lenvima inhibiting VEGFR1-3, FGFR1-4, PDGFRα, KIT and RET).

It will be appreciated by a person skilled in the art that the amount and dosage, respectively, of the anti-angiogenic compound to be administered to the subject will vary depending upon such factors as the patient's age, weight, height, sex, general medical condition and previous medical history. However, such dosage of the anti-angiogenic compound to be administered to the subject shall preferably be effective in inhibiting neovascularization of the tumor. Preferably, such neovascularization is effected by the interaction of VEGF with VEGFR1 and/or VEGFR2.

It will be appreciated by a person skilled in the art that the exact dosage for administration of the anti-angiogenic compound to the subject in the practicing of the method of the invention can be determined by routine measures.

The way (route of administration and/or formulation) the anti-angiogenic compound is administered is defined by the chemical nature of said the anti-angiogenic compound. For example, if the anti-angiogenic compound is a spiegelmer, aptamer or an antibody, it will be typically administered by intravenous administration. In accordance with the present invention, the anti-angiogenic compound will be discontinuously administered to the subject.

In the embodiment where the anti-angiogenic compound is an antibody such as an anti-VEGF antibody and bevacizumab in particular, such antibody is administered by intravenous infusion on a weekly, biweekly or threeweekly basis, preferably a biweekly or threeweekly basis. It will be appreciated by a person skilled in the art that the dosing scheme for the anti-angiogenic compound and the anti-VEGF antibody such as bevacizumab is synchronized with the therapy said anti-angiogenic compound is combined with.

In an embodiment, the method of the invention comprises a chemotherapy. In a preferred embodiment, such chemotherapy is a chemotherapy as used in the treatment of glioma and glioblastoma in particular, and more preferably a chemotherapy used as a standard of care in the treatment of glioma and glioblastoma.

In an embodiment, the chemotherapy comprises temozolomide (TMZ). TMZ is preferably administered every day during radiation therapy (6 weeks at 75 mg/m² daily) and then for six cycles after radiation during the maintenance phase. Each cycle of the maintenance phase lasts for 28 days, with temozolomide given the first five days of each cycle at 150-200 mg/m² daily followed by 23 days of rest. In a preferred embodiment, the tumor of the subject is sensitive to temozolomide.

In another embodiment, the chemotherapy comprises Lomustine (CCNU). A common scheme for CCNU-based chemotherapy is a dose of 110 mg/m²/day on day 1 of a 42-day cycle as per local standards, whereby treatment then continues for up to 6 total cycles. In a further embodiment, CCNU is used in the treatment of recurrent glioblastoma.

In another embodiment, the chemotherapy comprises procarbazine/ lomustine/ vincristine (PCV) or bevacizumab/irinotecan (BI) (Carvalho 2015, Oncol Res Treat 38:348). In a further embodiment, such chemotherapy is used in the treatment of recurrent glioblastoma.

In still another embodiment, the chemotherapy comprises dianhydrogalactitol (VAL-083) which is an alkylating agent (DNA synthesis inhibitor). Subjects suffering from brain tumor and glioblastoma in particular may receive dianhydrogalactitol in the maintenance period after radiotherapy and temozolomide (75 mg/m² TMZ orally daily during radiation therapy). Maintenance period: VAL-083 IV at 30 mg/m²) on Day 1, 2 and 3 of 21-day cycle.

In an embodiment of the present invention, the subject is a human subject.

In an embodiment of the present invention, an antibody may be a human, a humanized or a chimeric antibody. Preferably, the antibody is a monoclonal antibody (MAb).

In an embodiment of the present invention, an antibody may be a human, a humanized or a chimeric antibody, but are not limited thereto.

In an embodiment of the present invention and as preferably used herein, the term antibody includes any antigen or any target binding fragment of an antibody targeting or binding to such antigen or target. In other words, an antibody fragment of an antibody is a portion of said antibody Exemplary antibody fragments include, but are not limited to, F(ab')₂, Fab', Fab, Fv, sFv, the preparation of which is known to a person skilled in the art. An antibody fragment may also include single domain antibodies and IgG4 half-molecules, as discussed below. Regardless of structure, an antibody fragment binds with the same antigen that is recognized by the full-length antibody. The term "antibody fragment" also includes isolated fragments consisting of the variable regions of antibodies, such as the "Fv" fragments consisting of the variable regions of the heavy and light chains and recombinant single chain polypeptide molecules in which light and heavy variable regions are connected by a peptide linker ("scFv proteins").

In an embodiment and as preferably used herein, a human antibody is, e.g., an antibody that can be obtained from a human or from transgenic mice that have been "engineered" to produce specific human antibodies in response to antigenic challenge. In this technique, elements of the human heavy and light chain loci are introduced into strains of mice derived from embryonic stem cell lines that contain targeted disruptions of the endogenous murine heavy chain and light chain loci. The transgenic mice can synthesize human antibodies specific for particular antigens, and the mice can be used to produce human antibody-secreting hybridomas.

In an embodiment and as preferably used herein, a humanized antibody is a recombinant protein in which the CDRs from an antibody from one species; e.g., a rodent antibody, are transferred from the heavy and light variable chains of the rodent antibody into human heavy and light variable domains (e.g., framework region sequences). The constant domains of the antibody molecule are derived from those of a human antibody. In certain embodiments, a limited number of framework region amino acid residues from the parent (rodent) antibody may be substituted into the human antibody framework region sequences.

In an embodiment and as preferably used herein, a chimeric antibody is a recombinant protein that contains the variable domains including the complementarity determining regions (CDRs) of an antibody derived from one species, preferably a rodent antibody, while the constant domains of the antibody molecule are derived from those of a human antibody. For veterinary applications, the constant domains of the chimeric antibody may be derived from that of other species, such as a cat or dog.

It will be appreciated by a person skilled in the art that an antibody, including a therapeutically active antibody can be generated by routine methods against any target and antigen, respectively. Such methods are well known in the art. See, for example, Köhler and Milstein, Nature 256: 495 (1975), and Coligan et al. (eds.), CURRENT PROTOCOLS IN IMMUNOLOGY, VOL. 1, pages 2.5.1-2.6.7 (John Wiley & Sons 1991). Briefly, monoclonal antibodies can be obtained by injecting mice with a composition comprising an antigen, removing the spleen to obtain B-lymphocytes, fusing the B-lymphocytes with myeloma cells to produce hybridomas, cloning the hybridomas, selecting positive clones which produce antibodies to the antigen, culturing the clones that produce antibodies to the antigen, and isolating the antibodies from the hybridoma cultures.

MAbs can be isolated and purified from hybridoma cultures by a variety of well-established techniques. Such isolation techniques include affinity chromatography with Protein-A or Protein-G Sepharose, size-exclusion chromatography, and ion-exchange chromatography. See, for example, Coligan at pages 2.7.1-2.7.12 and pages 2.9.1-2.9.3. Also, see Baines et al., "Purification of Immunoglobulin G (IgG)," in METHODS IN MOLECULAR BIOLOGY, VOL. 10, pages 79-104 (The Humana Press, Inc. 1992).

After the initial raising of antibodies to the immunogen, the antibodies can be sequenced and subsequently prepared by recombinant techniques. Humanization and chimerization of murine antibodies and antibody fragments are well known to those skilled in the art, as discussed below.

A chimeric antibody is a recombinant protein in which the variable regions of a human antibody have been replaced by the variable regions of, for example, a mouse antibody, including the complementarity-determining regions (CDRs) of the mouse antibody. Chimeric antibodies exhibit decreased immunogenicity and increased stability when administered to a subject. General techniques for cloning murine immunoglobulin variable domains are disclosed, for example, in Orlandi et al., Proc. Nat'l Acad. Sci. USA 6: 3833 (1989). Techniques for constructing chimeric antibodies are well known to those of skill in the art. As an example, Leung et al., Hybridoma 13:469 (1994), produced an LL2 chimera by combining DNA sequences encoding the V_{κ} and V_{H} domains of murine LL2, an anti-CD22 monoclonal antibody, with respective human κ and IgG₁ constant region domains.

Techniques for producing humanized MAbs are well known in the art (see, e.g., Jones et al., Nature 321: 522 (1986), Riechmann et al., Nature 332: 323 (1988), Verhoeyen et al., Science 239: 1534 (1988), Carter et al., Proc. Nat'l Acad. Sci. USA 89: 4285 (1992), Sandhu, Crit. Rev. Biotech. 12: 437 (1992), and Singer et al., J. Immun. 150: 2844 (1993)). A chimeric or murine monoclonal antibody may be humanized by transferring the mouse CDRs from the heavy and light variable chains of the mouse immunoglobulin into the corresponding variable domains of a human antibody. The mouse framework regions (FR) in the chimeric monoclonal antibody are also replaced with human FR sequences. As simply transferring mouse CDRs into human FRs often results in a reduction or even loss of antibody affinity, additional modification might be required in order to restore the original affinity of the murine antibody. This can be accomplished by the replacement of one or more human residues in the FR regions with their murine counterparts to obtain an antibody that possesses good binding affinity to its epitope. See, for example, Tempest et al., Biotechnology 9:266 (1991) and Verhoeyen et al., Science 239: 1534 (1988). Preferred residues for substitution include FR residues that are located within 1, 2, or 3 Angstroms of a CDR residue side chain, that are located adjacent to a CDR sequence, or that are predicted to interact with a CDR residue.

Methods for producing fully human antibodies using either combinatorial approaches or transgenic animals transformed with human immunoglobulin loci are known in the art (*e*.*g*., Mancini et al., 2004, New Microbiol. 27:315-28; Conrad and Scheller, 2005, Comb. Chem. High Throughput Screen. 8:117-26; Brekke and Loset, 2003, Curr. Opin. Pharmacol. 3:544-50). A fully human antibody also can be constructed by genetic or chromosomal transfection methods, as well as phage display technology, all of which are known in the art. See for example, McCafferty et al., Nature 348:552-553 (1990). Where antibodies are to be utilized *in vivo,* for example in tumor therapy following detection of a Trop-2 positive cancer, such fully human antibodies are expected to exhibit even fewer side effects than chimeric or humanized antibodies and to function *in vivo* as essentially endogenous human antibodies.

In one alternative, the phage display technique may be used to generate human antibodies (*e*.*g*., Dantas-Barbosa et al., 2005, Genet. Mol. Res. 4:126-40). Human antibodies may be generated from normal humans or from humans that exhibit a particular disease state, such as cancer (Dantas-Barbosa et al., 2005). The advantage to constructing human antibodies from a diseased individual is that the circulating antibody repertoire may be biased towards antibodies against disease-associated antigens.

In one non-limiting example of this methodology, Dantas-Barbosa et al. (2005) constructed a phage display library of human Fab antibody fragments from osteosarcoma patients. Generally, total RNA was obtained from circulating blood lymphocytes (*Id.*). Recombinant Fab were cloned from the µ, γ and κ chain antibody repertoires and inserted into a phage display library (*Id*.). RNAs were converted to cDNAs and used to make Fab cDNA libraries using specific primers against the heavy and light chain immunoglobulin sequences (Marks et al., 1991, J. Mol. Biol. 222:581-97). Library construction was performed according to Andris-Widhopf et al. (2000, In: Phage Display Laboratory Manual, Barbas et al. (eds), 1st edition, Cold Spring Harbor Laboratory Press, Cold Spring Harbor, NY pp. 9.1 to 9.22). The final Fab fragments were digested with restriction endonucleases and inserted into the bacteriophage genome to make the phage display library. Such libraries may be screened by standard phage display methods, as known in the art. Phage display can be performed in a variety of formats, for their review, see e.g. Johnson and Chiswell, Current Opinion in Structural Biology 3:5564-571 (1993).

Human antibodies may also be generated by *in vitro* activated B-cells. See U.S. Patent Nos. 5,567,610 and 5,229,275. The skilled artisan will realize that these techniques are exemplary and any known method for making and screening human antibodies or antibody fragments may be utilized.

In another alternative, transgenic animals that have been genetically engineered to produce human antibodies may be used to generate antibodies against essentially any immunogenic target, using standard immunization protocols. Methods for obtaining human antibodies from transgenic mice are disclosed by Green et al., Nature Genet. 7:13 (1994), Lonberg et al., Nature 368:856 (1994), and Taylor et al., Int. Immun. 6:579 (1994). A non-limiting example of such a system is the XenoMouse^{®} (*e.g*., Green et al., 1999, J. Immunol. Methods 231:11-23) from Abgenix (Fremont, CA). In the XenoMouse^{®} and similar animals, the mouse antibody genes have been inactivated and replaced by functional human antibody genes, while the remainder of the mouse immune system remains intact.

The XenoMouse^{®} was transformed with germline-configured YACs (yeast artificial chromosomes) that contained portions of the human IgH and Igkappa loci, including the majority of the variable region sequences, along with accessory genes and regulatory sequences. The human variable region repertoire may be used to generate antibody producing B-cells, which may be processed into hybridomas by known techniques. A XenoMouse^{®} immunized with a target antigen will produce human antibodies by the normal immune response, which may be harvested and/or produced by standard techniques discussed above. A variety of strains of XenoMouse^{®} are available, each of which is capable of producing a different class of antibody. Transgenically produced human antibodies have been shown to have therapeutic potential, while retaining the pharmacokinetic properties of normal human antibodies (Green et al., 1999). The skilled artisan will realize that the claimed compositions and methods are not limited to use of the XenoMouse^{®} system but may utilize any transgenic animal that has been genetically engineered to produce human antibodies.

Antibody fragments are antigen binding portions of an antibody, such as F(ab') 2, Fab', F(ab)2, Fab, Fv, sFv, scFv and the like. Antibody fragments which recognize specific epitopes can be generated by known techniques. F(ab')2 fragments, for example, can be produced by pepsin digestion of the antibody molecule. These and other methods are described, for example, by Goldenberg, U.S. Pat. Nos. 4,036,945 and 4,331,647 and references contained therein. Also, see Nisonoff et al., Arch Biochem. Biophys. 89: 230 (1960); Porter, Biochem. J. 73: 119 (1959), Edelman et al., in METHODS IN ENZYMOLOGY VOL. 1, page 422 (Academic Press 1967), and Coligan at pages 2.8.1-2.8.10 and 2.10.-2.10.4. Alternatively, Fab' expression libraries can be constructed (Huse et al., 1989, Science, 246:1274-1281) to allow rapid and easy identification of monoclonal Fab' fragments with the desired specificity.

A single chain Fv molecule (scFv) comprises a VL domain and a VH domain. The VL and VH domains associate to form a target binding site. These two domains are further covalently linked by a peptide linker (L). A scFv molecule is denoted as either VL-L-VH if the VL domain is the N-terminal part of the scFv molecule, or as VH-L-VL if the VH domain is the N-terminal part of the scFv molecule. Methods for making scFv molecules and designing suitable peptide linkers are described in U.S. Pat. No. 4,704,692, U.S. Pat. No. 4,946,778, R. Raag and M. Whitlow, "Single Chain Fvs." FASEB Vol 9:73-80 (1995) and R. E. Bird and B. W. Walker, Single Chain Antibody Variable Regions, TIBTECH, Vol 9: 132-137 (1991).

Other antibody fragments, for example single domain antibody fragments, are known in the art and may be used in the claimed constructs. Single domain antibodies (VHH) may be obtained, for example, from camels, alpacas or llamas by standard immunization techniques. (See, e.g., Muyldermans et al., TIBS 26:230-235, 2001; Yau et al., J Immunol Methods 281:161-75, 2003; Maass et al., J Immunol Methods 324:13-25, 2007). The VHH may have potent antigen-binding capacity and can interact with novel epitopes that are inaccessible to conventional VH-VL pairs. (Muyldermans et al., 2001). Alpaca serum IgG contains about 50% camelid heavy chain only IgG antibodies (HCAbs) (Maass et al., 2007). Alpacas may be immunized with known antigens, such as TNF-α, and VHHs can be isolated that bind to and neutralize the target antigen (Maass et al., 2007). PCR primers that amplify virtually all alpaca VHH coding sequences have been identified and may be used to construct alpaca VHH phage display libraries, which can be used for antibody fragment isolation by standard biopanning techniques well known in the art (Maass et al., 2007).

An antibody fragment can also be prepared by proteolytic hydrolysis of a full-length antibody or by expression in E. coli or another host of the DNA coding for the fragment. An antibody fragment can be obtained by pepsin or papain digestion of full-length antibodies by conventional methods. For example, an antibody fragment can be produced by enzymatic cleavage of antibodies with pepsin to provide an approximate 100 kD fragment denoted F(ab')2. This fragment can be further cleaved using a thiol reducing agent, and optionally a blocking group for the sulfhydryl groups resulting from cleavage of disulfide linkages, to produce an approximate 50 Kd Fab' monovalent fragment. Alternatively, an enzymatic cleavage using papain produces two monovalent Fab fragments and an Fc fragment directly.

Other methods of cleaving antibodies, such as separation of heavy chains to form monovalent light-heavy chain fragments, further cleavage of fragments, or other enzymatic, chemical or genetic techniques may also be used, so long as the fragments bind to the antigen that is recognized by the intact antibody.

The various SEQ ID NOs:, the chemical nature of the nucleic aicd molecules according to the present invention and the target molecules CXCL12 as used herein, the actual sequence thereof and the internal reference number is summarized in the following table. It has to be noticed that the nucleic acids were characterized on the aptamer, i. e. D-nucleic acid level (D-RNA) with the biotinylated human D-CXCL12 (SEQ ID NO: 4) or on the Spiegelmer level, i. e. L-nucleic acid (L-RNA) with the natural configuration of CXCL12, the L- CXCL12 (human CXCL12α, SEQ ID NO: 1). The different nucleic acids share one internal reference name but one SEQ ID Nos: for the D-RNA (Aptamer) molecule and one SEQ ID Nos for the L-RNA (Spiegelmer) molecule, respectively. It will be appreciated by a person skilled in the art that the instant application contains further sequences and amino acid sequences in particular.

| **SEQ ID NO:** | ***RNA*/*Peptide*** | **Sequence** | ***Internal Reference*** |
|---|---|---|---|
| 1 | L-peptide | KPVSLSYRCPCRFFESHVARANVKHLKILNTPNCALQIVARLKNNNRQVCI DPKLKWIQEYLEKALNK | human/monkey/cat CXCL12α human/monkey/cat CXCL12 |
| 2 | L-peptide | KPVSLSYRCPCRFFESHVARANVKHLKILNTPNCALQIVARLKNNNRQVCI DPKLKWIQEYLEKALNKRFKM | human/monkey/cat CXCL12β |
| 3 | L-peptide | KPVSLSYRCPCRFFESHIARANVKHLKILNTPNCALQIVARLKNNNRQVCI DPKLKWIQEYLEKALNK | murine CXCL12α murine CXCL12 |
| 4 | D-peptide | KPVSLSYRCPCRFFESHVARANVKHLKILNTPNCALQIVARLKNNNRQVCI DPKLKWIQEYLEKALNKRFK-Biotin | biotinylated hu D-CXCL12 |
| 5 | L-RNA | AGCGUGGUGUGAUCUAGAUGUAGUGGCUGAUCCUAGUCAGGUACGCU | 193-C2-001 |
| 6 | L-RNA | AGCGUGGUGUGAUCUAGAUGUAUUGGCUGAUCCUAGUCAGGUACGCU | 193-G2-001 |
| 7 | L-RNA | AGCGUGGUGUGAUCUAGAUGUAAUGGCUGAUCCUAGUCAGGUGCGCU | 193-F2-001 |
| 8 | L-RNA | GCGAGGUGUGAUCUAGAUGUAGUGGCUGAUCCUAGUCAGGUGCGC | 193-G1-002 |
| 9 | L-RNA | GCGUGGUGUGAUCUAGAUGUAGUGGCUGAUCCUAGUCAGGUGCGC | 193-D2-002 |
| 10 | L-RNA | GCAUGGUGUGAUCUAGAUGUAGUGGCUGAUCCUAGUCAGGUGCCC | 193-A1-002 |
| 11 | L-RNA | GCGUGGUGUGAUCUAGAUGUAAUGGCUGAUCCUAGUCAGGGACGC | 193-D3-002 |
| 12 | L-RNA | GCGUGGUGUGAUCUAGAUGUAGAGGCUGAUCCUAGUCAGGUACGC | 193-B3-002 |
| 13 | L-RNA | GCGUGGUGUGAUCUAGAUGUAAAGGCUGAUCCUAGUCAGGUACGC | 193-H3-002 |
| 14 | L-RNA | GUGGUGUGAUCUAGAUGUAGUGGCUGUUCCUAGUCAGGUAUGC | 193-E3-002 |
| 15 | L-RNA | GCGUGGUGUGAUCUAGAUGUAGUGGCUGAUCCUAGUUAGGUACGC | 193-D1-002 |
| 16 | L-RNA | GCGUGGUGUGAUCUAGAUGUAGUGGCUGAUCCUAGUCAGGUACGC | 193-C2-002 |
| 17 | L-RNA | CGUGGUGUGAUCUAGAUGUAGUGGCUGAUCCUAGUCAGGUACG | 193-C2-003 |
| 18 | L-RNA | GUGGUGUGAUCUAGAUGUAGUGGCUGAUCCUAGUCAGGUAC | 193-C2-004 |
| 19 | L-RNA | UGGUGUGAUCUAGAUGUAGUGGCUGAUCCUAGUCAGGUA | 193-C2-005 |
| 20 | L-RNA | GGUGUGAUCUAGAUGUAGUGGCUGAUCCUAGUCAGGU | 193-C2-006 |
| 21 | L-RNA | GUGUGAUCUAGAUGUAGUGGCUGAUCCUAGUCAGG | 193-C2-007 |
| 22 | ***L-RNA*** | GCGUGGUGUGAUCUAGAUGUAUUGGCUGAUCCUAGUCAGGUACGC | 193-G2-012 |
| 23 | L-RNA | GCGCGGUGUGAUCUAGAUGUAUUGGCUGAUCCUAGUCAGGCGCGC | 193-G2-013 |
| 24 | L-RNA | GCGCGUGUGAUCUAGAUGUAUUGGCUGAUCCUAGUCAGGGCGC | 193-G2-014 |
| 25 | L-RNA | GGGCGUGUGAUCUAGAUGUAUUGGCUGAUCCUAGUCAGGGCCC | 193-G2-015 |
| 26 | L-RNA | GGCCGUGUGAUCUAGAUGUAUUGGCUGAUCCUAGUCAGGGGCC | 193-G2-016 |
| 27 | L-RNA | GCCCGUGUGAUCUAGAUGUAUUGGCUGAUCCUAGUCAGGGGGC | 193-G2-017 |
| 28 | L-RNA | 5′-40 kDa-PEG- GCGUGGUGUGAUCUAGAUGUAUUGGCUGAUCCUAGUCAGGUACGC | 193-G2-012-5'-PEG, NOX-A12 |
| 29 | D-RNA | AGCGUGGUGUGAUCUAGAUGUAGUGGCUGAUCCUAGUCAGGUACGCU | 193-C2-001 |
| 30 | D-RNA | AGCGUGGUGUGAUCUAGAUGUAUUGGCUGAUCCUAGUCAGGUACGCU | 193-G2-001 |
| 31 | D-RNA | AGCGUGGUGUGAUCUAGAUGUAAUGGCUGAUCCUAGUCAGGUGCGCU | 193-F2-001 |
| 32 | D-RNA | GCGAGGUGUGAUCUAGAUGUAGUGGCUGAUCCUAGUCAGGUGCGC | 193-G1-002 |
| 33 | D-RNA | GCGUGGUGUGAUCUAGAUGUAGUGGCUGAUCCUAGUCAGGUGCGC | 193-D2-002 |
| 34 | D-RNA | GCAUGGUGUGAUCUAGAUGUAGUGGCUGAUCCUAGUCAGGUGCCC | 193-A1-002 |
| 35 | D-RNA | GCGUGGUGUGAUCUAGAUGUAAUGGCUGAUCCUAGUCAGGGACGC | 193-D3-002 |
| 36 | D-RNA | GCGUGGUGUGAUCUAGAUGUAGAGGCUGAUCCUAGUCAGGUACGC | 193-B3-002 |
| 37 | D-RNA | GCGUGGUGUGAUCUAGAUGUAAAGGCUGAUCCUAGUCAGGUACGC | 193-H3-002 |
| 38 | D-RNA | GUGGUGUGAUCUAGAUGUAGUGGCUGUUCCUAGUCAGGUAUGC | 193-E3-002 |
| 39 | D-RNA | GCGUGGUGUGAUCUAGAUGUAGUGGCUGAUCCUAGUUAGGUACGC | 193-D1-002 |
| 40 | D-RNA | GCGUGGUGUGAUCUAGAUGUAGUGGCUGAUCCUAGUCAGGUACGC | 193-C2-002 |
| 41 | D-RNA | CGUGGUGUGAUCUAGAUGUAGUGGCUGAUCCUAGUCAGGUACG | 193-C2-003 |
| 42 | D-RNA | GUGGUGUGAUCUAGAUGUAGUGGCUGAUCCUAGUCAGGUAC | 193-C2-004 |
| 43 | D-RNA | UGGUGUGAUCUAGAUGUAGUGGCUGAUCCUAGUCAGGUA | 193-C2-005 |
| 44 | D-RNA | GGUGUGAUCUAGAUGUAGUGGCUGAUCCUAGUCAGGU | 193-C2-006 |
| 45 | D-RNA | GUGUGAUCUAGAUGUAGUGGCUGAUCCUAGUCAGG | 193-C2-007 |
| 46 | D-RNA | GCGUGGUGUGAUCUAGAUGUAUUGGCUGAUCCUAGUCAGGUACGC | 193-G2-012 |
| 47 | D-RNA | GCGCGGUGUGAUCUAGAUGUAUUGGCUGAUCCUAGUCAGGCGCGC | 193-G2-013 |
| 48 | D-RNA | GCGCGUGUGAUCUAGAUGUAUUGGCUGAUCCUAGUCAGGGCGC | 193-G2-014 |
| 49 | D-RNA | GGGCGUGUGAUCUAGAUGUAUUGGCUGAUCCUAGUCAGGGCCC | 193-G2-015 |
| 50 | D-RNA | GGCCGUGUGAUCUAGAUGUAUUGGCUGAUCCUAGUCAGGGGCC | 193-G2-016 |
| 51 | D-RNA | GCCCGUGUGAUCUAGAUGUAUUGGCUGAUCCUAGUCAGGGGGC | 193-G2-017 |
| 52 | L-RNA | GUGUGAUCUAGAUGUADWGGCUGWUCCUAGUYAGG | Type B Formula-1 |
| 53 | L-RNA | GUGUGAUCUAGAUGUADUGGCUGAUCCUAGUCAGG | Type B Formula-2 |
| 54 | L-RNA | AAAGUAACACGUAAAAUGAAAGGUAAC | |
| 55 | L-RNA | AAAGCAACAUGUCAAUGAAAGGUAGC | |
| 56 | L-RNA | GGUUAGGGCUAAAGUCGG | |
| 57 | L-RNA | GGUUAGGGCUAGAAGUCGG | |
| 58 | L-RNA | GGUUAGGGCUCGAAGUCGG | |
| 59 | L-RNA | GGUUAGGGCUUGAAGUCGG | |
| 60 | L-RNA | GCUGUGAAAGCAACAUGUCAAUGAAAGGUAGCCGCAGC | 192-A10-001 |
| 61 | L-RNA | GCUGUGAAAGUAACAUGUCAAUGAAAGGUAACCACAGC | 192-G10 |
| 62 | L-RNA | GCUGUGAAAGUAACACGUCAAUGAAAGGUAACCGCAGC | 192-F10 |
| 63 | L-RNA | GCUGUGAAAGUAACACGUCAAUGAAAGGUAACCACAGC | 192-B11 |
| 64 | L-RNA | GCUGUAAAAGUAACAUGUCAAUGAAAGGUAACUACAGC | 192-C9 |
| 65 | L-RNA | GCUGUAAAAGUAACAAGUCAAUGAAAGGUAACUACAGC | 192-E10 |
| 66 | L-RNA | GCUGUGAAAGUAACAAGUCAAUGAAAGGUAACCACAGC | 192-C10 |
| 67 | L-RNA | GCAGUGAAAGUAACAUGUCAAUGAAAGGUAACCACAGC | 192-D11 |
| 68 | L-RNA | GCUGUGAAAGUAACAUGUCAAUGAAAGGUAACCACUGC | 192-G11 |
| 69 | L-RNA | GCUAUGAAAGUAACAUGUCAAUGAAAGGUAACCAUAGC | 192-H11 |
| 70 | L-RNA | GCUGCGAAAGCGACAUGUCAAUGAAAGGUAGCCGCAGC | 192-D10 |
| 71 | L-RNA | GCUGUGAAAGCAACAUGUCAAUGAAAGGUAGCCACAGC | 192-E9 |
| 72 | L-RNA | GCUGUGAAAGUAACAUGUCAAUGAAAGGUAGCCGCAGC | 192-H9 |
| 73 | L-RNA | AGCGUGAAAGUAACACGUAAAAUGAAAGGUAACCACGCU | 191-A6 |
| 74 | L-RNA | AAAGYRACAHGUMAAX_{A}UGAAAGGUARC; X_{A} = A or absent | Type A Formula-1 |
| 75 | L-RNA | AAAGYRACAHGUMAAUGAAAGGUARC | Type A Formula-2 |
| 76 | L-RNA | AAAGYRACAHGUMAAAUGAAAGGUARC | Type A Formula-3 |
| 77 | L-RNA | AAAGYAACAHGUCAAUGAAAGGUARC | Type A Formula-4 |
| 78 | L-RNA | CUGUGAAAGCAACAUGUCAAUGAAAGGUAGCCGCAG | 192-A10-002 |
| 79 | L-RNA | UGUGAAAGCAACAUGUCAAUGAAAGGUAGCCGCA | 192-A10-003 |
| 80 | L-RNA | GUGAAAGCAACAUGUCAAUGAAAGGUAGCCGC | 192-A10-004 |
| 81 | L-RNA | UGAAAGCAACAUGUCAAUGAAAGGUAGCCG | 192-A10-005 |
| 82 | L-RNA | GAAAGCAACAUGUCAAUGAAAGGUAGCC | 192-A10-006 |
| 83 | L-RNA | AAAGCAACAUGUCAAUGAAAGGUAGC | 192-A10-007 |
| 84 | L-RNA | GCGUGAAAGCAACAUGUCAAUGAAAGGUAGCCGCGC | 192-A10-008 |
| 85 | L-RNA | GCGCGAAAGCAACAUGUCAAUGAAAGGUAGCCGCGC | 192-A10-015 |
| 86 | L-RNA | GCGGAAAGCAACAUGUCAAUGAAAGGUAGCCCGC | 192-A10-014 |
| 87 | L-RNA | CGUGAAAGCAACAUGUCAAUGAAAGGUAGCCGCG | 192-A10-016 |
| 88 | L-RNA | GCGCAAAGCAACAUGUCAAUGAAAGGUAGCGUGC | 192-A10-017 |
| 89 | L-RNA | GUGCAAAGCAACAUGUCAAUGAAAGGUAGCGCGC | 192-A10-018 |
| 90 | L-RNA | CGCGAAAGCAACAUGUCAAUGAAAGGUAGCCGUG | 192-A10-019 |
| 91 | L-RNA | GGGCAAAGCAACAUGUCAAUGAAAGGUAGCGCCC | 192-A10-020 |
| 92 | L-RNA | GGCCAAAGCAACAUGUCAAUGAAAGGUAGCGGCC | 192-A10-021 |
| 93 | L-RNA | GCCCAAAGCAACAUGUCAAUGAAAGGUAGCGGGC | 192-A10-022 |
| 94 | L-RNA | CCCCAAAGCAACAUGUCAAUGAAAGGUAGCGGGG | 192-A10-023 |
| 95 | L-RNA | GUGCUGCGGGGGUUAGGGCUAGAAGUCGGCCUGCAGCAC | 197-B2 |
| 96 | L-RNA | AGCGUGGCGAGGUUAGGGCUAGAAGUCGGUCGACACGCU | 191-D5-001 |
| 97 | L-RNA | GUGUUGCGGAGGUUAGGGCUAGAAGUCGGUCAGCAGCAC | 197-H1 |
| 98 | L-RNA | CGUGCGGCCUAAGAGGUUAGGGCUUAAAGUCGGUCUUUGGCCAACAC G | 190-D3 |
| 99 | L-RNA | CGUGCGCUUGAGAUAGGGGUUAGGGCUUAAAGUCGGCUGAUUCUCAC G | 190-A3-001 |
| 100 | L-RNA | CGUGAUUGGUGAGGGGUUAGGGCUUGAAGUCGGCCUUGUCCAGUCAC G | 190-A2 |
| 101 | L-RNA | AGCGUGAAGGGGUUAGGGCUCGAAGUCGGCUGACACGCU | 191-A5 |
| 102 | L-RNA | GUGCUGCGGGGGUUAGGGCUCGAAGUCGGCCCGCAGCAC | 197-H3 |
| 103 | L-RNA | GUGUUCCCGGGGUUAGGGCUUGAAGUCGGCCGGCAGCAC | 197-B1 |
| 104 | L-RNA | GUGUUGCAGGGGUUAGGGCUUGAAGUCGGCCUGCAGCAC | 197-E3 |
| 105 | L-RNA | GUGCUGCGGGGGUUAGGGCUCAAAGUCGGCCUGCAGCAC | 197-H2 |
| 106 | L-RNA | GUGCUGCCGGGGUUAGGGCUAA-AGUCGGCCGACAGCAC | 197-D1 |
| 107 | L-RNA | GUGCUGUGGGGGUCAGGGCUAGAAGUCGGCCUGCAGCAC | 197-D2 |
| 108 | L-RNA | GGUYAGGGCUHRX_{A}AGUCGG; X_{A} = A or absent | Type C Formula-1 |
| 109 | L-RNA | GGUYAGGGCUHRAAGUCGG | Type C Formula-2 |
| 110 | L-RNA | GGUYAGGGCUHRAGUCGG | Type C Formula-3 |
| 111 | L-RNA | GGUUAGGGCUHGAAGUCGG | Type C Formula-4 |
| 112 | L-RNA | UGAGAUAGGGGUUAGGGCUUAAAGUCGGCUGAUUCUCA | 190-A3-003 |
| 113 | L-RNA | GAGAUAGGGGUUAGGGCUUAAAGUCGGCUGAUUCUC | 190-A3-004 |
| 114 | L-RNA | GGGGUUAGGGCUUAAAGUCGGCUGAUUCU | 190-A3-007 |
| 115 | L-RNA | GCGUGGCGAGGUUAGGGCUAGAAGUCGGUCGACACGC | 191-D5-002 |
| 116 | L-RNA | CGUGGCGAGGUUAGGGCUAGAAGUCGGUCGACACG | 191-D5-003 |
| 117 | L-RNA | CGGGCGAGGUUAGGGCUAGAAGUCGGUCGACCG | 191-D5-004 |
| 118 | L-RNA | CGGGCGAGGUUAGGGCUAGAAGUCGGUCGCCCG | 191-D5-005 |
| 119 | L-RNA | CGGCGAGGUUAGGGCUAGAAGUCGGUCGCCG | 191-D5-006 |
| 120 | L-RNA | CGGGAGGUUAGGGCUAGAAGUCGGUCCCG | 191-D5-007 |
| 121 | L-RNA | GGGAGGUUAGGGCUAGAAGUCGGUCCC | 191-D5-010 |
| 122 | L-RNA | CCGCGGUUAGGGCUAGAAGUCGGGCGG | 191-D5-017 |
| 123 | L-RNA | CCCGGGUUAGGGCUAGAAGUCGGCGGG | 191-D5-029 |
| 124 | L-RNA | GGCGGGUUAGGGCUAGAAGUCGGCGCC | 191-D5-024 |
| 125 | L-RNA | CCCGCGGUUAGGGCUAGAAGUCGGGCGGG | 191-D5-017-29a |
| 126 | L-RNA | GCCGCGGUUAGGGCUAGAAGUCGGGCGGC | 191-D5-017-29b |
| 127 | L-RNA | CCCCGGGUUAGGGCUAGAAGUCGGCGGGG | 191-D5-019-29a |
| 128 | L-RNA | CGGCGGGUUAGGGCUAGAAGUCGGCGCCG | 191-D5-024-29a |
| 129 | L-RNA | GGGCGGGUUAGGGCUAGAAGUCGGCGCCC | 191-D5-024-29b |
| 130 | L-RNA | UGCUGCGGGGGUUAGGGCUAGAAGUCGGCCUGCAGCA | 197-B2-001 |
| 131 | L-RNA | GCUGCGGGGGUUAGGGCUAGAAGUCGGCCUGCAGC | 197-B2-002 |
| 132 | L-RNA | CUGCGGGGGUUAGGGCUAGAAGUCGGCCUGCAG | 197-B2-003 |
| 133 | L-RNA | UGCGGGGGUUAGGGCUAGAAGUCGGCCUGCA | 197-B2-004 |
| 134 | L-RNA | GCGGGGGUUAGGGCUAGAAGUCGGCCUGC | 197-B2-005 |
| 135 | L-RNA | GCCGGGGUUAGGGCUAGAAGUCGGCCGGC | 197-B2-006 |
| 136 | L-RNA | GGCCGGGGUUAGGGCUAGAAGUCGGCCGGCC | 197-B2-006-31a |
| 137 | L-RNA | CGCCGGGGUUAGGGCUAGAAGUCGGCCGGCG | 197-B2-006-31b |
| 138 | L-RNA | RKSBUSNVGR | Type C Formula-5-5' |
| 139 | L-RNA | YYNRCASSMY | Type C Formula-5-3' |
| 140 | L-RNA | RKSBUGSVGR | Type C Formula-6-5' |
| 141 | L-RNA | YCNRCASSMY | Type C Formula-6-3' |
| 142 | L-RNA | CGUGGUCCGUUGUGUCAGGUCUAUUCGCCCCGGUGCAGGGCAUCCGCG | 194-A2-001 |
| 143 | L-RNA | GCAGUGUGACGCGGACGUGAUAGGACAGAGCUGAUCCCGCUCAGGUGAG | 196-B12-003 |
| 144 | L-RNA | CAACAGCAGUGUGACGCGGACGUGAUAGGACAGAGCUGAUCCCGCUCAG | 196-B12-004 |
| 145 | L-RNA | 5'-40 kDa-PEG-GCGUGAAAGCAACAUGUCAAUGAAAGGUAGCCGCGC | 192-A10-008-5'-PEG |
| 146 | D-RNA | GCUGUGAAAGCAACAUGUCAAUGAAAGGUAGCCGCAGC | 192-A10-001 |
| 147 | D-RNA | GCUGUGAAAGUAACAUGUCAAUGAAAGGUAACCACAGC | 192-G10 |
| 148 | D-RNA | GCUGUGAAAGUAACACGUCAAUGAAAGGUAACCGCAGC | 192-F10 |
| 149 | D-RNA | GCUGUGAAAGUAACACGUCAAUGAAAGGUAACCACAGC | 192-B11 |
| 150 | D-RNA | GCUGUAAAAGUAACAUGUCAAUGAAAGGUAACUACAGC | 192-C9 |
| 151 | D-RNA | GCUGUAAAAGUAACAAGUCAAUGAAAGGUAACUACAGC | 192-E10 |
| 152 | D-RNA | GCUGUGAAAGUAACAAGUCAAUGAAAGGUAACCACAGC | 192-C10 |
| 153 | D-RNA | GCAGUGAAAGUAACAUGUCAAUGAAAGGUAACCACAGC | 192-D11 |
| 154 | D-RNA | GCUGUGAAAGUAACAUGUCAAUGAAAGGUAACCACUGC | 192-G11 |
| 155 | D-RNA | GCUAUGAAAGUAACAUGUCAAUGAAAGGUAACCAUAGC | 192-H11 |
| 156 | D-RNA | GCUGCGAAAGCGACAUGUCAAUGAAAGGUAGCCGCAGC | 192-D10 |
| 157 | D-RNA | GCUGUGAAAGCAACAUGUCAAUGAAAGGUAGCCACAGC | 192-E9 |
| 158 | D-RNA | GCUGUGAAAGUAACAUGUCAAUGAAAGGUAGCCGCAGC | 192-H9 |
| 159 | D-RNA | AGCGUGAAAGUAACACGUAAAAUGAAAGGUAACCACGCU | 191-A6 |
| 160 | D-RNA | CUGUGAAAGCAACAUGUCAAUGAAAGGUAGCCGCAG | 192-A10-002 |
| 161 | D-RNA | UGUGAAAGCAACAUGUCAAUGAAAGGUAGCCGCA | 192-A10-003 |
| 162 | D-RNA | GUGAAAGCAACAUGUCAAUGAAAGGUAGCCGC | 192-A10-004 |
| 163 | D-RNA | UGAAAGCAACAUGUCAAUGAAAGGUAGCCG | 192-A10-005 |
| 164 | D-RNA | GAAAGCAACAUGUCAAUGAAAGGUAGCC | 192-A10-006 |
| 165 | D-RNA | AAAGCAACAUGUCAAUGAAAGGUAGC | 192-A10-007 |
| 166 | D-RNA | GCGUGAAAGCAACAUGUCAAUGAAAGGUAGCCGCGC | 192-A10-008 |
| 167 | D-RNA | GCGCGAAAGCAACAUGUCAAUGAAAGGUAGCCGCGC | 192-A10-015 |
| 168 | D-RNA | GCGGAAAGCAACAUGUCAAUGAAAGGUAGCCCGC | 192-A10-014 |
| 169 | D-RNA | CGUGAAAGCAACAUGUCAAUGAAAGGUAGCCGCG | 192-A10-016 |
| 170 | D-RNA | GCGCAAAGCAACAUGUCAAUGAAAGGUAGCGUGC | 192-A10-017 |
| 171 | D-RNA | GUGCAAAGCAACAUGUCAAUGAAAGGUAGCGCGC | 192-A10-018 |
| 172 | D-RNA | CGCGAAAGCAACAUGUCAAUGAAAGGUAGCCGUG | 192-A10-019 |
| 173 | D-RNA | GGGCAAAGCAACAUGUCAAUGAAAGGUAGCGCCC | 192-A10-020 |
| 174 | D-RNA | GGCCAAAGCAACAUGUCAAUGAAAGGUAGCGGCC | 192-A10-021 |
| 175 | D-RNA | GCCCAAAGCAACAUGUCAAUGAAAGGUAGCGGGC | 192-A10-022 |
| 176 | D-RNA | CCCCAAAGCAACAUGUCAAUGAAAGGUAGCGGGG | 192-A10-023 |
| 177 | D-RNA | GUGCUGCGGGGGUUAGGGCUAGAAGUCGGCCUGCAGCAC | 197-B2 |
| 178 | D-RNA | AGCGUGGCGAGGUUAGGGCUAGAAGUCGGUCGACACGCU | 191-D5-001 |
| 179 | D-RNA | GUGUUGCGGAGGUUAGGGCUAGAAGUCGGUCAGCAGCAC | 197-H1 |
| 180 | D-RNA | CGUGCGGCCUAAGAGGUUAGGGCUUAAAGUCGGUCUUUGGCCAACAC G | 190-D3 |
| 181 | D-RNA | CGUGCGCUUGAGAUAGGGGUUAGGGCUUAAAGUCGGCUGAUUCUCAC G | 190-A3-001 |
| 182 | D-RNA | CGUGAUUGGUGAGGGGUUAGGGCUUGAAGUCGGCCUUGUCCAGUCAC G | 190-A2 |
| 183 | D-RNA | AGCGUGAAGGGGUUAGGGCUCGAAGUCGGCUGACACGCU | 191-A5 |
| 184 | D-RNA | GUGCUGCGGGGGUUAGGGCUCGAAGUCGGCCCGCAGCAC | 197-H3 |
| 185 | D-RNA | GUGUUCCCGGGGUUAGGGCUUGAAGUCGGCCGGCAGCAC | 197-B1 |
| 186 | D-RNA | GUGUUGCAGGGGUUAGGGCUUGAAGUCGGCCUGCAGCAC | 197-E3 |
| 187 | D-RNA | GUGCUGCGGGGGUUAGGGCUCAAAGUCGGCCUGCAGCAC | 197-H2 |
| 188 | D-RNA | GUGCUGCCGGGGUUAGGGCUAA-AGUCGGCCGACAGCAC | 197-D1 |
| 189 | D-RNA | GUGCUGUGGGGGUCAGGGCUAGAAGUCGGCCUGCAGCAC | 197-D2 |
| 190 | D-RNA | UGAGAUAGGGGUUAGGGCUUAAAGUCGGCUGAUUCUCA | 190-A3-003 |
| 191 | D-RNA | GAGAUAGGGGUUAGGGCUUAAAGUCGGCUGAUUCUC | 190-A3-004 |
| 192 | D-RNA | GGGGUUAGGGCUUAAAGUCGGCUGAUUCU | 190-A3-007 |
| 193 | D-RNA | GCGUGGCGAGGUUAGGGCUAGAAGUCGGUCGACACGC | 191-D5-002 |
| 194 | D-RNA | CGUGGCGAGGUUAGGGCUAGAAGUCGGUCGACACG | 191-D5-003 |
| 195 | D-RNA | CGGGCGAGGUUAGGGCUAGAAGUCGGUCGACCG | 191-D5-004 |
| 196 | D-RNA | CGGGCGAGGUUAGGGCUAGAAGUCGGUCGCCCG | 191-D5-005 |
| 197 | D-RNA | CGGCGAGGUUAGGGCUAGAAGUCGGUCGCCG | 191-D5-006 |
| 198 | D-RNA | CGGGAGGUUAGGGCUAGAAGUCGGUCCCG | 191-D5-007 |
| 199 | D-RNA | GGGAGGUUAGGGCUAGAAGUCGGUCCC | 191-D5-010 |
| 200 | D-RNA | CCGCGGUUAGGGCUAGAAGUCGGGCGG | 191-D5-017 |
| 201 | D-RNA | CCCGGGUUAGGGCUAGAAGUCGGCGGG | 191-D5-029 |
| 202 | D-RNA | GGCGGGUUAGGGCUAGAAGUCGGCGCC | 191-D5-024 |
| 203 | D-RNA | CCCGCGGUUAGGGCUAGAAGUCGGGCGGG | 191-D5-017-29a |
| 204 | D-RNA | GCCGCGGUUAGGGCUAGAAGUCGGGCGGC | 191-D5-017-29b |
| 205 | D-RNA | CCCCGGGUUAGGGCUAGAAGUCGGCGGGG | 191-D5-019-29a |
| 206 | D-RNA | CGGCGGGUUAGGGCUAGAAGUCGGCGCCG | 191-D5-024-29a |
| 207 | D-RNA | GGGCGGGUUAGGGCUAGAAGUCGGCGCCC | 191-D5-024-29b |
| 208 | D-RNA | UGCUGCGGGGGUUAGGGCUAGAAGUCGGCCUGCAGCA | 197-B2-001 |
| 209 | D-RNA | GCUGCGGGGGUUAGGGCUAGAAGUCGGCCUGCAGC | 197-B2-002 |
| 210 | D-RNA | CUGCGGGGGUUAGGGCUAGAAGUCGGCCUGCAG | 197-B2-003 |
| 211 | D-RNA | UGCGGGGGUUAGGGCUAGAAGUCGGCCUGCA | 197-B2-004 |
| 212 | D-RNA | GCGGGGGUUAGGGCUAGAAGUCGGCCUGC | 197-B2-005 |
| 213 | D-RNA | GCCGGGGUUAGGGCUAGAAGUCGGCCGGC | 197-B2-006 |
| 214 | D-RNA | GGCCGGGGUUAGGGCUAGAAGUCGGCCGGCC | 197-B2-006-31a |
| 215 | D-RNA | CGCCGGGGUUAGGGCUAGAAGUCGGCCGGCG | 197-B2-006-31b |
| 216 | D-RNA | CGUGGUCCGUUGUGUCAGGUCUAUUCGCCCCGGUGCAGGGCAUCCGCG | 194-A2-001 |
| 217 | D-RNA | GCAGUGUGACGCGGACGUGAUAGGACAGAGCUGAUCCCGCUCAGGUGAG | 196-B12-003 |
| 218 | D-RNA | CAACAGCAGUGUGACGCGGACGUGAUAGGACAGAGCUGAUCCCGCUCAG | 196-B12-004 |
| 219 | L-RNA | 5'-40 kDa-PEG-UAAGGAAACUCGGUCUGAUGCGGUAGCGCUGUGCAGAGCU | Control Spiegelmer |
| 220 | L-RNA | CGUGCGCUUGAGAUAGG | |
| 221 | L-RNA | CUGAUUCUCACG | |
| 222 | L-RNA | CUGAUUCUCA | |
| 223 | L-RNA | 5'-40 kDa-PEG-GCCGGGGUUAGGGCUAGAAGUCGGCCGGC | 197-B2-006-5'-PEG |
| 224 | L-RNA | 5'-40 kDa-PEG-CGGGAGGUUAGGGCUAGAAGUCGGUCCCG | 191-D5-007-5'PEG |
| 225 | L-RNA | 5′-40 kDa-PEG- CGCAUGGACUGAUCCUAGUCGGUUAUGUAGAUCUAGUGUGGUGCG | revNOX-A12 |

The invention will be further illustrated by means of and reference to the following Figs. and examples from which further advantages, features and embodiments may be taken, whereby
- Fig. 1: shows an alignment of sequences of CXCL12 binding nucleic acid molecules of "type A";
- Figs. 2A+B: show derivatives of CXCL12 binding nucleic acid molecule 192-A10-001 (CXCL12 binding nucleic acid molecules of "type A");
- Fig. 3: shows an alignment of sequences of CXCL12 binding nucleic acid molecules of "type B";
- Figs. 4A+B: show derivatives of CXCL12 binding nucleic acid molecules 193-C2-001 and 193-G2-001 (CXCL12 binding nucleic acid molecules of type B);
- Fig. 5: shows an alignment of sequences of CXCL12 binding nucleic acid molecules of "type C";
- Fig. 6: shows derivatives of CXCL12 binding nucleic acid molecule 190-A3-001 (CXCL12 binding nucleic acid molecules of "type C");
- Figs. 7A+B: show derivatives of CXCL12 binding nucleic acid moleculs 190-D5-001 (CXCL12 binding nucleic acid molecules of "type C");
- Fig. 8: shows derivatives of CXCL12 binding nucleic acid molecule 197-B2 (CXCL12 binding nucleic acid molecule of "type C");
- Fig. 9: shows further CXCL12 binding nucleic acid molecules molecules which are, in addition to other CXCL12 binding nucleic acid molecules, also referred to as CXCL12 binding nucleic acid molecules of "type D";

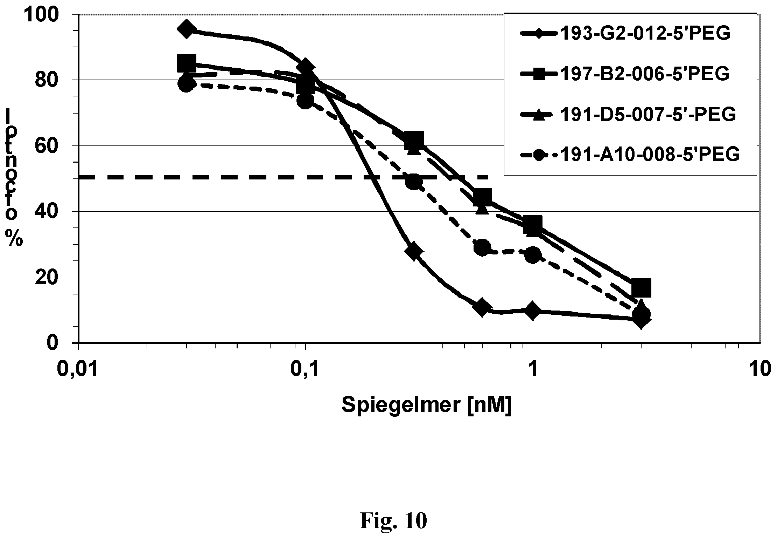

- Fig. 10: shows a diagram illustrating the efficacy of CXCL12 binding Spiegelmers 193-G2-012-5'-PEG (also referred to as NOX-A12), 197-B2-006-5'-PEG, 191-D5-007-5'-PEG and 191-A10-008-5'-PEG in a chemotaxis assay with the human T cell leukemia cell line Jurkat whereby cells were allowed to migrate towards 0.3 nM human CXCL12 preincubated at 37°C with various amounts of Spiegelmers 193-G2-012-5'-PEG, 197-B2-006-5'-PEG, 191-D5-007-5'-PEG and 191-A10-008-5'-PEG, represented as percentage of control over concentration of Spiegelmers 193-G2-012-5'-PEG, 197-B2-006-5'-PEG, 191-D5-007-5'-PEG and 191-A10-008-5'-PEG;
- Fig. 11A: shows a diagram illustrating the efficacy of CXCL12 binding Spiegelmer NOX-A12 in a chemotaxis assay with the human pre-B ALL cell line Nalm-6 whereby cells were allowed to migrate towards 0.3 nM human CXCL12 preincubated at 37°C with various amounts of Spiegelmer NOX-A12 represented as percentage of control over concentration of Spiegelmer NOX-A12;
- Fig. 11B: shows a diagram illustrating the efficacy of CXCL12 binding Spiegelmer NOX-A12 in a chemotaxis assay with the human leukemic monocyte lymphoma cell line U937 whereby cells were allowed to migrate towards 3 nM human CXCL12 preincubated at 37°C with various amounts of Spiegelmer NOX-A12 represented as percentage of control over concentration of Spiegelmer NOX-A12;
- Fig. 12: shows a diagram illustrating the efficacy of CXCL12 binding Spiegelmer NOX-A12 in a chemotaxis assay with the human pre-B cell leukemia cell line BV-173 whereby cells were allowed to migrate towards 3 nM human CXCL12 preincubated at 37°C with various amounts of Spiegelmer NOX-A12 represented as percentage of control over concentration of Spiegelmer NOX-A12;
- Fig. 13: shows a diagram illustrating the efficacy of CXCL12 binding Spiegelmer NOX-A12 in a complementation assay with CHO cells stably expressing CXCR7 and β-arrestin both fused to a fragment of β-galactosidase whereby CXCR7 of the cells were activated towards 10 nM human CXCL12 preincubated at 37°C with various amounts of Spiegelmer NOX-A12 represented as percentage of control over concentration of Spiegelmer NOX-A12;
- Fig. 14: is a diagram illustrating the treatment scheme underlying the clinical study using triple therapy as subject to Example 6;
- Fig. 15: is a diagram illustrating the treatment scheme underlying the clinical study using triple therapy further including chemotherapy as subject to Example 7;

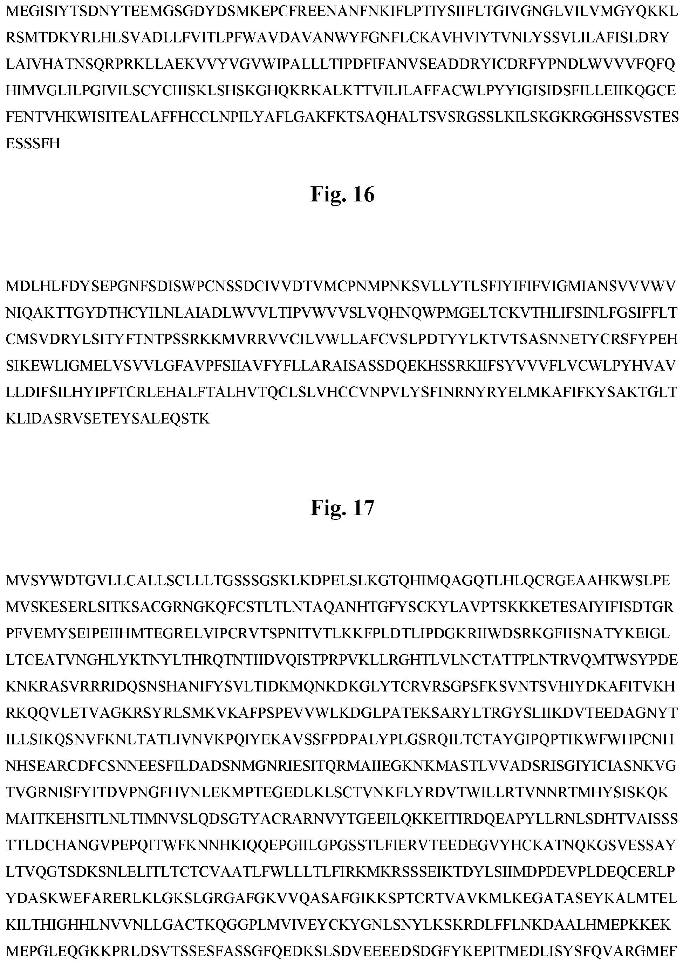

- Fig. 16: shows the amino acid sequence of human CXCR4;
- Fig. 17: shoes the amino acid sequence of human CXCR7;

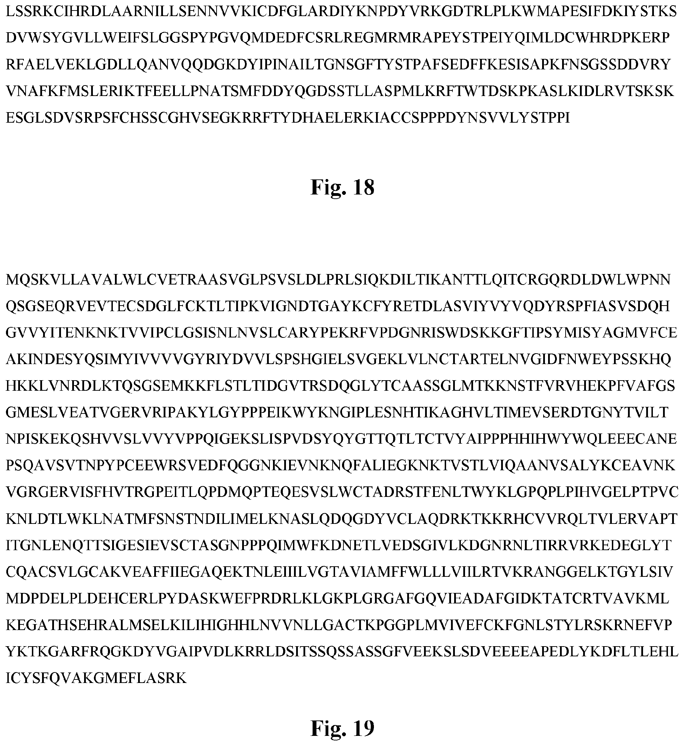

- Fig. 18: shows the amino acid sequence of human VEGFR1; and
- Fig. 19: shows the amino acid sequence of human VEGFR2.

### Example 1: Nucleic acids that bind human CXCL12

In the following the terms 'nucleic acid' and 'nucleic acid molecule' are used herein in a synonymous manner if not indicated to the contrary. Moreover, the terms 'stretch' and 'stretch of nucleotide' are used herein in a synonymous manner if not indicated to the contrary.

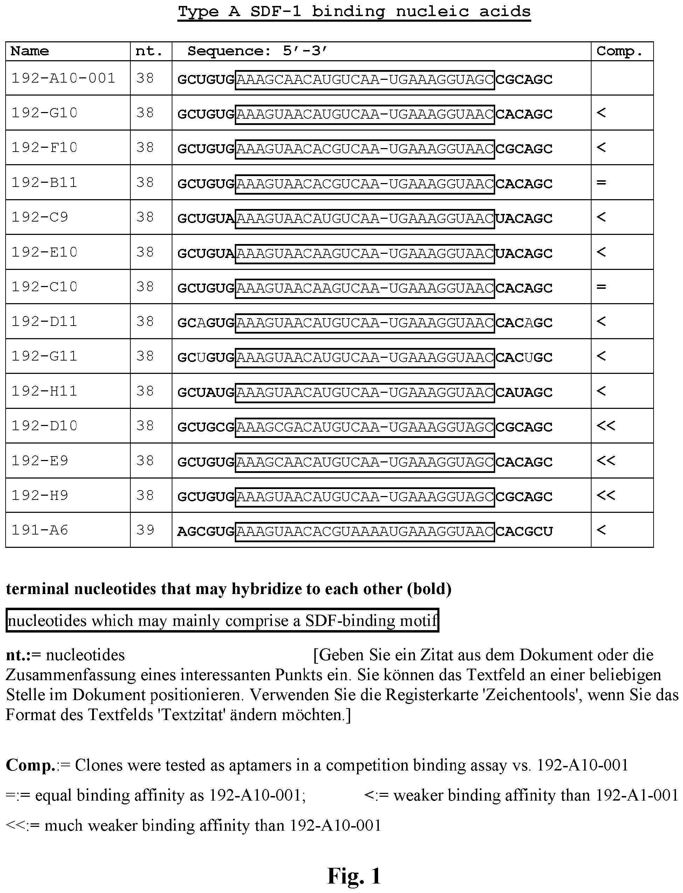

L-nucleic acid molecules that bind to human CXCL12 and the respective nucleotide sequences are depicted in Figures 1 to 9. The nucleic acids were characterized on the aptamer, i.e. D-nucleic acid level using competitive or direct pull-down binding assays with biotinylated human D-CXCL12 (protocol, see Example 3). Spiegelmers were tested with the natural configuration of CXCL12 (L-CXCL12) by surface plasmon resonance measurement using a Biacore 2000 instrument (protocol, see Example 5) and a cell culture *in vitro* chemotaxis assay (protocol, see Example 4).

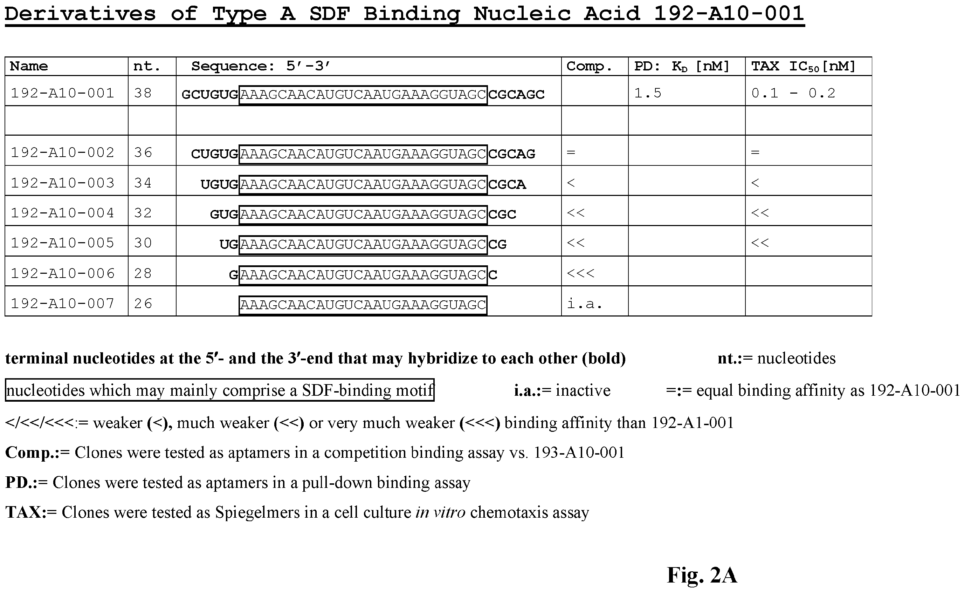

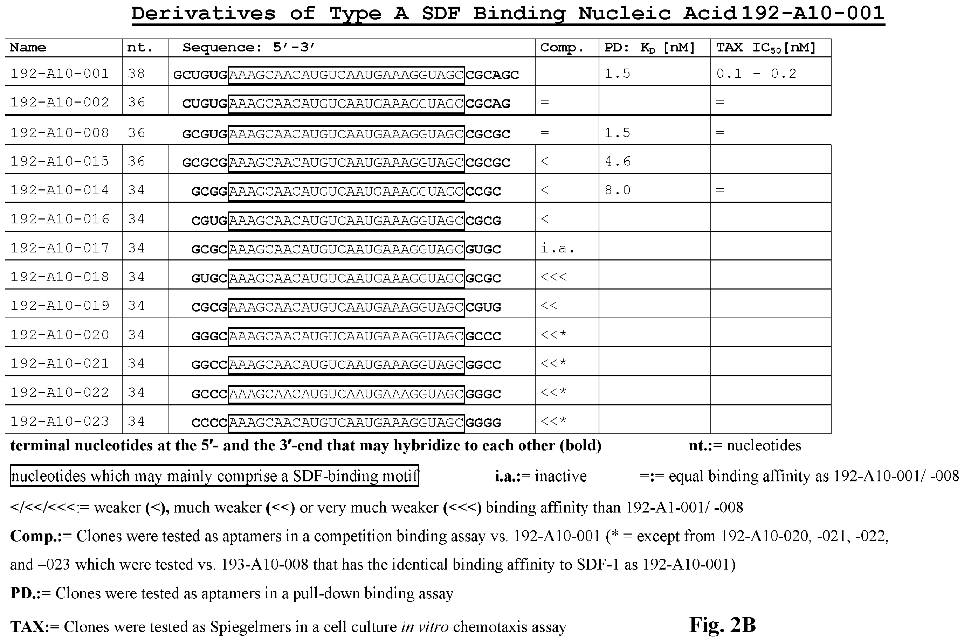

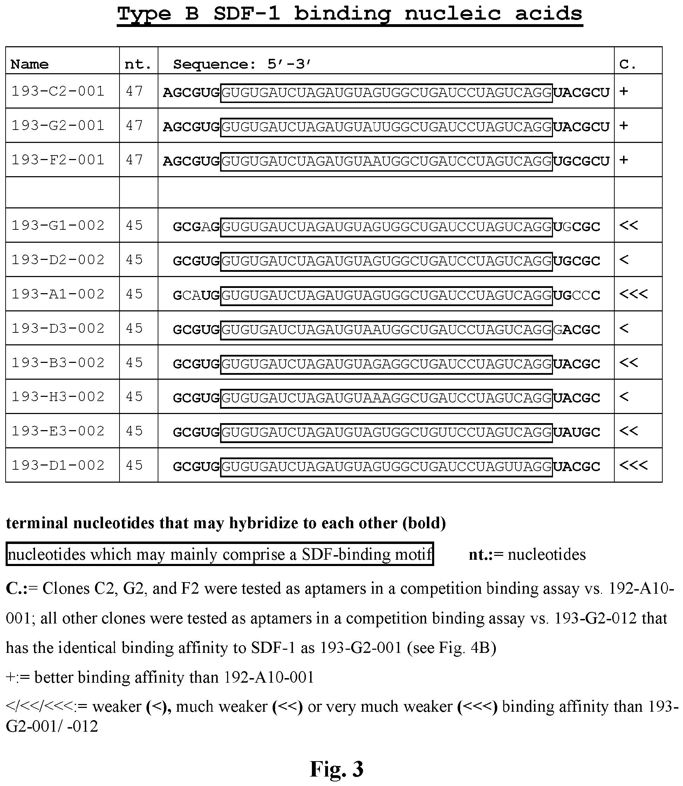

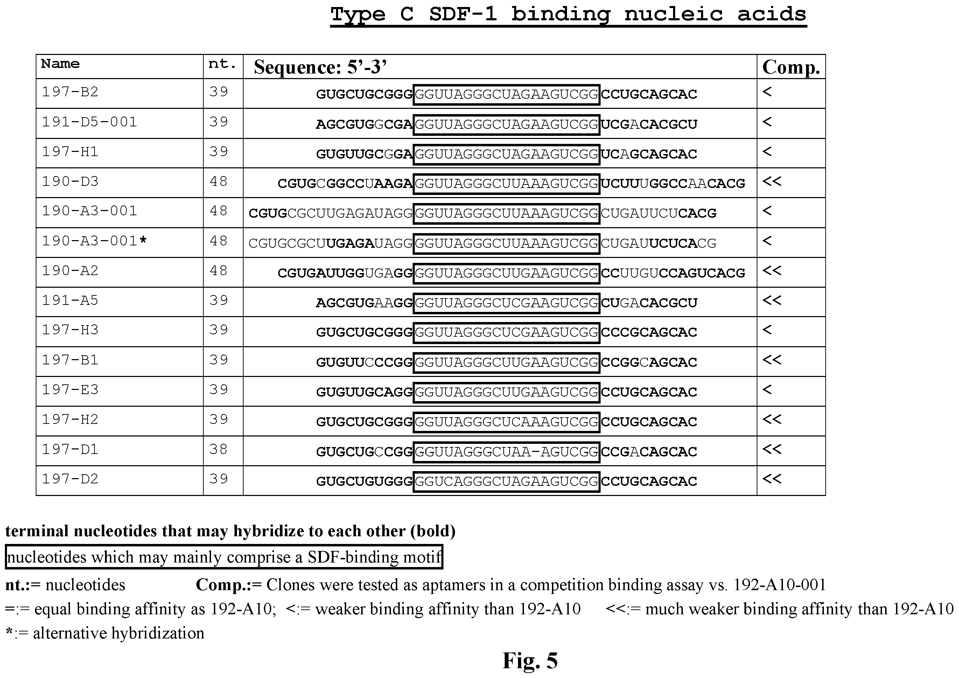

The CXCL12 binding nucleic acid molecules exhibit different sequence motifs, three main types are defined in Figs. 1, 2A and 2B (Type A), Figs. 3, 4A and 4B (Type B), Figs. 5, 4, 7A, 7B and 8 (Type C). The nucleic acid molecules exhibit different sequence motifs. For definition of nucleotide sequence motifs, the IUPAC abbreviations for ambiguous nucleotides is used:

| | | |
|---|---|---|
| S | strong | G or C; |
| W | weak | A or U; |
| R | purine | G or A; |
| Y | pyrimidine | C or U; |
| K | keto | G or U; |
| M | imino | A or C; |
| B | not A | C or U or G; |
| D | not C | A or G or U; |
| H | not G | A or C or U; |
| V | not U | A or C or G; |
| N | all | A or G or C or U |

If not indicated to the contrary, any nucleic acid sequence or sequence of stretches and boxes, respectively, is indicated in the 5' → 3' direction.

### CXCL12 binding nucleic acid molecules of type A

As depicted in Fig. 1 all sequences of CXCL12 binding nucleic acid moleculess of type A comprise one central stretch of nucleotides which is flanked by the first (5'-) terminal and the second (3'-) terminal stretch of nucleotides (also referred to as first terminal stretch of nucleotides and second stretch of nucleotides) whereby both stretches can hybridize to each other. However, such hybridization is not necessarily given in the molecule.

In the following the terms 'CXCL12 binding nucleic acid molecules of type A' and 'Type A CXCL12 binding nucleic acids' or Type A CXCL12 binding nucleic acid molecules'are used herein in a synonymous manner if not indicated to the contrary.

The sequences of the defined boxes or stretches of nucleotides may be different between the CXCL12 binding nucleic acids of type A which influences the binding affinity to CXCL12. Based on binding analysis of the different CXCL12 binding nucleic acids summarized as Type A CXCL12 binding nucleic acids, the central strectch of nucleotides and its nucleotide sequences as described in the following are individually and more preferably in their entirety essential for binding to CXCL12.

The central stretch of nucleotides of all identified sequences of Type A CXCL12 binding nucleic acids share the sequence AAAGYRACAHGUMAAX_{A}UGAAAGGUARC (Type A Formula-1, SEQ ID NO: 74), whereby X_{A} is either absent or is 'A'. If 'A' is absent, the sequence of the central nucleotide sequence can be summarized as Type A Formula-2 (AAAGYRACAHGUMAA-UGAAAGGUARC, SEQ ID NO: 75. Type A CXCL12 binding nucleic acid 191-A6 (central nucleotide sequence: AAAGUAACACGUAAAAUGAAAGGUAAC, SEQ ID NO: 54) carrying the additional nucleotide 'A' within the central nucleotide sequence and still binding to CXCL12 let conclude an alternative central nucleotide sequence (AAAGYRACAHGUMAAAUGAAAGGUARC, Type A Formula-3, SEQ ID NO: 76). Exemplarily for all the other nucleic acids of Type A CXCL12 binding nucleic acids, the Type A CXCL12 binding nucleic acid 192-A10-001 was characterized for its binding affinity to human CXCL12. The equilibrium binding constant K_{D} was determined using the pull-down binding assay (K_{D} = 1.5 nM) and by surface plasmon resonance measurement (K_{D} = 1.0 nM). The IC₅₀ (inhibitory concentration 50%) of 0.12 nM for 192-A10-001 was measured using a cell culture *in vitro* chemotaxis assay. Consequently, all Type A CXCL12 binding nucleic acids as depicted in Fig. 1 were analyzed in a competitive pull-down binding assay vs. 192-A10-001. The Type A CXCL12 binding nucleic acids 192-B11 and 192-C10 showed equal binding affinities as 192-A10-001 in these competition experiments. Weaker binding affinity was determined for Type A CXCL12 binding nucleic acids 192-G10, 192-F10, 192-C9, 192-E10, 192-D11, 192-G11, 192-H11 and 191-A6. The Type A CXCL12 binding nucleic acids 192-D10, 192-E9 and 192-H9 have much weaker binding affinity than 192-A10-001.

As mentioned above, the Type A CXCL12 binding nucleic acid 192-B11 and 192-C10 exhibit equal binding affinity to CXCL12 as 192-A10-001. However, they show slight differences in the nucleotide sequence of the central stretch of nucleotides. Therefore, the consensus sequence of the three molecules binding to CXCL12 with almost the same high affinity can be summarized by the nucleotide sequence AAAGYAACAHGUCAAUGAAAGGUARC (Type A Formula-4, SEQ ID NO: 77)) whereby the nucleotide sequence of the central stretch of nucleotides of 192-A10-001 (nucleotide sequence: AAAGCAACAUGUCAAUGAAAGGUAGC, SEQ ID NO: 84) represents the nucleotide sequence with the best binding affinity of Type A CXCL12 binding nucleic acids.

Five or six out of the six nucleotides of the 5'-terminal stretch (also referred to as first terminal stretch) of Type A CXCL12 binding nucleic acids may hybridize to the respective five or six nucleotides out of the six nucleotides of the 3'-terminal stretch (also referred to as second terminal stretch) to form a terminal helix. Although these nucleotides are variable at several positions, the different nucleotides allow for hybridization of five or six out of the six nucleotides of the 5'- and 3'-terminal stretches each. The 5'-terminal and 3'-terminal stretches of Type A CXCL12 binding nucleic acids as shown in Fig. 1 can be summarized in a generic formula for the 5'-terminal stretch ('RSHRYR', Type A Formula-5-5') and for the 3'-terminal stretch ('YRYDSY', Type A Formula-5-3'). Truncated derivatives of Type A CXCL12 binding nucleic acid 192-A10-001 were analyzed in a competitive pull-down binding assay vs. the original molecule 192-A10-001 and 192-A10-008 (Fig. 2A and 2B). These experiments showed that a reduction of the six terminal nucleotides (5'end: **GCUGUG**; 3'end: **CGCAGC**) of 192-A10-001 to five nucleotides (5'end: **CUGUG**; 3'end: **CGCAG**) of the derivative 192-A10-002 could be done without reduction of binding affinity. However, the truncation to four terminal nucleotides (5'end: **UGUG**; 3'end: **CGCA**; 192-A10-003) or less (192-A10-004/ -005/ -006/ -007) led to reduced binding affinity to CXCL12 (Fig. 2A). The determined 5'-terminal and 3'-terminal stretches with a length of five and four nucleotides of the derivatives of Type A CXCL12 binding nucleic acid 192-A10-001 as shown in Figs. 2A and 2B can be described in a generic formula for the 5'-terminal stretch ('X₂BBBS', Type A Formula-6-5') and of the 3'-terminal stretch ('SBBVX₃'; Type A Formula-6-3'), whereby X₂ is either absent or is 'S' and X₃ is either absent or is 'S'.

The nucleotide sequence of the 5'- and 3'-terminal stretches has an influence on the binding affinity of Type A CXCL12 binding nucleic acids. This is not only shown by the nucleic acids 192-F10 and 192-E10, but also by derivatives of 192-A10-001 (Fig. 2B). The central stretch of 192-F10 and 192-E10 are identical to 192-B11 and 192-C10, but comprise slight differences at the 3'-end of 5'-terminal stretch and at the 5'-end of 3'-terminal stretch resulting in reduced binding affinity.

The substitution of 5'- and 3'-terminal nucleotides **'CUGUG'** and **'CGCAG'** of Type A CXCL12 binding nucleic acid 192-A10-002 by **'GCGCG'** and **'CGCGC'** (192-A10-015) resulted in a reduced binding affinity whereas substitutions by **'GCGUG'** and **'CGCGC'** (192-A10-008) resulted in same binding affinity as shown for 192-A10-002 (Fig. 2B). Additionally, nine derivatives of Type A CXCL12 binding nucleic acid 192-A10-001 (192-A10-014/ -015/ -016/ -017/ -018/ -019/ -020/ -021/ -022/ -023) bearing four 5'- and 3'-terminal nucleotides respectively were tested as aptamers for their binding affinity vs. 192-A10-001 or its derivative 192-A10-008 (both have the identical binding affinity to CXCL12). All molecules showed weaker, much weaker or very much weaker binding affinity to CXCL12 as 192-A10-001 (six nucleotides forming a terminal helix) or as 192-A10-008 with five terminal nucleotides, respectively (Fig. 2B). Consequently, the sequence and the number of nucleotides of the 5'- and 3'-terminal stretches are essential for an effective binding to CXCL12. As shown for Type A CXCL12 binding nucleic acids 192-A10-002 and 192-A10-08 the preferred combination of 5'- and 3'-terminal stretches are 'CUGUG' and 'CGCAG' (5'- and 3'-terminal stretches of Type A CXCL12 binding nucleic acid 192-A10-002) and 'GCGUG' and 'CGCGC' (5'- and 3'-terminal stretches of Type A CXCL12 binding nucleic acid 192-A10-008).

However, combining the 5'-and 3'-terminal stretches of all tested Type A CXCL12 binding nucleic acids the generic formula for the 5'-terminal stretch of Type A CXCL12 binding nucleic acids is 'X₁X₂NNBV' (Type A Formula-7-5') and the generic formula for the 3'-terminal stretch of Type A CXCL12 binding nucleic acids is 'BNBNX₃X₄' (Type A Formula-7-3'), whereas
X₁ is 'R' or absent, X₂ is 'S', X₃ is 'S' and X₄ is 'Y' or absent;
   or
X₁ is absent, X₂ is 'S' or absent, X₃ is 'S' or absent and X₄ is absent.

In order to prolong the Spiegelmer's plasma residence time *in vivo,* Spiegelmers 192-A10-008 was covalently coupled to a 40 kDa polyethylene glycol (PEG) moiety at the 5'-end as described in chapter 2. The PEG-moiety has no influence on Spiegelmers potency to inhibit CXCL12 induced chemotaxis.

### CXCL12 binding nucleic acid molecules of type B

As depicted in Fig. 3 all sequences of CXCL12 binding nucleic acids of type B comprise one central stretch of nucleotides which is flanked by 5'- and 3'-terminal stretches (also referred to as first and second terminal stretch of nucleotides) that can hybridize to each other. However, such hybridization is not necessarily given in the molecule.

In the following the terms 'CXCL12 binding nucleic acid molecules of type B' and 'Type B CXCL12 binding nucleic acids' or Type B CXCL12 binding nucleic acid molecules are used herein in a synonymous manner if not indicated to the contrary.

The sequences of the defined boxes or stretches may be different between the CXCL12 binding nucleic acids which influences the binding affinity to CXCL12. Based on binding analysis of the different CXCL12 binding nucleic acids, the central stretch of nucleotides and its nucleotide sequences as described in the following are individually and more preferably in their entirety essential for binding to CXCL12.

The central stretch of nucleotides of all identified sequences of CXCL12 binding nucleic acids 193-C2-001, 193-G2-001, 193-F2-001, 193-G1-002, 193-D2-002, 193-A1-002, 193-D3-002, 193-B3-002, 193-H3-002, 193-E3-002 and 193-D1-002 share the sequence GUGUGAUCUAGAUGUADWGGCUGWUCCUAGUYAGG(Type B Formula-1, SEQ ID NO: 52).

The CXCL12 binding nucleic acids 193-G2-001, 193-C2-001 and 193-F2-001 that differ in one position of the central stretch of nucleotides (consenus sequence of central stretch of nucleotides: GUGUGAUCUAGAUGUADUGGCUGAUCCUAGUCAGG (Type B Formula-2, SEQ ID NO: 53) were analyzed in a competitive pull-down binding assay vs. the CXCL12 binding nucleic acid 192-A10-001 (K_{D} of 1.5 nM determined in a pull-down binding assay, IC₅₀ of 0.12 nM). Each of the CXCL12 binding nucleic acids 193-G2-001, 193-C2-001 and 193-F2 showed superior binding to human CXCL12 in comparison to CXCL12 binding nucleic acid 192-A10-001 whereby the binding affinity of 193-G2-001 is as good as 193-C2-001 and 193-F2-001 (Fig. 3). The data suggests that the difference in the nucleotide sequence of the central stretch of nucleotides of CXCL12 binding nucleic acids 193-G2-001, 193-C2-001 and 193-F2-001 has no influence on the binding affinity to CXCL12. The CXCL12 binding nucleic acids 193-G1-002, 193-D2-002, 193-A1-002, 193-D3-002, 193-B3-002, 193-H3-002, 193-E3-002 and 193-D1-002 showed reduced binding to human CXCL12 in comparison to CXCL12 binding nucleic acid 193-G2-001. CXCL12 binding nucleic acid 193-G2-001 was characterized for its binding affinity to human CXCL12. The equilibrium binding constant K_{D} was determined using the pull-down binding assay (K_{D} = 0.3 nM). The IC₅₀ (inhibitory concentration 50%) of 0.08 nM for 193-G2-001 was measured using a cell culture *in vitro* chemotaxis assay.

Four, five or six nucleotides out of the six nucleotides of the 5'-terminal stretch of CXCL12 binding nucleic acids may hybridize to the respective four, five or six out of the six nucleotides of the 3'-terminal stretch of CXCL12 binding nucleic acids to form a terminal helix. Although the nucleotides are variable at several positions, the different nucleotides allow the hybridization for four, five or six nucleotides out of the six nucleotides of the 5'- and 3'-terminal stretches each. The 5'-terminal and 3'-terminal stretches of CXCL12 binding nucleic acids as shown in Fig. 3 can be summarized in a generic formula for the 5'-terminal stretch ('X₁X₂GCRWG' whereas X₁ is 'A' or absent, X₂ is 'G') and of the 3'-terminal stretch ('KRYSCX₃X₄' whereas X₃ is 'G', X₄ is 'U' or absent). CXCL12 binding nucleic acids 193-G1-002, 193-D2-002, 193-A1-002 and 193-D3-002 have weaker binding affinities to CXCL12 although they share the identical central stretch of nucleotides with 193-C2-001, 193-G2-001 and 193-F2-001 (Fig. 3). The-unfavorable binding properties of CXCL12 binding nucleic acids 193-G1-002, 193-D2-002, 193-A1-002 and 193-D3-002 may be due to the number of nucleotides and sequence of the 5'- and 3'-terminal stretches.

Truncated derivatives of the CXCL12 binding nucleic acids 193-G2-001 and 193-C2-001 were analyzed in a competitive pull-down binding assay vs. 193-G2-001 and 193-G2-012, respectively (Fig. 4A and 4B). These experiments showed that a reduction of the six terminal nucleotides (5'end: **AGCGUG**; 3'end: **UACGCU**) of CXCL12 binding nucleic acids 193-G2-001 and 193-C2-001 to five nucleotides (5'end: **GCGUG**; 3'end: **UACGC**) lead to molecules with similar binding affinity (193-C2-002 and 193-G2-012). The equilibrium dissociation constant K_{D} was determined using the pull-down binding assay (K_{D} = 0.3 nM). A truncation to four (5'end: **CGUG**; 3'end: **UACG**; **193-C2-003**) or less nucleotides (193-C2-004, 193-C2-005, 193-C2-006, 193-C2-007) resulted in a reduced binding affinity to CXCL12 which was measured by using the competition pull-down binding assay (Fig. 4A). The nucleotide sequence of the five terminal nucleotides at the 5'- and 3'-end, respectively, has an influence on the binding affinity of CXCL12 binding nucleic acids. The substitution of 5'- and 3'-terminal nucleotides '**GCGUG**' and '**UACGC**' (193-C2-002, 193-G2-12) by '**GCGCG**' and '**CGCGC**' (193-G2-013) resulted in a reduced binding affinity. Additionally, the four different derivatives of CXCL12 binding nucleic acid 193-G2-001 with a terminal helix with a length of four base-pairing nucleotides (193-G2-014/ -015/ -016/ -017) were tested. All of them showed reduced binding affinity to CXCL12 (Fig. 4B). Therefore the sequence and the length of the 5'- and 3'-terminal nucleotides are essential for an effective binding to CXCL12. The 5'-terminal and 3'-terminal stretches with a length of five and four nucleotides of the derivatives of CXCL12 binding nucleic acids 193-C2-003 and 193-G2-012 as shown in Figs. 4A and 4B can be described in a generic formula for the 5'-terminal stretch ('X₁X₂SSBS'), whereby X₁ is absent, X₂ is either absent or is 'G', and of the 3'-terminal stretch ('BVSSX₃X₄'), and whereby X₃ is either absent or is 'C' and X₄ is absent. As shown for CXCL12 binding nucleic acids 193-G2-001 and 193-C2-01 and their derivatives 193-G2-012 and 193-C2-002 the preferred combination of 5'- and 3'-terminal stretches are 'X₁X₂GCGUG' (5'-terminal stretch) and 'UACGCX₃X₄' (3'-terminal stretch), whereas X₁ is either 'A' or absent, X₂ is 'G' and X₃ is 'C' and 'X₄ is 'U' or absent.

However, combining the 5'-and 3'-terminal stretches of all tested CXCL12 binding nucleic acids the generic formula for the 5'-terminal stretch of CXCL12 binding nucleic acids is 'X₁X₂SVNS' and the generic formula for the 3'-terminal stretch CXCL12 binding nucleic acids is 'BVBSX₃X₄', whereas
X₁ is 'A' or absent, X₂ is 'G', X₃ is 'C' and X₄ is 'U' or absent;
or X₁ is absent, X₂ is 'G' or absent, X₃ is 'C' or absent and X₄ is absent.

In order to prolong the Spiegelmer's plasma residence time *in vivo,* Spiegelmers 193-G2-012 was covalently coupled to a 40 kDa polyethylene glycol (PEG) moiety at the 5'-end as described in chapter 2 (PEGylated-nucleic acid molecule: 193-G2-012-5'-PEG also referred to as NOX-A12). The PEGylated Spiegelmer NOX-A12 was analyzed in cell culture in an *in vitro* chemotaxis-assay and an inhibition of CXCL12 induced chemotaxis was determined (IC₅₀ of 0.2 nM). The PEGylated Spiegelmer NOX-A12 was analyzed by Biacore measurement and a binding constant (K_{D}) of 0.2 nM was determined.

### CXCL12 binding nucleic acid molecules of type C

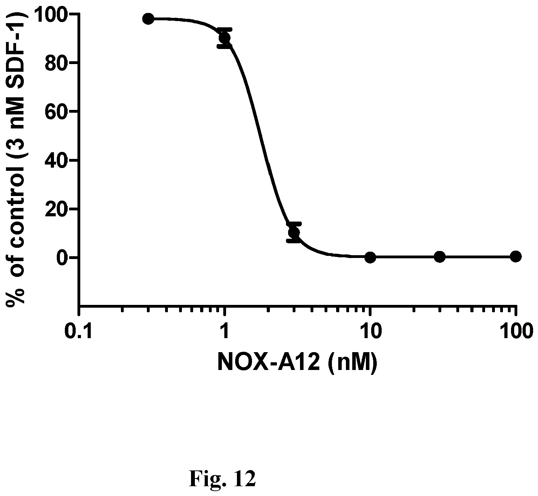

As depicted in Fig. 12 all sequences of CXCL12 binding nucleic acids of type C comprise one central stretch of nucleotides which is flanked by 5'- and 3'-terminal stretches (also referred to as first terminal stretch and second terminal stretch of nucleotides) that can hybridize to each other. However, such hybridization is not necessarily given in the molecule.

In the following the terms 'CXCL12 binding nucleic acid molecules of type C' and 'Type C CXCL12 binding nucleic acids' or Type C CXCL12 binding nucleic acid molecules'are used herein in a synonymous manner if not indicated to the contrary.

The sequences of the defined boxes or stretches may be different between the CXCL12 binding nucleic acids of Type C which influences the binding affinity to CXCL12. Based on binding analysis of the different CXCL12 binding nucleic acids summarized as Type C CXCL12 binding nucleic acids, the central stretch of nucleotides and its nucleotide sequence as described in the following are individually and more preferably in their entirety essential for binding to CXCL12.

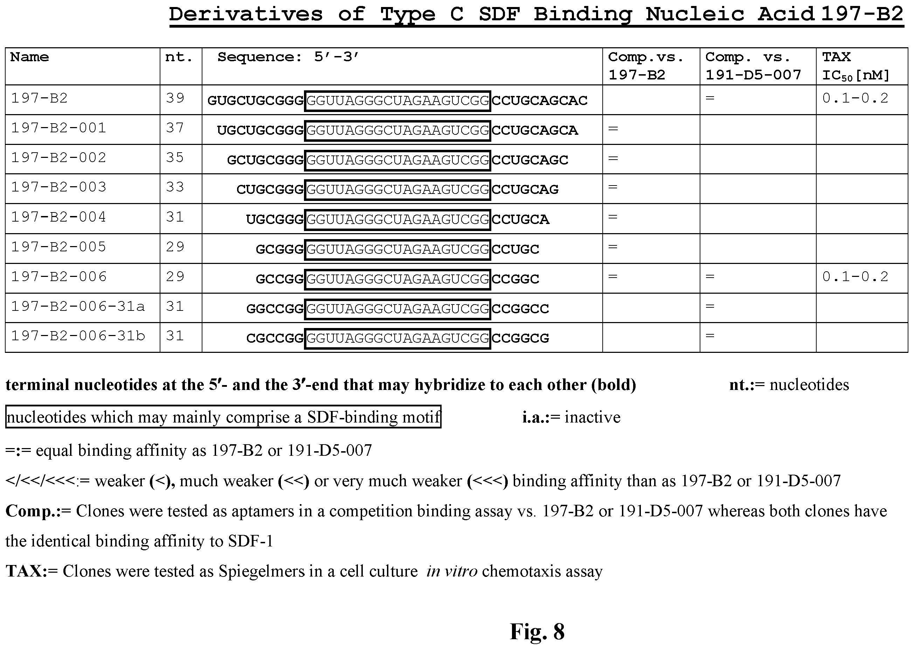

The central stretch of nucleotides of all identified sequences of Type C CXCL12 binding nucleic acids share the sequence GGUYAGGGCUHRX_{A}AGUCGG (Type C Formula-1, SEQ ID NO: 108), whereby X_{A} is either absent or is 'A'. With the exception of Type C CXCL12 binding nucleic acid 197-D1 the central stretch of nucleotides of all identified sequences of Type C CXCL12 binding nucleic acids share the nucleotide sequence GGUYAGGGCUHRAAGUCGG (Type C Formula-2, SEQ ID NO: 109). Type C CXCL12 binding nucleic acid 197-D1 (central stretch of nucleotides: GGUUAGGGCUAA-AGUCGG (SEQ ID NO: 56) missing one nucleotide 'A' within the central stretch of nucleotides and still binding to CXCL12 let conclude an alternative central stretch of nucleotides (GGUYAGGGCUHR-AGUCGG, Type C Formula-3, SEQ ID NO: 110). Initially, all Type C CXCL12 binding nucleic acids as depicted in Fig. 5 were analyzed in a competitive pull-down binding assay vs. Type A CXCL12 binding nucleic acid 192-A10-001 (K_{D} = 1.5 nM determined by pull-down assay and by surface plasmon resonance measurements; IC₅₀ = 0.12 nM). The Type C CXCL12 binding nucleic acids 191-D5-001, 197-B2, 190-A3-001, 197-H1, 197-H3 and 197-E3 showed weaker binding affinities than 192-A10-001 in competition experiments. Much weaker binding affinity was determined for 191-A5, 197-B1, 197-D1, 197-H2 and 197-D2 (Fig. 5). The molecules or derivatives thereof were further characterized by further competitive pull-down binding assays, plasmon resonance measurements and an *in vitro* chemotaxis assay. The Type C CXCL12 binding nucleic acid 191-D5-001 was characterized for its binding affinity to human CXCL12 whereas the equilibrium binding constant K_{D} was determined by surface plasmon resonance measurement (K_{D} = 0.8 nM). The IC₅₀ (inhibitory concentration 50%) of 0.2 nM for 191-D5-001 was measured using a cell-culture *in vitro* chemotaxis assay. The binding affinity of Type C CXCL12 binding nucleic acid 197-B2 for human CXCL12 was determined by surface plasmon resonance measurement (K_{D} = 0.9 nM), its IC₅₀ (inhibitory concentration 50%) of 0.2 nM was analyzed in a cell-culture *in vitro* chemotaxis assay. These data indicates that Type C CXCL12 binding nucleic acids 191-D5-001 and 197-B2 have the similar binding affinity to CXCL12 (Fig. 5 and 8).

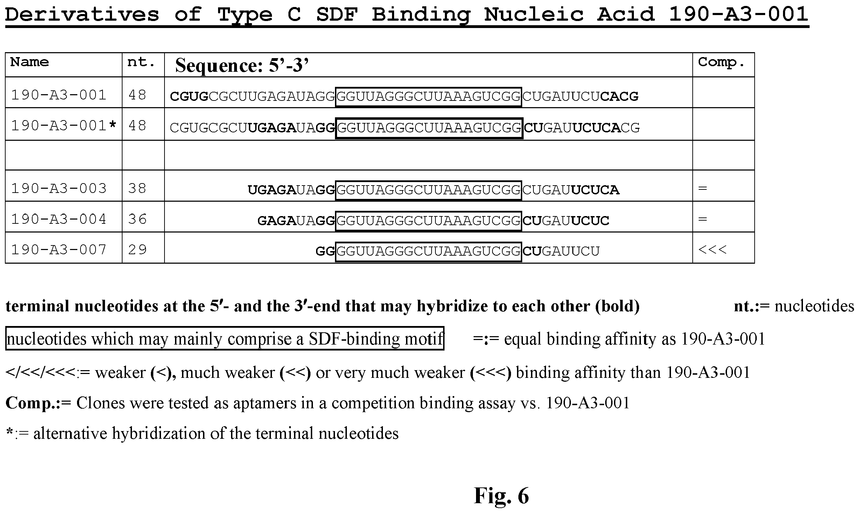

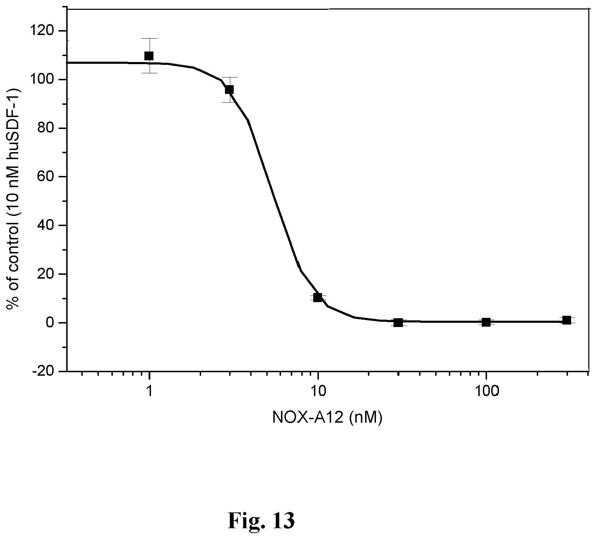

Type C CXCL12 binding nucleic acid 190-A3-001 comprises a 5'-terminal stretch of 17 nucleotides ('CGUGCGCUUGAGAUAGG', SEQ ID NO: 220) and a 3'-terminal stretch of 12 nucleotides ('CUGAUUCUCACG', SEQ ID NO: 221) whereby on the one hand the four nucleotides at the 5'-end of the 5'-terminal stretch and the four nucleotides at the 3'-end of the 3'-terminal stretch may hybridize to each other to form a terminal helix. Alternatively, the nucleotides '**UGAGA**' in the 5'-terminal stretch may hybridize to the nucleotides '**UCUCA**' in the 3'-terminal stretch to form a terminal helix. A reduction to nine nucleotides of the 5'-terminal stretch ('**UGAGAUAGG**') and to ten ('**CUGAUUCUCA**', SEQ ID NO: 222) nucleotides of the 3'-terminal stretch ('**CUGAUUCUC**') of molecule 190-A3-001 does not have an influence on the binding affinity to CXCL12 (190-A3-003; Fig. 13). A reduction to eight nucleotides of the 5'-terminal stretch ('**GAGAUAGG**') and to nine nucleotides of the 3'-terminal stretch (**'CUGAUUCUC'**) of molecule 190-A3-001 does not have an influence on the binding affinity to CXCL12 (190-A3-004; Fig. 6). The equilibrium binding constant K_{D} of 190-A3-004 was determined using the pull-down binding assay (K_{D} = 4.6 nM) and by surface plasmon resonance measurement (K_{D} = 4.7 nM). The IC₅₀ (inhibitory concentration 50%) of 0.1 nM for 190-A3-004 was measured using a cell-culture *in vitro* chemotaxis assay. However, the truncation to two nucleotides at the 5'-terminal stretch leads to a very strong reduction of binding affinity (190-A3-007; Fig. 6).

The Type C CXCL12 binding nucleic acids 191-D5-001, 197-B2 and 197-H1 (central stretch of nucleotides: GGUUAGGGCUAGAAGUCGG, SEQ ID 57, 197-H3/191-A5 (central stretch of nucleotides: GGUUAGGGCUCGAAGUCGG, SEQ ID NO: 58 and 197-E3/197-B1 (central stretch of nucleotides: GGUUAGGGCUUGAAGUCGG, SEQ ID NO: 59 share an almost identical central stretch of nucleotides (Type C formula-4; nucleotide sequence: GGUUAGGGCUHGAAGUCGG SEQ ID NO: 111). 191-D5-001, 197-B2 and 197-H1 do not share a similar 5'- and 3'-terminal stretch (197-H3 and 197-E3 have the identical 5'- and 3'-terminal stretch as 197-B2). However, the respective ten (197-B2, 197-E3, 197-H3) or nine out of the ten (191-D5-001, 197-H1) nucleotides of the 5'-terminal stretch may hybridize to the respective ten (197-B2, 197-E3, 197-H3) or nine out of the ten (191-D5-001, 197-H1) nucleotides of the 3'-terminal stretch (Fig. 5). Thus, the 5'-terminal stretch of Type C CXCL12 binding nucleic acids 197-B2, 191-D5-001, 197-H1, 197-E3 and 197-H3 as mentioned above plus 191-A5, 197-B1, 197-H2, 197-D1 and 197-D2 comprise a common generic nucleotide sequence of 'RKSBUSNVGR' (Type C Formula-5-5', SEQ ID NO: 138). The 3'-terminal stretch of Type C CXCL12 binding nucleic acids 197-B2, 191-D5-001, 197-H1, 197-E3, and 197-H3 as mentioned above plus 191-A5, 197-B1, 197-H2, 197-D1 and 197-D2 comprise a common generic nucleotide sequence of 'YYNRCASSMY' (Type C Formula-5-3', SEQ ID NO: 139), whereby the 5' and the 3'-terminal stretches of Type C CXCL12 binding nucleic acids 197-B2, 191-D5-001, 197-H1, 197-E3 and 197-H3 are preferred. These preferred 5'- and 3'-terminal stretches of Type C CXCL12 binding nucleic acids 197-B2, 191-D5-001, 197-H1, 197-E3 and 197-H3 can be summarized in the generic formula 'RKSBUGSVGR' (Type C Formula-6-5'; 5'-terminal stretch, SEQ ID NO: 140) and 'YCNRCASSMY' (Type C Formula-6-3'; 3'-terminal stretch, SEQ ID NO: 141).

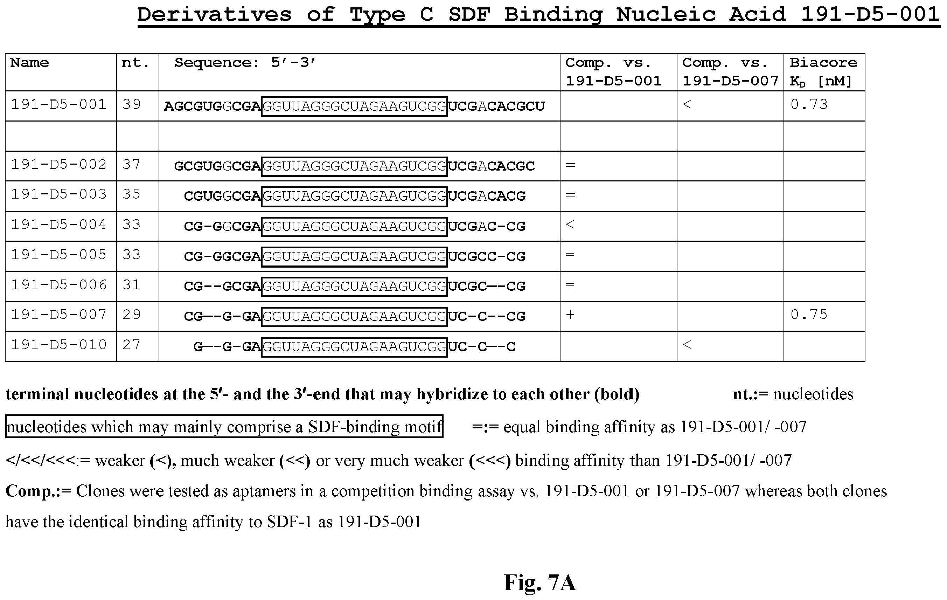

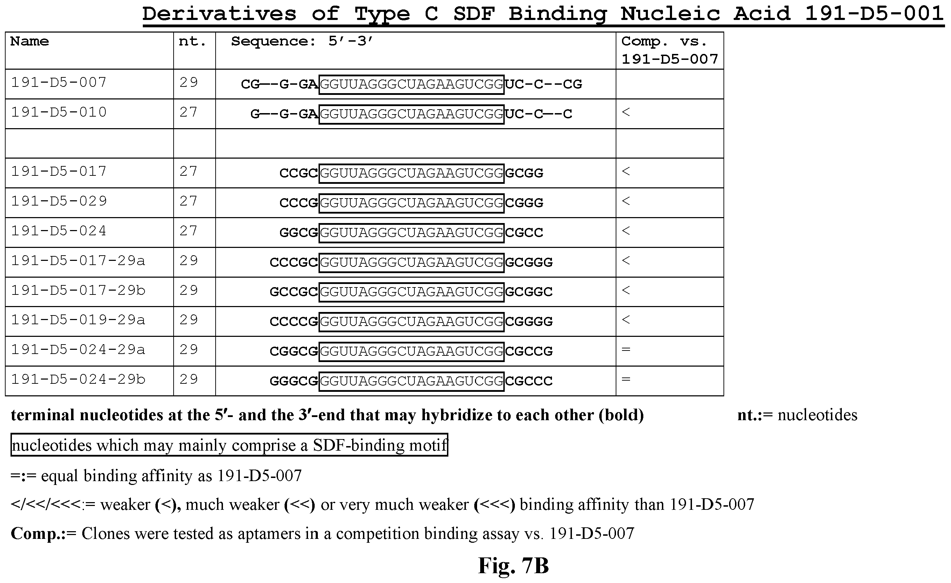

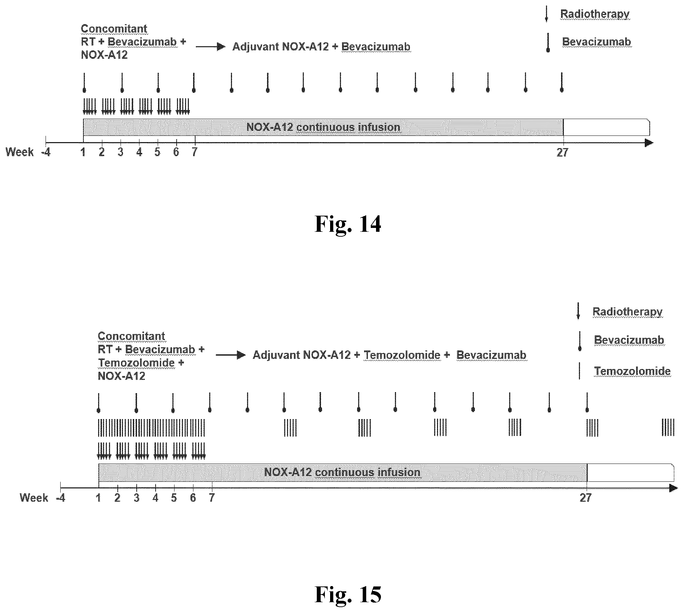

Truncated derivatives of Type C CXCL12 binding nucleic acid 191-D5-001 were constructed and tested in a competitive pull-down binding assay vs. the original molecule 191-D5-001 (Fig. 7A, Fig. 7B). At first the length of the 5'- and 3'-terminal stretches were shortened from ten nucleotides (191-D5-001) each to seven nucleotides each (191-D5-004) as depicted in Fig. 14A whereby nine out of the ten (191-D5-001) or six out of the seven nucleotides (191-D5-004) of the 5'-terminal stretch and of the 3'-terminal stretch, respectively can hybridize to each other. The reduction to seven nucleotides of the 5'-and 3'- terminal stretch respectively (whereas six out of the seven nucleotides can hybridize to each other) led to reduced binding affinity to CXCL12 (191-D5-004). The terminal stretches of Type C CXCL12 binding nucleic acid 191-D5-004 were modified whereby the non-pairing nucleotide 'A' within the 3'-terminal stretch of 191-D5-004 was substituted by a 'C' (191-D5-005). This modification led to an improvement of binding. This derivative, Type C CXCL12 binding nucleic acid 191-D5-005, showed similar binding to CXCL12 as 191-D5-001. Further truncation of the 5' - and 3' -terminal stretch to five nucleotides respectively led to a molecule with a length of total 29 nucleotides (191-D5-007). Because of the similarities of 191-D5-001 and of the Type C CXCL12 binding nucleic acids 197-B2, 191-D5-001, 197-H1, 191-A5, 197-H3, 197-B1, 197-E3, 197-D1, 197-H2 and 197-D2 and because of the data shown for 191-D5-007 it may assume that the 5'-and 3'-terminal stretch can in principle be truncated down to five nucleotides whereby the nucleotide sequence 'CGGGA' for 5'-terminal stretch and 'UCCCG' for the 3'-terminal stretch was successfully tested (Type C CXCL12 binding nucleic acid 191-D5-007). Type C CXCL12 binding nucleic acid 191-D5-007 surprisingly binds somewhat better to CXCL12 than 191-D5-001 (determined on aptamer level using the competition binding assay). The equilibrium binding constant K_{D} of 191-D5-007 was determined using the pull-down binding assay (K_{D} = 2.2 nM) and by surface plasmon resonance measurement (K_{D} = 0.8 nM). The IC₅₀ (inhibitory concentration 50%) of 0.1 nM for 191-D5-007 was measured using a cell-culture *in vitro* chemotaxis assay. Further truncation of both terminal stretches to four nucleotides (191-D5-010, Fig.7A).

Further derivatives of Type C CXCL12 binding nucleic acid 191-D5-001 (191-D5-017/ -024/ -029) bearing 5'- and 3'-terminal stretches of respectively four nucleotides also showed reduced binding affinity to CXCL12 in the competition pull-down binding assay vs. 191-D5-007 (Fig. 7B). Alternative 5'- and 3'-terminal stretches with a length of respectively five nucleotides were additionally tested, too (191-D5-017-29a, 191-D5-017-29b, 191-D5-019-29a, 191-D5-024-29a, 191-D5-024-29b). The generic formula of these derivatives for the 5'-terminal stretch is 'X_{S}SSSV' (Type C Formula-7-5') and for the 3'-stretch is `BSSSX_{S}' Type C Formula-7-3'), whereby X_{S} is absent or ,S'. Two out of the five tested variants showed identical binding affinity to CXCL12 as 191-D5-007 (191-D5-024-29a, 191-D5-024-29b; Fig. 7B). The sequences of the 5'-terminal and 3'-terminal stretches of 191-D5-001-derivatives that show the best binding affinity to CXCL12 and comprise a 5'-terminal and 3'-terminal stretch of five nucleotides respectively (191-D5-007, 191-D5-024-29a, 191-D5-024-29b) can be summarized in a generic formula (5'-terminal stretch: 'SGGSR', Type C Formula-8-5'; 3'-terminal stretch: , YSCCS', Type C Formula-8-3').

Truncated derivatives of Type C CXCL12 binding nucleic acid 197-B2 were analyzed in a competitive pull-down binding assay vs. the original molecule 197-B2 and 191-D5-007 (Fig. 7). Using the competitive pull-down binding assay vs. 191-D5-007 it was shown that 197-B2 has the same binding affinity to CXCL12 as 191-D5-007. The 5'- and 3'-terminal stretches were shortened without loss of binding affinity from ten nucleotides (197-B2) each to five nucleotides each (197-B2-005) whereby the nucleotides of the 5'-terminal stretch and of the 3'-terminal stretch can completely hybridize to each other. If the 5'-terminal **('GCGGG')** and 3'-terminal **('CCUGC')** stretch of 197-B2-005 was substituted by **'GCCGG'** (5'-terminal stretch) and by **'CCGGC'** (3'-terminal stretch) of 197-B2-006, the binding affinity to CXCL12 fully persisted. Because 197-B2 and 191-D5-001 (and their derivatives) share the identical core nucleotide sequence and several derivatives of 191-D5 with 5'- and 3'-terminal stretches with a length of respectively four nucleotides were tested, a further truncation of the 5'- and 3'-terminal stretch was omitted. Two further derivatives were designed that comprise six nucleotides at the 5'- and 3'-end (5'- and 3'-terminal stretches) respectively. The binding affinity to CXCL12 of both molecules (197-B2-006-31a and 197-B2-006-31b) is the same as shown for 191-D5-007 and 197-B2-006 (Fig. 15). The sequences of the 5'-terminal and 3'-terminal stretches of 197-B2 derivatives that show the best binding affinity to CXCL12 and comprise a 5'-terminal and 3'-terminal stretch of five nucleotides respectively can be summarized in a generic formula (5'-terminal stretch: 'GCSGG', Type C Formula-9-5'; 3'-terminal stretch: ,CCKGC', Type C Formula-9-3').

Combining the preferred 5'- and 3'-stretches of truncated derivatives of Type C CXCL12 binding nucleic acids 191-D5-001 (5'-terminal stretch: 'SGGSR', Type C Formula-8-5'; 3'-terminal stretch: ,YSCCS', Type C Formula-8-3') and 197-B2 (5'-terminal stretch: 'GCSGG', Type C Formula-9-5'; 3'-terminal stretch: ,CCKGC', Type C Formula-9-3') the common preferred generic formula for the 5'-terminal and the 3'-terminal stretch is 'SSSSR' (5'-terminal stretch, Type C Formula-10-5') and 'YSBSS' (3'-terminal stretch: Type C Formula-10-3').

In order to prolong the Spiegelmer's plasma residence time *in vivo,* Spiegelmers 197-B2-006 and 191-D5-007 were covalently coupled to a 40 kDa polyethylene glycol (PEG) moiety at their 5'-ends as described in chapter 2. The PEGylated Spiegelmers 197-B2-006 and 191-D5-007 were analyzed in cell culture in an *in vitro* chemotaxis. The PEG-moiety has no influence on Spiegelmers potency to inhibit CXCL12 induced chemotaxis.

### CXCL12 binding nucleic acid molecules of type D

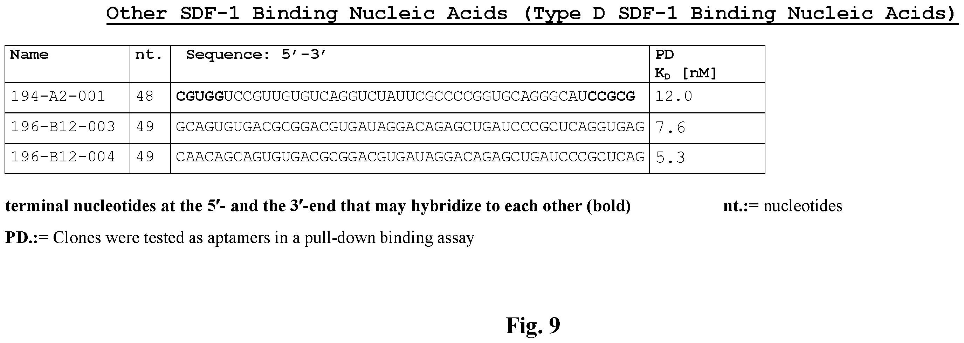

Additionally, further three CXCL12 binding nucleic acids that do not share the CXCL12 binding motifs of 'Type A', 'Type B' and 'Type C' were identified and are referred to herein as "type D". There were analyzed as aptamers using the pull-down binding assay (Fig. 9).

It is to be understood that any of the sequences shown in Figs. 1 through 9 are nucleic acid molecules according to the present invention, including those truncated forms thereof but also including those extended forms thereof under the proviso, however, that the thus truncated and extended, respectively, nucleic acid molecules are still capable of binding to the target.

### Example 2: Determination of binding constants (Pull-down binding assay)

### Direct pull-down binding assay

The affinity of aptamers to biotinlayted human D-CXCL12 was measured in a pull-down binding assay format at 37°C. Aptamers were 5'-phosphate labeled by T4 polynucleotide kinase (Invitrogen, Karlsruhe, Germany) using [γ-³²P]-labeled ATP (Hartmann Analytic, Braunschweig, Germany). The specific radioactivity of labeled aptamers was 200,000 - 800,000 cpm/pmol. Aptamers were incubated after de- and renaturation at 10, 20, 30 or 40 pM concentration at 37°C in selection buffer (20 mM Tris-HCl pH 7.4; 137 mM NaCl; 5 mM KCl; 1 mM MgCl₂; 1 mM CaCl₂; 0.1% [w/vol] Tween-20) together with varying amounts of biotinlayted human D-CXCL12 for 4 - 12 hours in order to reach equilibrium at low concentrations. Selection buffer was supplemented with 10 µg/ml human serum albumin (Sigma-Aldrich, Steinheim, Germany), and 10 µg/ml yeast RNA (Ambion, Austin, USA) in order to prevent adsorption of binding partners with surfaces of used plasticware or the immobilization matrix. The concentration range of biotinlayted human D-CXCL12 was set from 8 pM to 100 nM; total reaction volume was 1 ml. Peptide and peptide-aptamer complexes were immobilized on 1.5 µl Streptavidin Ultralink Plus particles (Pierce Biotechnology, Rockford, USA) which had been preequilibrated with selection buffer and resuspended in a total volume of 6 µl. Particles were kept in suspension for 30 min at the respective temperature in a thermomixer. Immobilized radioactivity was quantitated in a scintillation counter after detaching the supernatant and appropriate washing. The percentage of binding was plotted against the concentration of biotinlayted human D-CXCL12 and dissociation constants were obtained by using software algorithms (GRAFIT; Erithacus Software; Surrey U.K.) assuming a 1:1 stoichiometry.

### Competitive pull-down binding assay

In order to compare different D-CXCL12 binding aptamers, a competitive ranking assay was performed. For this purpose the most affine aptamer available was radioactively labeled (see above) and served as reference. After de- and renaturation it was incubated at 37°C with biotinlayted human D-CXCL12 in 1 ml selection buffer at conditions that resulted in around 5 - 10 % binding to the peptide after immobilization and washing on NeutrAvidin agarose or Streptavidin Ultralink Plus (both from Pierce) without competition. An excess of de- and renatured non-labeled D-RNA aptamer variants was added to different concentrations (e.g. 2, 10, and 50 nM) with the labeled reference aptamer to parallel binding reactions. The aptamers to be tested competed with the reference aptamer for target binding, thus decreasing the binding signal in dependence of their binding characteristics. The aptamer that was found most active in this assay could then serve as a new reference for comparative analysis of further aptamer variants.

### Example 3 Binding Analysis by Surface Plasmon Resonance Measurement

The Biacore 2000 instrument (Biacore AB, Uppsala, Sweden) was used to analyze binding of Spiegelmers to human CXCL12α. When coupling of human CXCL12α was to be achieved via amine groups, human CXCL12α was dialyzed against water for 1 - 2 h (Millipore VSWP mixed cellulose esters; pore size, 0.025 µM) to remove interfering amines. CM4 sensor chips (Biacore AB, Uppsala, Sweden) were activated before protein coupling by a 35-µl injection of a 1:1 dilution of 0.4 M NHS and 0.1 M EDC at a flow of 5 µl/min. Human MCP-1 or human CXCL12α was then injected in concentrations of 0.1 - 1.5 µg/ml at a flow of 2 µl/min until the instrument's response was in the range of 1000 - 2000 RU (relative units). Unreacted NHS esters were deactivated by injection of 35 µl ethanolamine hydrochloride solution (pH 8.5) at a flow of 5 µl/min. The sensor chip was primed twice with binding buffer and equilibrated at 10 µl/min for 1 - 2 hours until the baseline appeared stable. For all proteins, kinetic parameters and dissociation constants were evaluated by a series of Spiegelmer injections at concentrations of 1000, 500, 250, 125, 62.5, 31.25, and 0 nM in selection buffer (Tris-HCl, 20 mM; NaCl, 137 mM; KCl, 5 mM; CaCl₂, 1 mM; MgCl₂, 1 mM; Tween20, 0.1% [w/v]; pH 7.4). In all experiments, the analysis was performed at 37°C using the Kinject command defining an association time of 180 and a dissociation time of 360 seconds at a flow of 10 µl/min. Data analysis and calculation of dissociation constants (K_{D}) was done with the BIAevaluation 3.0 software (BIACORE AB, Uppsala, Sweden) using the Langmuir 1:1 stochiometric fitting algorithm.

### Example 4: Analysis of the inhibition of CXCL12-induced chemotaxis by CXCL12-binding Spiegelmers

The human T cell leukemia cell line Jurkat, the human leukemic monocyte lymphoma cell line U937, the human pre-B cell leukemia cell line BV-173 and human pre-B ALL cell line Nalm-6 express CXCR4. While Jurkat cells do not express CXCR7, the leukemia lines BV-173 and U-937 were tested positive for CXCR7 expression. All cells used were obtained from the DSMZ (Braunschweig). All cell lines were cultivated at 37°C and 5% CO2 in RPMI 1640 medium with Glutamax (Invitrogen, Karlsruhe, Germany) which contains 10% fetal bovine serum, 100 units/ml penicillin and 100 µg/ml streptomycin (Invitrogen, Karlsruhe, Germany). One day before the experiment, cells were seeded in a new T175 flask with a density of 0.3 × 10⁶/ml (Jurkat, U937, BV-173) or 0,75 × 10⁶/ml (Nalm-6), respectively.

For the experiment, cells were centrifuged (5min at 300g), resuspended, counted and washed once with 15 ml HBH (Hanks balanced salt solution containing 1 mg/ml bovine serum albumin and 20 mM HEPES; Invitrogen, Karlsruhe, Germany). Then the cells were resuspended at 1.33 × 10⁶/ml (Jurkat, U937, BV-173) or 2.67 × 10⁶/ml (Nalm-6), respectively. Cells were then allowed to migrate through the porous membranes of the filter plates for three hours towards a solution containing CXCL12 and various amounts of Spiegelmer. The stimulation solutions (CXCL12 + various concentrations of Spiegelmer) were made up as 10X solutions in a 0.2 ml low profile 96-tube plate. 212 µl HBH were pipetted into the lower compartments of the transport plate and 23,5 µl of the stimulation solutions were added. All conditions were made up as triplicates. After 20 to 30 min the filter plate was inserted into the plate containing the stimulation solutions and 75 µl of a cell suspension with 1.33 × 10⁶ /ml or 2.67 × 10⁶/ml, respectively, were added to the wells of the filter plate (1 × 10⁵ or 2 × 10⁵ cells/well). The cells were then allowed to migrate for 3 h at 37°C. For calibration, 0, 10 and 30 µl of the cell suspension was added to 235, 225 and 205 µl HBH, respectively, in wells of a separate 96-well plate. After 3 hours incubation, the insert plate was removed and 30 µl resazurin working solution (440 µM in PBS) were added to the lower wells and to the wells of the calibration plate. The plates were then incubated at 37°C for 2.5 h. After incubation, 100µl of each well were transferred to a black 96 well plate.

For evaluation, fluorescence values were corrected for background fluorescence (no cells in well). Then the difference between experimental conditions with and without CXCL12 was calculated. The value for the sample without Spiegelmer (CXCL12 only) was set 100% and the values for the samples with Spiegelmer were calculated as per cent of this. For a dose-response curve the per cent-values were plotted against Spiegelmer concentration and the IC₅₀-value (concentration of Spiegelmer at which 50% of the activity without Spiegelmer is present) was determined graphically from the resulting curve.

### Results

Human CXCL12 was found to stimulate migration of Jurkat cells in a dose dependent manner, with half-maximal stimulation at about 0.3 nM.

Human CXCL12 was found to stimulate migration of cells of the human leukemic monocyte lymphoma cell line U937 in a dose dependent manner, with half-maximal stimulation at about 3 nM.

Human CXCL12 was found to stimulate migration of cells of the human pre-B cell leukemia cell line BV-173 in a dose dependent manner, with half-maximal stimulation at about 3 nM.

Human CXCL12 was found to stimulate migration of cells of the human pre-B ALL cell line Nalm-6 in a dose dependent manner, with half-maximal stimulation at about 0.3 nM.

When cells were allowed to migrate towards a solution containing human CXCL12 plus increasing concentrations of CXCL12 binding Spiegelmers, dose-dependent inhibition was observed. The respective IC50s of the tested Spiegelmers as specified in Example 1 were determined in human T cell leukemia cell line Jurkat cells. For example, for CXCL12 binding Spiegelmer NOX-A12 (also referred to as 193-G2-012-5'-PEG) an IC50 of 0.2 nM was determined (Fig. 10). When an unspecific Control Spiegelmer was used instead of CXCL12 binding Spiegelmers, no inhibitory effect was observed up to 1 µM.

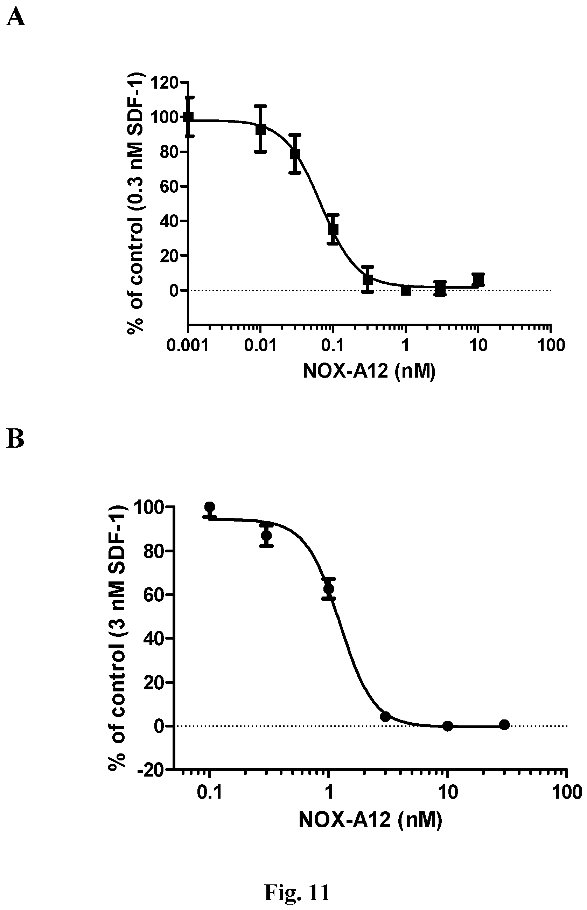

Inhibition of the CXCL12 induced chemotaxis by CXCL12 binding spiegelmer NOX-A12 was also observed in three other different leukemia cell types: the human leukemic monocyte lymphoma cell line U937 (Fig. 11B), the human pre-B cell leukemia cell line BV-173 (Fig. 12) and the human pre-B ALL cell line Nalm-6 (Fig. 11A). Furthermore, the inventors have evidence that primary chronic lymphocytic leukemia cells migrate towards CXCL12 and that CXCL12 dependent chemotaxis is effectively blocked by NOX-A12.

The leukemia lines BV-173 and U-937 were tested positive also for CXCR7 expression. The potency of SDF-binding spiegelmer NOX-A12 to block interaction of CXCL12 and CXCR7 was determined as shown in Example 6.

### Example 5: Inhibition of CXCR7 activation by CXCL12-binding Spiegelmer NOX-A12

Besides CXCR4, CXCL12 also binds to the chemokine receptor CXCR7. The inhibitory potential of CXCL12-binding Spiegelmer NOX-A12 towards CXCR7 was tested in a complementation assay with CHO cells stably expressing CXCR7 and β-arrestin both fused to a fragment of β-galactosidase (PathHunterTM - β-arrestin assay, DiscoveRX, CA, USA). Upon CXCL12 binding β-arrestin complexed with CXCR7 and thus led to complementation and activation of the β-galactosidase which was measured with a chemiluminescence substrate.

### Method

PathHunter eXpress CHO-K1 Human CXCR7 β-arrestin cells were plated for 48 hours in OCC2 Medium and stimulated with 10 nM CXCL12 and various concentrations of CXCL12-binding Spiegelmer NOX-A12 for 90 minutes. Following stimulation, signal was detected using the PathHUnter Detection Kit and the manufacturer's recommended protocol (DiscoveRX, CA, USA).

### Results

Stimulation of β-galactosidase and hence CXCR7 activation with 10 nM human CXCL12 was efficiently blocked by CXCL12-binding Spiegelmer NOX-A12 with an IC50 of 5.4 nM (Fig.13).

### Example 6: Clinical study using triple therapy

An arm in a clinical Phase 1/2 study was designed to assess safety of NOX-A12 plus bevacizumab when administered concomitantly to radiation therapy in glioblastoma patients and to explore the benefit of these combinations on progression-free survival when administered in addition to irradiation and to provide an estimate of the effect on overall survival.

The study was expected to advance the understanding of the important role that CXCL12 plays in neovascularization by recruiting endothelial and other bone marrow-derived pro-angiogenic cells through a CXCR4- and CXCR7-dependent mechanism.

The title of the respective study within which the NOX-A12 plus bevacizumab arm is performed is "A single-arm, dose-escalation Phase 1/2 Study of olaptesed pegol (NOX-A12) in combination with irradiation in inoperable or partially resected first-line glioblastoma patients with unmethylated MGMT promoter with a 3-arm expansion group including fully resected patients and combination with bevacizumab or pembrolizumab."

Study details are as follow.

**Patient Population:** Patients with newly diagnosed glioblastoma (WHO grade IV) with unmethylated MGMT promoter status after incomplete or no tumor resection

### Type of study: Multi-center, prospective, open-label

### Study objectives:

**Primary:** To investigate the safety of either olaptesed pegol in combination with radiation therapy or olaptesed pegol combination with radiation therapy and bevacizumab or pembrolizumab in patients with newly diagnosed glioblastoma of unmethylated MGMT promoter status
**Secondary:** (i) to investigate the efficacy of olaptesed pegol in combination with radiation therapy and bevacizumab in patients with glioblastoma of unmethylated MGMT promoter status either not amenable to resection (biopsy only) or after incomplete tumor resection; (ii) to investigate the pharmacokinetics of olaptesed pegol during continuous administration; (iii) to monitor symptoms (NANO) and QoL

### Study endpoints:

**Primary:** Safety (adverse events)
**Secondary:** PFS at 6 months (PFS-6); Median progression-free survival (mPFS); Median overall survival (mOS); Tumor vascularization as per vascular MRI scans at baseline and 2, 4, and 6 months; Topography of recurrence; Determination of maximum tolerated dose (MTD); Definition of recommended Phase 2 dose (RP2D); NOX-A12 plasma levels at steady state; NANO assessment; Quality of Life

### Criteria for inclusion:

1. Written informed consent
2. Age ≥ 18 years
3. Patient agreement to diagnostic and scientific work-up of glioblastoma tissue obtained during the preceding surgery or biopsy (e.g., MGMT promoter analysis, cytogenetic markers such as IDH-1 mutations, etc.)
4. Patient agrees to subcutaneous port implantation
5. Newly diagnosed, histologically confirmed, supratentorial WHO grade IV glioblastoma
6. Status post biopsy or incomplete resection (detectable residual tumor as per postoperative T1-weighted, contrast-enhanced MRI scan) (Arm A)
7. Unmethylated MGMT promoter status
8. Maximum Eastern Cooperative Oncology Group (ECOG) score 2
9. Estimated minimum life expectancy 3 months
10. Stable or decreasing dose of corticosteroids during the week prior to inclusion
11. The following laboratory parameters should be within the ranges specified:
   a. Total bilirubin ≤ 1.5 × upper limit normal (ULN)
   b. Creatinine ≤ 1.5 × ULN or glomerular filtration rate ≥ 60 mL/min/1.73m²
   c. ALT (alanine transaminase) ≤ 3 × ULN
   d. AST (aspartate transaminase) ≤ 3 × ULN
12. Female patients of child-bearing potential must have a negative serum pregnancy test within 21 days prior to enrollment and agree to use a highly effective method of birth control (failure rate less than 1% per year when used consistently and correctly such as contraceptive implants, vaginal rings, sterilization, or sexual abstinence) during and for 6 months following last dose of drug (more frequent pregnancy tests may be conducted if required per local regulations)
13. Male patients must use an effective barrier method of contraception during study and for 6 months following the last dose if sexually active with a FCBP

### Criteria for exclusion:

1. Inability to understand and collaborate throughout the study or inability or unwillingness to comply with study requirements
2. Participation in any clinical research study with administration of an investigational drug or therapy within 30 days from screening visit or observation period of competing studies
3. Contra-indication or known hypersensitivity to MRI contrast agents, bevacizumab, olaptesed pegol or polyethylene glycol
4. Planned hypofractionated radiotherapy
5. Cytostatic therapy (chemotherapy) within the past 5 years
6. History of other cancers (except for adequately treated basal or squamous cell skin cancer, in situ cervical cancer, or other cancer from which the patient was disease-free for ≥ 5 years)
7. Secondary malignancy which is currently active
8. Clinically significant or uncontrolled cardiovascular disease, including
   a. Myocardial infarction in the previous 12 months
   b. Uncontrolled angina
   c. Congestive heart failure (New York Heart Association functional classification of ≥2)
   d. Diagnosed or suspected congenital long QT syndrome
   e. QTc prolongation on an electrocardiogram prior to entry (>470 ms)
   f. Uncontrolled hypertension (blood pressure ≥ 160/95 mmHg)
   g. Heart rate <50/min on the baseline electrocardiogram
   h. History of ventricular arrhythmias of any clinically significant type (such as ventricular tachycardia, ventricular fibrillation or torsades de pointes)
   i. Cerebrovascular accident
9. Prior radiotherapy to the head
10. Any other previous or concomitant experimental glioblastoma treatments
11. Placement of Gliadel^{®} wafer, seeds, or ferromagnetic nanoparticles
12. Patients with a history of arterial or venous thrombosis (or any other disease) requiring permanent intake of anticoagulants
13. Pregnancy or lactation
14. Uncontrolled intercurrent illness including, but not limited to ongoing or active infection, chronic liver disease (e.g., cirrhosis, hepatitis), diabetes mellitus, or subjects with either of the following: fasting blood glucose (FBG defined as fasting for at least 8 hours) ≥ 200 mg/dL (7.0 mmol/L), or HbAlc ≥ 8%, chronic renal disease, pancreatitis, chronic pulmonary disease, autoimmune diseases or psychiatric illness/social situations that would limit compliance with study requirements. Patients must be free of any clinically relevant disease (other than glioma) that would, in the treating investigator's opinion, interfere with the conduct of the study or study evaluations.
15. Prolongation of coagulation factors ≥ 2.5 × ULN
16. Treatment not initiated within 6 weeks after first biopsy or surgery of glioblastoma
17. Prior enrolment into this study

### Treatment in the arm to combine NOX-A12 with radiotherapy and bevacizumab:

| | Weeks 1-6 | Weeks 7-26 |
|---|---|---|
| NOX-A12 | 600 mg/week continuous infusion | 600 mg/week continuous infusion |
| Irradiation | 2 Gy Mo-Fr | - |
| Bevacizumab | 10 mg/kg every 2 weeks | 10 mg/kg every 2 weeks |

The treatment regimen is also shown in Fig. 14.

### Results obtained so far

Currently, all of the 6 planned patients to be treated with NOX-A12, radiotherapy and bevacizumab have been included, and all are under treatment with NOX-A12 and bevacizumab. MRI assessments of tumor response has already been performed for up to week 27 for one patient, up to week 18 for two patients and up to week 9 for five patients. Reduction of T1 enhancing signal (SPDP, sum of the product of diameters of tumor lesions) of more than 50% was observed for all patients where data are available, thus all patients exhibit partial response, see table below.

| **Patient #** | **Baseline SPDP Sum of lesions** | **MRI week 9 SPDP** | **MRI Week 18 SPDP** | **MRI Week 27 SPDP** |
|---|---|---|---|---|
| 1 | 247.5 mm² | 132.2 mm² | 57.6 mm² | 43.3 mm² |
| | | -50.2% CFB | -76.7% CFB | -82.5% CFB |
| | | | -53.2% CFN | -24.8% CFN |
| | | **pPR** | **cPR** | **cPR** |
| 2 | 375.2 mm² | 148.6 mm² | 169.7 mm² | n.a. |
| | | -60.4% CFB | -54.8% CFB | |
| | | | +14.2% CFN | |
| | | **pPR** | **cPR** | |
| 3 | 247.8 mm² | 86.8 mm² | n.a. | n.a. |
| | | -65.0% CFB | | |
| | | **pPR** | | |
| 4 | 1495.8 mm² | 599.6 mm² | n.a. | n.a. |
| | | -59.9% CFB | | |
| | | **pPR** | | |
| 5 | 168.5 mm² | 9.0 mm² | n.a. | n.a. |
| | | -94.7% CFB | | |
| | | **pPR** | | |

| | | | | |
|---|---|---|---|---|
| CFB = change from baseline; CFN = change from nadir; pPR = preliminary partial response; cPR = confirmed partial response; SPDP = sum of the product of diameters of tumor lesions | | | | |

The treatment regimen is shown in Fig. 14.

### Example 7: Clinical study using triple therapy further including chemotherapy

A similar clinical study in glioblastoma patients is performed as described in Example 6, but in this study in addition the patients receive the chemotherapeutic temozolomide which is standard of care for glioblastoma. The title of the study is "A single-arm Phase 1/2 Study of olaptesed pegol (NOX-A12) in combination with irradiation, bevacizumab and temozolomide in first-line glioblastoma patients with methylated and unmethylated MGMT promoter".

The study assesses safety of NOX-A12 plus bevacizumab and temozolomide when administered concomitantly to radiation therapy in glioblastoma patients and to explore the benefit of these combinations on progression-free survival when administered in addition to irradiation and to provide an estimate of the effect on overall survival.

Differences between this study and the study described in Example 6 are:
**Patient Population:** Patients with newly diagnosed glioblastoma (WHO grade IV) with methylated or unmethylated MGMT promoter status and all degrees of tumor resection.
**Treatment:** In this study, NOX-A12 is combined with radiotherapy, bevacizumab and temozolomide.

| | Weeks 1-6 | Weeks 7-26 | Weeks 11-31 |
|---|---|---|---|
| NOX-A12 | 600 mg/week continuous infusion | 600 mg/week continuous infusion | |
| Irradiation | 2 Gy Mo-Fr | | |
| Bevacizumab | 10 mg/kg every 2 weeks | 10 mg/kg every 2 weeks | |
| Temozolomide | 75 mg/m² daily | | 5 × daily 150-200 mg/m² on D1-5 of six 28-day cycles |

According to the non-overlapping modes of action of either NOX-A12, bevacizumab or radiotherapy with temozolomide, there is no reason to expect a relevantly different outcome for this study in comparison to the study in Example 6. However, as also patients with MGMT methylated promotor status are included in this study that are sensitive to temozolomide, some additional benefit is seen for this group of patients.

The treatment regimen is shown in Fig. 15.

### References

The complete bibliographic data of the documents recited herein are, if not indicated to the contrary, as follows, whereby the disclosure of said references is incorporated herein by reference.

Altschul S.F., Gish W., et al. (1990) Basic local alignment search tool. J Mol Biol. 215(3):403-10.

Altschul S.F., Madden T.L., et al. (1997) Gapped BLAST and PSI-BLAST: a new generation of protein database search programs. Nucleic Acids Res. 25(17):3389-402.

Balabanian K., Lagane B., et al. (2005) The chemokine SDF-1/CXCL12 binds to and signals through the orphan receptor RDC1 in T lymphocytes. J Biol Chem 280(42): 35760-35766

Balabanian, K., Lagane B., et al. (2005) WHIM syndromes with different genetic anomalies are accounted for by impaired CXCR4 desensitization to CXCL12. Blood 105(6): 2449-57.

Balkwill F. (2004) Cancer and the chemokine network. Nat Rev Cancer 4(7): 540-50.

Brooks H.L. Jr., Caballero S. Jr., et al. (2004) Vitreous levels of vascular endothelial growth factor and stromal-derived factor 1 in patients with diabetic retinopathy and cystoid macular edema before and after intraocular injection of triamcinolone. Arch Ophthalmol 122(12): 1801-7.

Buckley C.D., Amft N., et al. (2000) Persistent induction of the chemokine receptor CXCR4 by TGF-beta 1 on synovial T cells contributes to their accumulation within the rheumatoid synovium. J Immunol 165(6): 3423-9.

Burger J.A., Tsukada N., et al. (2000) Blood-derived nurse-like cells protect chronic lymphocytic leukemia B cells from spontaneous apoptosis through stromal cell-derived factor-1. Blood. 96(8):2655-63.

Burns J.M., Summers B.C., et al. (2006) A novel chemokine receptor for SDF-1 and I-TAC involved in cell survival, cell adhesion, and tumor development. J Exp Med 203(9): 2201-2213

Butler J.M., Guthrie S.M., et al. (2005) SDF-1 is both necessary and sufficient to promote proliferative retinopathy. J Clin Invest 115(1): 86-93

Cabioglu, N., Sahin A., et al. (2005) Chemokine receptor CXCR4 expression in breast cancer as a potential predictive marker of isolated tumor cells in bone marrow. Clin Exp Metastasis 22(1): 39-46.

Corcione A., Ottonello L., et al. (2000) Stromal cell-derived factor-1 as a chemoattractant for follicular center lymphoma B cells. J Natl Cancer Inst 92(8): 628-35.

Fedyk E.R., Jones D., et al. (2001) Expression of stromal-derived factor-1 is decreased by IL-1 and TNF and in dermal wound healing. J Immunol. 166(9):5749-54.

Geminder H., Sagi-Assif O., et al. (2001) A possible role for CXCR4 and its ligand, the CXC chemokine stromal cell-derived factor-1, in the development of bone marrow metastases in neuroblastoma. J Immunol 167(8): 4747-57.

Grassi F., Cristino S., et al. (2004) CXCL12 chemokine up-regulates bone resorption and MMP-9 release by human osteoclasts: CXCL12 levels are increased in synovial and bone tissue of rheumatoid arthritis patients. J Cell Physiol 199(2): 244-51.

Grunewald M., Avraham I., et al. (2006) VEGF-induced adult neovascularization: recruitment, retention, and role of accessory cells. Cell 124(1): 175-89.

Gulino, A.V., Moratto D., et al. (2004) Altered leukocyte response to CXCL12 in patients with warts hypogammaglobulinemia, infections, myelokathexis (WHIM) syndrome. Blood 104(2): 444-52.

Hwang J.H., Chung H.K., et al. (2003) CXC chemokine receptor 4 expression and function in human anaplastic thyroid cancer cells. J Clin Endocrinol Metab 88(1): 408-16.

Kanbe K., Takagishi K., et al. (2002) Stimulation of matrix metalloprotease 3 release from human chondrocytes by the interaction of stromal cell-derived factor 1 and CXC chemokine receptor 4. Arthritis Rheum 46(1): 130-7.

Kawai T., Choi U., et al. (2005) Enhanced function with decreased internalization of carboxy-terminus truncated CXCR4 responsible for WHIM syndrome. Exp Hematol 33(4): 460-8.

Kioi M., Vogel H., et al. (2010) Inhibition of vasculogenesis, but not angiogenesis, prevents the recurrence of glioblastoma after irradiation in mice. J Clin Invest 120(3): 694-705.

Klussmann S. (2006). The Aptamer Handbook - Functional Oligonucleotides and their Applications. Edited by S. Klussmann. WILEY-VCH, Weinheim, Germany, ISBN 3-527-31059-2

Koshiba T., Hosotani R., et al. (2000) Expression of stromal cell-derived factor 1 and CXCR4 ligand receptor system in pancreatic cancer: a possible role for tumor progression. Clin Cancer Res 6(9): 3530-5.

Kozin S.V., Kamoun W.S., et al. (2010) Recruitment of myeloid but not endothelial precursor cells facilitates tumor regrowth after local irradiation. Cancer Res 70(14): 5679-85.

Krumbholz M., Theil D., et al. (2006) Chemokines in multiple sclerosis: CXCL12 and CXCL13 upregulation is differentially linked to CNS immune cell recruitment. Brain 129: 200-211.

Kusser W. (2000) Chemically modified nucleic acid aptamers for in vitro selections: evolving evolution. J Biotechnol 74(1): 27-38.

Marechal V., Arenzana-Seisdedos F., et al. (1999) Opposite effects of SDF-1 on human immunodeficiency virus type 1 replication. J Virol 73(5): 3608-15.

McGinnis S., Madden T.L. et al. (2004) BLAST: at the core of a powerful and diverse set of sequence analysis tools. Nucleic Acids Res. 32(Web Server issue):W20-5.

Meleth A.D., Agron E., et al. (2005) Serum inflammatory markers in diabetic retinopathy. Invest Ophthalmol Vis Sci 46(11): 4295-301.

Muller, A., Homey B., et al. (2001) Involvement of chemokine receptors in breast cancer metastasis. Nature 410(6824): 50-6.

Murdoch, C. (2000) CXCR4: chemokine receptor extraordinaire. Immunol Rev 177: 175-84.

Needleman and Wunsch (1970) A general method applicable to the search for similarities in the amino acid sequence of two proteins. J Mol Biol. 48(3):443-53.

Pearson and Lipman (1988) Improved tools for biological sequence comparison. Proc. Nat'l. Acad. Sci. USA 85: 2444

Salcedo R., Wasserman K., et al. (1999) Vascular endothelial growth factor and basic fibroblast growth factor induce expression of CXCR4 on human endothelial cells: In vivo neovascularization induced by stromal-derived factor-lalpha. Am J Pathol 154(4): 1125-1135

Salcedo, R. and Oppenheim J.J. (2003) Role of chemokines in angiogenesis: CXCL12/SDF-1 and CXCR4 interaction, a key regulator of endothelial cell responses. Microcirculation 10(3-4): 359-70.

Salvucci O., Yao L., et al. (2002) Regulation of endothelial cell branching morphogenesis by endogenous chemokine stromal-derived factor-1. Blood 99(8): 2703-11.

Scotton C.J., Wilson J.L., et al. (2002) Multiple actions of the chemokine CXCL12 on epithelial tumor cells in human ovarian cancer. Cancer Res. 62(20):5930-8

Shirozu M., Nakano T., et al. (1995) Structure and chromosomal localization of the human stromal cell-derived factor 1 (SDF1) gene. Genomics 28(3): 495-500.

Smith and Waterman (1981), Adv. Appl. Math. 2: 482

Soriano A., Martinez C., et al. (2002) Plasma stromal cell-derived factor (SDF)-1 levels, SDF1-3'A genotype, and expression of CXCR4 on T lymphocytes: their impact on resistance to human immunodeficiency virus type 1 infection and its progression. J Infect Dis 186(7): 922-31.

Venkatesan N., Kim S.J., et al. (2003) Novel phosphoramidite building blocks in synthesis and applications toward modified oligonucleotides. Curr Med Chem 10(19): 1973-91.

Wang J., Guan E., et al. (2001) Role of tyrosine phosphorylation in ligand-independent sequestration of CXCR4 in human primary monocytes-macrophages. J Biol Chem 276(52): 49236-43.

Wang N., Wu Q.L., et al. (2005) Expression of chemokine receptor CXCR4 in nasopharyngeal carcinoma: pattern of expression and correlation with clinical outcome. J Transl Med 3: 26.

Yamaguchi J., Kusano K.F., et al. (2003) Stromal cell-derived factor-1 effects on ex vivo expanded endothelial progenitor cell recruitment for ischemic neovascularization. Circulation 107(9): 1322-8.

Yang J., Zhang B. et al. (2008) Breast cancer metastasis suppressor 1 inhibits SDF-1alpha-induced migration of non-small cell lung cancer by decreasing CXCR4 expression. Cancer Lett 269(1):46-56

Zagzag D., Esencay M., et al. (2008) Hypoxia- and vascular endothelial growth factor-induced stromal cell-derived factor-1alpha/CXCR4 expression in glioblastomas: one plausible explanation of Scherer's structures. Am J Pathol 173(2): 545-560

Zhou Y., Larsen P.H., et al. (2002) CXCR4 is a major chemokine receptor on glioma cells and mediates their survival. J Biol Chem 277(51): 49481-7.

The features of the present invention disclosed in the specification, the claims and/or the drawings may both separately and in any combination thereof be material for realizing the invention in various forms thereof.

## Claims

1. A C-X-C motif chemokine 12 (CXCL12) antagonist for use in a method for treating a tumor in a subject, wherein the method comprises administering to the subject
- the C-X-C motif chemokine 12 (CXCL12) antagonist,
- a radiotherapy, and
- an anti-angiogenic compound,
wherein the tumor is a brain tumor.

2. The C-X-C motif chemokine 12 (CXCL12) antagonist for use of claim 1, wherein the tumor is a grade IV astrocytoma or glioblastoma.

3. The C-X-C motif chemokine 12 (CXCL12) antagonist for use of any one of claims 1 to 2, wherein the CXCL12 antagonist inhibits the binding of CXCL12 to CXCL12 receptor C-X-C chemokine receptor type 4 (CXCR4) and/or to CXCL12 receptor C-X-C chemokine receptor type 7 (CXCR7), preferably the CXCL12 antagonist is binding to CXCL12.

4. The C-X-C motif chemokine 12 (CXCL12) antagonist for use of any one of claims 1 to 3, wherein the CXCL12 antagonist is a nucleic acid molecule binding to CXCL12, wherein the nucleic acid molecule binding to CXCL12 comprises a central stretch of nucleotides, whereby the central stretch of nucleotides comprises the following nucleotide sequence:
5' GUGUGAUCUAGAUGUADWGGCUGWUCCUAGUYAGG 3' (SEQ ID NO: 52).

5. The C-X-C motif chemokine 12 (CXCL12) antagonist for use of any one of claims 1 to 4, wherein the nucleic acid molecule binding to CXCL12 comprises a nucleotide sequence according to any one of SEQ ID NO: 22 to SEQ ID NO: 28 and SEQ ID NO: 5 to SEQ ID NO: 20, or a nucleotide sequence at least 80 % identical thereto,
preferably a nucleotide sequence according to any one of SEQ ID NO: 22, SEQ ID NO: 28, SEQ ID NO: 5, SEQ ID NO: 6, SEQ ID NO: 7 and SEQ ID NO: 16, or a nucleotide sequence at least 80 % identical thereto,
more preferably a nucleotide sequence according to any one of SEQ ID NO: 22 and SEQ ID NO: 28, or a nucleotide sequence at least 80 % identical thereto.

6. The C-X-C motif chemokine 12 (CXCL12) antagonist for use of claim 5, wherein the nucleic acid molecule binding to 12 is an L-ribonucleic acid molecule comprising a nucleotide sequence of
GCGUGGUGUGAUCUAGAUGUAUUGGCUGAUCCUAGUCAGGUACGC (SEQ ID NO: 22), preferably the L-ribonucleic acid molecule comprises a 40 kDa polyethylene glycol moiety, wherein the 40 kDa polyethylene glycol moiety is attached to the 5'end of nucleotide sequence.

7. The C-X-C motif chemokine 12 (CXCL12) for use of any one of claims 1 to 6, wherein the radiotherapy is administered by means of external beam radiotherapy or internal radiation therapy (brachytherapy).

8. The C-X-C motif chemokine 12 (CXCL12) for use of claim 7, wherein the radiotherapy is external beam radiotherapy, wherein the radiotherapy is a fractionated radiotherapy and wherein a total absorbed radiation dose of the radiotherapy is from about 10 Gy to about 100 Gy, preferably from about 40 Gy to about 60 Gy, more preferably about 40 Gy or about 60 Gy.

9. The C-X-C motif chemokine 12 (CXCL12) for use of claim 7, wherein the radiotherapy is external beam radiotherapy, wherein radiotherapy is a hypofractionated radiotherapy and wherein the individual absorbed radiation dose is from about 0.5 Gy to about 5 Gy, preferably from about 2 Gy to about 2.7 Gy, more preferably about 2 Gy or about 2.7 Gy.

10. The C-X-C motif chemokine 12 (CXCL12) for use of any one of claims 1 to 9, wherein the anti-angiogenic compound is an anti-VEGF antibody.

11. The C-X-C motif chemokine 12 (CXCL12) for use of claim 10, wherein the anti-VEGF antibody comprises the following CDRs of the light chain variable region CDR1: QDISNY, CDR2: FTS, and CDR3: QQYSTVPWT, and/or the following CDRs of the heavy chain variable region CDR1: GYTFTNYG, CDR2: INTYTGEP, and CDR3: AKYPHYYGSSHWYFDV.

12. The C-X-C motif chemokine 12 (CXCL12) for use of any one of claims 10 to 12, wherein the anti-VEGF antibody is Bevacizumab.

13. The C-X-C motif chemokine 12 (CXCL12) for use of any one of claims 1 to 12, wherein the method further comprises administering a chemotherapy to the subject.

14. The C-X-C motif chemokine 12 (CXCL12) for use of claim 13, wherein the chemotherapy is or comprises temozolomide.
